(19) Europäisches Patentamt
European Patent Office
Office européen des brevets

(11) Veröffentlichungsnummer: **0 286 926 B1**

# (12) EUROPÄISCHE PATENTSCHRIFT

(45) Veröffentlichungstag der Patentschrift: **11.01.95**

(51) Int. Cl.[6]: **C07H 15/252**, A61K 31/70, C07C 50/36

(21) Anmeldenummer: **88105330.0**

(22) Anmeldetag: **01.04.88**

## (54) Semisynthetische Rhodomycine, Verfahren zu ihrer Herstellung und ihre Verwendung als Zytostatika.

(30) Priorität: **11.04.87 DE 3712350**

(43) Veröffentlichungstag der Anmeldung:
**19.10.88 Patentblatt 88/42**

(45) Bekanntmachung des Hinweises auf die Patenterteilung:
**11.01.95 Patentblatt 95/02**

(84) Benannte Vertragsstaaten:
**AT BE CH DE ES FR GB GR IT LI LU NL SE**

(56) Entgegenhaltungen:
**EP-A- 0 171 677** **EP-A- 0 203 329**
**EP-A- 0 242 695** **EP-A- 0 270 992**
**EP-A- 0 290 744** **EP-A- 0 299 350**
**GB-A- 2 056 443** **GB-A- 2 120 667**
**US-A- 4 302 449**

**TETRAHEDRON, Band 40, Nr. 22, 1984, Seiten 4633-4642, Pergamon Press Ltd, GB;J.S. SWENTON et al.: "The synthesis of 6-deoxy-anthracyclinones: (+-)alpha-citromycinone and (+-)4-demethoxy-6-deoxydaunomycinone"**

(73) Patentinhaber: **BEHRINGWERKE Aktiengesellschaft
Postfach 1140
D-35001 Marburg (DE)**

(72) Erfinder: **Gerken, Manfred, Dr.
Wannkopfstrasse 12
D-3550 Marburg (DE)**
Erfinder: **Kolar, Cenek, Dr.
Deutschhausstrasse 20
D-3550 Marburg (DE)**
Erfinder: **Kraemer, Hans Peter, Dr.
Birkenweg 16
D-3550 Marburg (DE)**
Erfinder: **Hoffmann, Dieter, Dr.
Im Stetefeld 6
D-3551 Lahntal (DE)**
Erfinder: **Hermentin, Peter, Dr.
Barfüssertor 30
D-3550 Marburg (DE)**

EP 0 286 926 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 108, Nr. 9, 29. Februar 1988, Seite 31,Zusammenfassung Nr. 68421s, Columbus, Ohio, US; T. RAIM et al.: "Interactionspecificity of violamycins BI and BII with DNA as studied by hyperchromicspectra", & BIOFIZIKA 1987, 32(6), 1006-10& FORMULA INDEX, C22-Z; C28H33NO11, 5,12-Naphthacenedione, 8-ethyl-7,8,9,10-tetrahydro-1,4,6,8,10,11-hexahydroxy-7-[[2,3,6-trideoxy-3-(dimethylamino)-alpha-L--lyxo-hexopyranosy1]oxy]-[7R-(7alpha,8beta,10beta]-[-112720-41-9], 68421s

CHEMICAL ABSTRACTS, Band 109, Nr. 19, 7. November 1988, Seite 759,Zusammenfassung Nr. 170806d, Columbus, Ohio, US;& JP-A-63 91 395 (KIRIN BREWERY CO., LTD) 22-04-1988

**Beschreibung**

Die Erfindung betrifft Verbindungen der Formel I, deren Salze eine anorganischen oder organischen Säure,

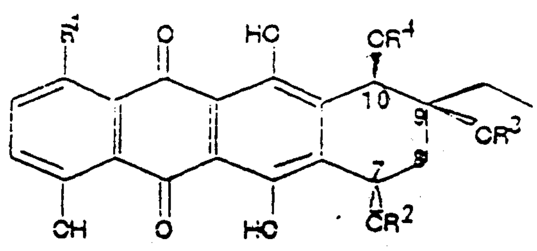

I

für welche gilt:
$R^1$ ist Wasserstoff oder eine Hydroxygruppe
$R^2$ ist Wasserstoff, eine Struktur der Formel II oder IV oder zusammen mit $R^3$, der Formel III,

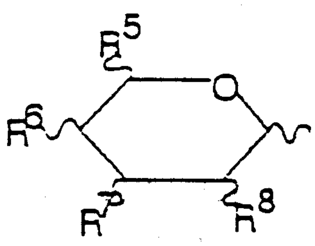

II

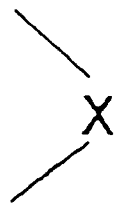

III

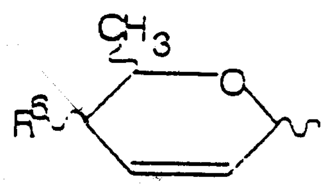

IV

$R^3$ ist Wasserstoff, eine Struktur der Formel II oder IV, oder zusammen mit $R^2$ der Formel III,
$R^4$ ist Wasserstoff, Trimethylsilyl, eine in der Kohlenhydratchemie übliche Schutzgruppe vorzugsweise Acetyl, Trifluoracetyl, Benzoyl oder substituiertes Benzoyl wie para-Nitrobenzoyl, eine Struktur der Formel II oder eine Struktur der Formel IV,
$R^5$ ist Wasserstoff, Mehthyl, Hydroxymethyl, Acyloxymethyl (C1-C8) oder Alkyloxymethyl (C1-C8),
$R^6$ ,$R^7$ und $R^8$ sind unabhängig voneinander, Wasserstoff, Hydroxy, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy oder Halogen, wobei $R^7$ weiterhin $NH_2$, NHAcyl (C1-C8), $N(Alkyl)_2$ (C1-C8), $N(CH_2CN)_2$, $NH(CH_2CN)$ oder Azido sein kann, und
X ist eine in der Kohlenhydratchemie übliche zweizähnige Schutzgruppe, vorzugsweise Alklboronyl, Phenylboronyl, ein Ortho-Alkyl-Carbonsäurealkylester, vorzugsweise Orthoameisensäuremethylester oder Orthoessigsäureethylester, ein Ketal oder Acetal, vorzugsweise Isopropyliden oder Benzyliden,
wobei die Verbindungen mit $R^1 = R^2 = R^3 = R^4 = H$, $R^1 = OH$ und $R^2 = R^3 = R^4 = H$, $R^1 = R^3 = R^4 = H$ und $R^2 = \alpha$-L-Daunosaminyl oder $\alpha$-L-Rhodosaminyl oder 4'-Acyl-$\alpha$-L-rhodosaminyl, $R^1 = OH$ oder H, $R^3 = H$ und $R^2 = R^4 = \alpha$-L-Rhodosaminyl oder 4'-Acyl-$\alpha$-L-rhodosaminyl und $R^1 = R^2 = R^3 = H$ und $R^4 = \alpha$-L-Rhodosaminyl sowie $R^1$ = OH, $R^3$ = $R^4$ = H und $R^2$ = Rhodosaminyl oder Daunosaminyl ausgenommen sind.

Zahlreiche Anthracycline zeigen zytostatische Wirksamkeit und einige werden für die Therapie von Tumoren eingesetzt.

Bekannt sind $\beta$-Rhodomycine (Strukturen der Formel I mit $R^1 = H$), bei denen die Position 7 und die Position 10 mit L-Rhodosamin $\alpha$-glycosidisch verknüpft ist oder bei denen nur die Position 7 $\alpha$-glycosidisch mit L-Rhodosamin oder L-Daunosamin verknüpft ist, sowie $\beta$-Iso-rhodomycine (Strukturen der Formel I mit $R^1 = OH$), bei denen Position 7 und 10 jeweils mit $\alpha$-L-Rhodosamin verknüpft sind, sowie die photolytischen Mono-demethylierungsprodukte dieser L-Rhodosamin-glycoside und $\beta$-Rhodomycinon mit Oligosaccharidseitenketten an den Positionen 7 oder 10 oder an 7 und 10, Cytorhodine genannt. Aus Journal of Antibiotics 33, 1331 (1980) ist die mikrobielle Glycosidierung eines Trisaccharides bestehend aus L-Rhodosamin, L-Desoxyfucose und Cinerulose A an der 7-Position des $\beta$-Rhodomycinon bekannt.

In EP-A-0 203 329 ist eine Verbindung der Formel I, wobei $R^1$ = OH, $R^3$ = $R^4$ = H und $R^2$ = Rhodosaminyl sind, und in EP-A-0 242 695 eine Verbindung der Formel I, wobei $R^1$ = OH, $R^3$ = $R^4$ = H

und $R^2$ = Daunosaminyl sind, beschrieben.

Es wurde bislang kein chemisches Glycosidierungsverfahren für β-Rhodomycinon und β-iso-Rhodomycinon beschrieben. Durch die mehrfachen Glycosidierungsmöglichkeiten der drei Hydroxylgruppen im A-Ring des β-Rhodomycinons (Struktur der Formel I mit $R^1 = R^2 = R^3 = R^4 = H$) sind selektive Glycosidierungen ungleich schwieriger.

Überraschenderweise zeigte sich, daß β-Rhodomycinon und β-iso-Rhodomycinon sich unter Verwendung bestimmter Schutzgruppen an den Positionen 7,9 und 10 selektiv glycosidieren lassen und die so erhaltenen Glycoside, besonders das 7-O-α-L-Acosaminyl-β-rhodomycinon oder das 7-0-(3'-N,N-Dimethyl-α-L-acosaminyl)-β-rhodomycinon eine zytostatische Wirksamkeit besitzen, die mit der von Adriamycin vergleichbar ist.

Der vorliegenden Erfindung liegt daher die Aufgabe zugrunde, ausgehend von den auf biologischem Wege erhältlichen β-(iso-)Rhodomycinonen neue mono-, bis- und tris-Glycosyl-β-(iso-)Rhodomycinone herzustellen, die sich durch zytostatische Wirksamkeit auszeichnen und deshalb zur Behandlung von Tumoren geeignet sind.

Gelöst wurde diese Aufgabe durch die Herstellung von Verbindungen der oben angegebenen Formel I mit den dort für $R^1$ bis $R^8$ und X angegebenen Definitionen und den angegebenen Ausnahmen.

Gegenstand der Erfindung sind daher Verbindungen dieser Formel I mit den angegebenen Definitionen und Ausnahmen.

In der folgenden Aufstellung von Verbindungen der Formel I mit den dabei angegebenen Resten $R^1$ bis $R^8$ is jeweils die auf eine Gruppe von Verbindungen folgende Gruppe von Verbindungen gegenüber jener Gruppe bevorzugt.

1) $R^1$ bis $R^8$ haben die angegebene Bedeutung.

2) $R^1$ und $R^4$ haben die angegebene Bedeutung, $R^2$ und $R^3$ sind unabhängig voneinander Wasserstoff, eine Struktur der Formel II oder IV mit den angegebenen Bedeutungen für $R^5$ bis $R^8$.

3) $R^1$ ist Wasserstoff, die übrigen Definitionen sind wie unter 2).

4) $R^3$ ist Wasserstoff, die übrigen Definitionen sind wie unter 2).

5) $R^1$ und $R^4$ haben die angegebene Bedeutung, $R^2$ und $R^3$ sind unabhängig voneinander Wasserstoff, eine Struktur der Formel II oder IV, wobei $R^5$ Methyl ist, $R^6$ und $R^7$ die angegebene Bedeutung haben und $R^8$ Wasserstoff oder Halogen ist.

6) $R^1$ ist Wasserstoff, die übrigen Definitionen sind wie unter 5).

7) $R^3$ ist Wasserstoff, die übrigen Definitionen sind wie unter 5).

8) $R^1$ und $R^4$ haben die angegebene Bedeutung, $R^2$ und $R^3$ sind unabhängig voneinander Wasserstoff, eine Struktur der Formel II oder IV, wobei $R^5$ Methyl ist, $R^6$ die angegebene Bedeutung hat, $R^7$ $NH_2$, NHAcyl (C1-C8), $N(Alkyl)_2$ (C1-C8), $N(CH_2CN)_2$, $NH(CH_2CN)$ oder Azido ist, $R^8$ Wasserstoff oder Halogen ist.

9) $R^1$ ist Wasserstoff, die übrigen Definitionen sind wie unter 8).

10) $R^3$ ist Wasserstoff, die übrigen Definitionen sind wie unter 8).

11) $R^1$ und $R^4$ haben die angegebene Bedeutung, $R^2$ und $R^3$ sind unabhängig voneinander Wasserstoff, eine Struktur der Formel II oder IV, wobei $R^5$ Methyl ist, $R^6$ und $R^7$ die angegebene Bedeutung haben und $R^8$ Wasserstoff oder Halogen ist und die Strukturen der Formel II oder IV der L-Reihe der Kohlenhydrate angehören.

12) $R^1$ ist Wasserstoff, die übrigen Definitionen sind wie unter 11).

13) $R^3$ ist Wasserstoff, die übrigen Definitionen sind wie unter 11).

14) $R^1$ und $R^4$ haben die angegebene Bedeutung, $R^2$ und $R^3$ sind unabhängig voneinander Wasserstoff, eine Struktur der Formel II oder IV, wobei $R^5$ Methyl ist, $R^6$ die angegebene Bedeutung hat, $R^7$ ist $NH_2$, NHAcyl (C1-C8), $N(Alkyl)_2$ (C1-C8), $N(CH_2CN)_2$, $NH(CH_2CN)$ oder Azido, $R^8$ ist Wasserstoff oder Halogen, die Strukturen der Formel II oder IV gehören der L-Reihe der Kohlenhydrate an.

15) $R^1$ ist Wasserstoff, die übrigen Definitionen sind wie unter 14).

16) $R^3$ ist Wasserstoff, die übrigen Definitionen sind wie unter 14).

17) $R^1$ und $R^3$ sind Wasserstoff, die übrigen Definitionen sind wie unter 2).

18) $R^1$ und $R^3$ sind Wasserstoff, die übrigen Definitionen sind wie unter 5).

19) $R^1$ und $R^3$ sind Wasserstoff, die übrigen Definitionen sind wie unter 8).

20) $R^1$ und $R^3$ sind Wasserstoff, die übrigen Definitioneen sind wie unter 11).

21) $R^1$ und $R^3$ sind Wasserstoff, die übrigen Definitionen sind wie unter 14).

22) $R^2$ und $R^3$ sind Wasserstoff, die übrigen Definitionen sind wie unter 2).

23) $R^3$ und $R^4$ sind Wasserstoff, die übrigen Definitionen sind wie unter 2).

24) $R^2$ und $R^3$ sind Wasserstoff, die übrigen Definitionen sind wie unter 5).

25) $R^3$ und $R^4$ sind Wasserstoff, die übrigen Definitionen sind wie unter 5).

26) $R^2$ und $R^3$ sind Wasserstoff, die übrigen Definitionen sind wie unter 8).
27) $R^3$ und $R^4$ sind Wasserstoff, die übrigen Definitionen sind wie unter 8).
28) $R^2$ und $R^3$ sind Wasserstoff, die übrigen Definitionen sind wie unter 11).
29) $R^3$ und $R^4$ sind Wasserstoff, die übrigen Definitionen sind wie unter 11).
30) $R^2$ und $R^3$ sind Wasserstoff, die übrigen Definitionen sind wie unter 14).
31) $R^3$ und $R^4$ sind Wasserstoff, die übrigen Definitionen sind wie unter 14).
32) $R^1$, $R^2$ und $R^3$ sind Wasserstoff, $R^4$ ist wie unter 2).
33) $R^1$, $R^3$ und $R^4$ sind Wasserstoff, $R^2$ ist wie unter 2).
34) $R^1$, $R^2$ und $R^3$ sind Wasserstoff, $R^4$ ist wie unter 5).
35) $R^1$, $R^3$ und $R^4$ sind Wasserstoff, $R^2$ ist wie unter 5).
36) $R^1$, $R^2$ und $R^3$ sind Wasserstoff, $R^4$ ist wie unter 8).
37) $R^1$, $R^3$ und $R^4$ sind Wasserstoff, $R^2$ ist wie unter 8).
38) $R^1$, $R^2$ und $R^3$ sind Wasserstoff, $R^4$ ist wie unter 11).
39) $R^1$, $R^3$ und $R^4$ sind Wasserstoff, $R^2$ ist wie unter 11).
40) $R^1$, $R^2$ und $R^3$ sind Wasserstoff, $R^4$ ist wie unter 14).
41) $R^1$, $R^3$ und $R^4$ sind Wasserstoff, $R^2$ ist wie unter 14).

Gegenstand der Erfindung ist auch ein Verfahren zur Herstellung einer Verbindung der Formel I mit den angegebenen Definitionen, dadurch gekennzeichnet, daß man
eine Verbindung der Formel I, in welcher $R^1$ die anfangs genannte Bedeutung hat und $R^2$ bis $R^4$ Wasserstoff sind, zunächst wie unten beschrieben, an den Positionen 7 und 9 selektiv durch eine Struktur der Formel III blockiert, wobei eine Verbindung der Formel I erhalten wird, in der $R^2$ und $R^3$ zusammen eine Struktur der Formel III sind, und anschließend $R^4 = H$ durch eine Acylschutzgruppe, eine Trimethylsilylgruppe oder ein Kohlenhydratderivat der Struktur II oder IV ersetzt und gegebenenfalls entblockiert und/oder an der Aminofunktion modifiziert, woraufhin nach selektiver Abspaltung der Schutzgruppen an Position 7 und 9 entweder nur die Position 7 oder Position 7 und 9 glycosidiert und die Glycosylreste entblockiert und modifiziert werden können, oder aber, indem man nach Einführung einer Acylgruppe oder Trimethylsilylgruppe an Position 10 und anschließender selektiver Entblockierung an Position 7 und 9 zunächst an Position 7 oder gleichzeitig an Position 7 und 9 glycosidiert, anschließend ganz oder teilweise entblockiert und gegebenenfalls an den Aminofunktionen modifiziert und erst dann die Position 10 glycosidiert und dieses Glycosid modifiziert, was im einzelnen dadurch erfolgt, daß man

a) eine Verbindung der Formel I, in welcher $R^1$ die anfangs genannte Bedeutung hat und $R^2$ bis $R^4$ Wasserstoff sind, in einem geeigneten organischen Lösungsmittel wie Toluol oder DMF oder deren Mischungen, mit einem Katalysator wie einer Mineral-, Carbon- oder Sulfonsäure, bei einer Temperatur zwischen 0 °C und der Siedetemperatur des Lösungsmittels, mit einer Boronsäure wie Phenylboronsäure oder mit einem Keton wie Aceton oder einem Ketal wie 2,2-Dimethoxypropan oder einem Acetal wie Benzaldehyddimethylacetal, gegebenenfalls unter Zugabe eines Wasserfängers wie einem 4Å Molekularsieb zur Reaktion bringt, worauf man eine Verbindung der Formel I erhält, bei der $R^1$ die anfangs genannte Bedeutung hat und $R^2$ zusammen mit $R^3$ eine Verbindung der Formel III bilden, welche man durch Abfiltrieren und Entfernen des Lösungsmittels isoliert, und aus einem organischen Lösungsmittel wie Petrolether, auskristallisiert, woraufhin die Hydroxylgruppe an der Position 10 in geeigneter Weise derivatisiert wird,

b) gegebenenfalls durch eine Acylierung mit einem Carbonsäureanhydrid wie Essigsäureanhydrid, Trifluoressigsäureanhydrid oder einen Phenylcarbonsäureanhydrid oder einem Carbonsäurehalogenid oder durch Umsetzung mit Trifluormethansulfonsäure-trimethylsilylester in einem geeigneten organischen Lösungsmittel wie Chloroform, Dichlormethan oder Toluol oder deren Mischungen, bei einer Temperatur zwischen -40 °C und der Siedetemperatur des Lösungsmittels und in Gegenwart einer Base wie Triethylamin oder Pyridin, wobei eine Verbindung der Formel I erhalten wird, bei der
$R^1$ die anfangs genannte Bedeutung hat,
$R^2$ zusammen mit $R^3$ eine Struktur der Formel III und
$R^4$ Acyl oder Trimethylsilyl ist,

c) oder beispielsweise durch Umsetzung der unter a) erhaltenen Verbindung mit 3,4-Dihydro-2H-pyran in einem geeigneten organischen Lösungsmittel wie Chloroform, Dichlormethan, DMF oder Toluol in Gegenwart eines Katalysators wie para-Toluolsulfonsäure und eines Trockenmittels wie 4Å Molekularsieb, bei einer Temperatur zwischen -30 °C und der Siedetemperatur des Lösungsmittels, wobei eine Verbindung der Formel I entsteht,
bei der
$R^1$ die anfangs genannte Bedeutung hat,
$R^2$ zusammen mit $R^3$ eine Struktur der Formel III

$R^4$ eine Struktur der Formel II mit $R^5$ bis $R^8$ Wasserstoff ist,
d) oder beispielsweise durch Umsetzung der unter a) erhaltenen Verbindung unter den in der Kohlenhydratchemie üblichen Bedingungen mit einem Kohlenhydratderivat der Formel V,

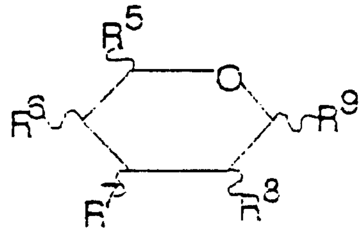

V

wobei
$R^5$ bis $R^8$ die anfangs genannte Bedeutung als geeignete Schutzgruppen haben und
$R^9$ Halogen wie Cl oder Br, O-Acyl- oder eine andere bei Glycosidierungsreaktionen übliche Abgangsgruppe ist, wobei eine Verbindung der Formel I entsteht, bei der
$R^1$ die anfangs genannte Bedeutung hat,
$R^2$ zusammen mit $R^3$ eine Struktur der Formel III und
$R^4$ eine Struktur der Formel II ist,
e) oder beispielsweise, indem man die unter a) erhaltene Verbindung mit einem funktionalisierten Kohlenhydrat der allgemeinen Formel V oder VI,

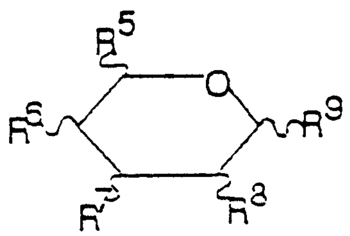

V

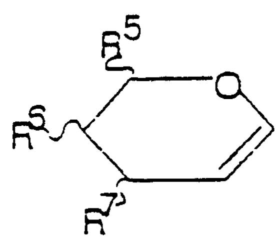

VI

worin
$R^5$ bis $R^8$ die anfangs genannten Bedeutungen mit den Ausnahmen Hydroxymethyl, Hydroxy, $NH_2$, N-$(CH_2CN)_2$ und $NH(CH_2CN)$ haben und
$R^9$ über Sauerstoff gebundenes Acyl wie aliphatisches Acyloxy (C1-C8) wie Acetyl, Benzoyloxy, oder substituiertes Benzyloxy wie para-Nitrobenzoyloxy ist, in Gegenwart eines organischen Lösungsmittels wie Chloroform, Dichlormethan, Toluol, Ether, DMF, Aceton, Acetonitril oder Nitromethan oder deren Mischungen, eines Katalysators, wie para-Toluolsulfonsäure oder eines Trifluormethansulfonsäure-trialkylsilylesters und gegebenenfalls eines Säurefängers und eines Trockenmittels wie einem Molekularsieb, bei einer Reaktionstemperatur von -70 °C bis +30 °C unter einer Schutzgasatmosphäre wie Stickstoff oder Argon, zu einer Verbindung der Formel I umsetzt,
wobei
$R^1$ die genannte Bedeutung hat,
$R^2$ zusammen mit $R^3$ eine Struktur der Formel III und
$R^4$ eine Struktur der Formel II oder IV ist, bei der
$R^5$ bis $R^8$ die anfangs genannten Bedeutungen mit den Ausnahmen Hydroxymethyl, Hydroxy, $NH_2$, N-$(CH_2CN)_2$ und $NH(CH_2CN)$ haben,
f) und gegebenenfalls man die Verbindung der Stufen b) bis e) an den Positionen 7 und 9 entblockieren kann, indem man diese Verbindung in einem geeigneten organischen Lösungsmittel wie Chloroform, Dichlormethan, DMF, Toluol oder Methanol mit einem Katalysator wie verdünnte wässrige Lösungen von Carbonsäuren oder para-Toluolsulfonsäure und gegebenenfalls mit einem Diol wie 2-Methyl-2,4-pentandiol, bei einer Temperatur zwischen 0 °C und der Siedetemperatur des Lösungsmittels zur Reaktion

bringt, woraufhin man eine Verbindung der allgemeinen Formel I erhält, bei der
$R^1$ die genannte Bedeutung hat,
$R^2$ und $R^3$ Wasserstoff sind und
$R^4$ die genannte Bedeutung außer Wasserstoff hat,
g) woraufhin man gegebenenfalls die Verbindung der Stufe f) bei der $R^4$ eine Struktur der Formel II oder IV mit einer der in der Kohlenhydratchemie üblichen Schutzgruppen als Reste $R^5$ bis $R^8$ ist, unter in der Kohlenhydratchemie üblichen Bedingungen selektiv an den geschützten Hydroxyfunktionen und/oder an den geschützten Aminofunktionen in an sich bekannter Weise mittels einer anorganischen oder organischen Base wie Alkali-oder Erdalkalihydroxiden, Natriumcarbonat und Triethylamin in einem Lösungsmittel wie Wasser, Methanol, Ethanol oder THF oder deren Mischungen partiell oder vollständig entblockiert kann, wobei eine Verbindung der Formel I entsteht, bei der
$R^1$ Wasserstoff oder Hydroxyl,
$R^2$ und $R^3$ Wasserstoff und
$R^4$ eine Struktur der Formel II oder IV sind, in welchen
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ $N(Alkyl)_2$, Azido, Hydroxy oder Alkyloxy sind und
$R^8$ die gleiche Bedeutung wie $R^6$ hat, aber unabhängig davon ist,
h) und man gegebenenfalls die Verbindung der Stufe g) der Formel I, bei der
$R^1$ bis $R^3$ die unter g) genannte Bedeutung haben und
$R^4$ eine Struktur der Formel II, wobei
$R^5$, $R^6$ und $R^8$ die unter g) angegebene Bedeutung haben und
$R^7$ $NH_2$ sind,
unter den an sich bekannten Bedingungen der reduktiven Aminierung in die entsprechend Verbindung der Formel I umsetzt, wobei
$R^1$ bis $R^3$ die unter g) genannte Bedeutung haben und
$R^4$ eine Struktur der Formel II ist,
bei der
$R^5$, $R^6$ und $R^8$ die unter g) genannte Bedeutung haben und
$R^6$ $N(Alkyl)_2$ ist, oder
i) weiterhin eine Verbindung der Stufe g) der allgemeinen Formel I, bei der
$R^1$ bis $R^3$, die unter g) genannte Bedeutung haben und
$R^4$ eine Struktur der Formel II mit den für
$R^5$, $R^6$ und $R^7$ unter g) angegebenen Bedeutungen und
$R^7$ $NH_2$ sind,
durch Umsetzung mit Jodacetonitril oder Bromacetonitril in einem geeigneten Lösungsmittel, beispielsweise Dimethylformamid, in Gegenwart einer geeigneten Base wie Triethylamin in eine Verbindung der Formel I überführt, bei der $R^1$ bis $R^3$ die unter g) genannte Bedeutung haben und $R^4$ eine Struktur der Formel II ist, bei der
$R^5$, $R^6$ und $R^8$ die unter g) genannten Bedeutungen haben und
$R^7$ $N(CH_2CN)_2$ oder $NH(CH_2CN)$ ist, und
k) gegebenenfalls die bei der Stufe f), h) oder i) entstandene Verbindung nach den bereits bei der Stufe e) genannten Bedingungen glycosidiert, wobei je nach Menge des benutzten Glycosyldonors entweder nur Position 7 oder gleichzeitig Position 7 und 9 glycosidiert werden, was zu Produkten führt, die der allgemeinen Formel I entsprechen, bei der
$R^1$ die genannte Bedeutung hat,
$R^2$ eine Struktur der Formel II oder IV ist, bei der
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben,
$R^3$ Wasserstoff ist oder $R^2$ entspricht und
$R^4$ Acyl, Trimethylsilyl oder eine Struktur der allgemeinen Formel II oder IV, wobei
$R^5$ bis $R^8$ die anfangs benannten Bedeutungen mit den Ausnahmen Hydroxymethylen, Hydroxy und $NH_2$ haben, ist, und gegebenenfalls
l) eine der bei der Stufe f), h) oder i) entstandenen Verbindungen in Gegenwart eines organischen Lösungsmittels wie Chloroform, Dichlormethan, Toluol, Ether, Aceton und Acetonitril oder deren Mischungen mit einem Glycal der Formel VI, wobei $R^5$ bis $R^7$ die bei Stufe e) genannte Bedeutung haben, mit N-Jodsuccinimid und gegebenenfalls mit einem Trockenmittel wie einem Molekularsieb bei einer Temperatur von $-40\,^\circ C$ bis $+40\,^\circ C$ unter einer Schutzgasatmosphäre wie Stickstoff oder Argon, zu einer

Verbindung der Formel I überführen bei der
$R^1$ die genannte Bedeutung hat und
$R^2$ eine Struktur der Formel II ist, bei der
$R^5$ Wasserstoff, Methyl, Acyloxymethyl (C1-C8), Alkyloxymethyl (C1-C8),
$R^6$ Acyloxy, Alkyloxy, Allyloxy oder Benzyloxy,
$R^7$ Acyloxy, Alkyloxy, Allyloxy, Benzyloxy, NHAcyl, $N(Alkyl)_2$, Azido und
$R^8$ Jod ist, und
$R^3$ und $R^4$ die bei Stufe k) genannte Bedeutung haben, und
m) gegebenenfalls die bei der Stufe k) oder l) entstandene Verbindung gemäß den Bedingungen der Stufe g) entblokkiert, wobei man eine Verbindung der Formel I erhält,
bei der
$R^1$ die genannte Bedeutung hat, und
$R^2$ eine Struktur der Formel II oder IV ist, bei der
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ oder $N(Alkyl)_2$, Azido, Hydroxy oder Alkyloxy ist und
$R^8$ die gleiche Bedeutung wie $R^6$ hat, aber unabhängig davon ist,
$R^3$ Wasserstoff ist oder $R^2$ entspricht, und
$R^4$ Wasserstoff, Trimethylsilyl oder eine Struktur der allgemeinen Formel II oder IV ist, wobei
$R^5$ Wasserstoff, Methyl, Hydroxymethylen oder Alkyloxymethyl,
$R^6$ Wasserstoff, Hydroxy, Alkyloxy oder Halogen,
$R^7$ $NH_2$, $N(Alkyl)_2$, $N(CH_2CN)_2$ oder $NH(CH_2CN)$, Azido, Hydroxy oder Alkyloxy ist und
$R^8$ die gleiche Bedeutung wie $R^6$ hat, aber unabhängig davon ist, oder
n) die bei der Stufe k) oder l) entstandene Verbindung unter den Bedingungen der Stufe g) auch so entblokkiert, daß selektiv nur die Position 10 entblockiert wird und man eine Verbindung der Formel I erhält, bei der
$R^1$ die genannte Bedeutung hat,
$R^2$ eine Struktur der Formel II oder IV ist, bei denen $R^5$ bis $R^8$ die anfangs genannten Bedeutungen mit den Ausnahmen Hydroxymethyl, Hydroxy und $NH_2$ haben, $R^3$ Wasserstoff ist oder $R^2$ entspricht und
$R^4$ Wasserstoff ist,
o) woraufhin sich gegebenenfalls die Verbindung der Stufe m), bei der $R^2$ eine Struktur der Formel II mit $R^7$ gleich $NH_2$ und $R^3$ Wasserstoff oder $R^2$ ist, wiederum in der für Stufe h) beschriebenen Art und Weise der reduktiven Aminierung in die entsprechenden Verbindungen umwandelt, bei der $R^6$ $N(Alkyl)_2$, ist, oder gegebenenfalls
p) eine Verbindung der Stufe m), bei der $R^2$ eine Struktur der Formel II mit $R^7$ gleich $NH_2$ und $R^3$ Wasserstoff oder $R^2$ ist, gemäß den Bedingungen der Stufe i) in die entsprechenden Cyanomethyl-Derivate überführt, bei der $R^7$ $N(CH_2CN)_2$ oder $NH(CH_2CN)$ ist, und gegebenenfalls
q) eine Verbindung der Stufe n) nach den bereits bei der Stufe e) genannten Bedingungen glycosidiert, wobei je nach Menge des benutzten Glycosyldonors entweder nur Position 10, oder gleichzeitig Positionen 9 und 10 glycosidiert werden, was zu einer Verbindung der Formel I
führt, bei der
$R^1$ Wassestoff oder Hydroxy ist und
$R^2$ eine Struktur der allgemeinen Formel II oder IV sind, bei der
$R^5$ bis $R^8$ die anfangs genannte Bedeutung mit den Ausnahmen von Hydroxymethyl, Hydroxy und $NH_2$ haben,
$R^3$ Wasserstoff oder $R^2$ oder $R^4$ ist und
$R^4$ eine Struktur der allgemeinen Formel II oder IV ist,
bei der
$R^5$ bis $R^8$ die anfangs genannte Bedeutung mit den Ausnahmen von Hydroxymethyl, Hydroxy, $NH_2$, $N(CH_2CN)_2$,$NH(CH_2CN)$ haben, und gegebenenfalls
r) die Reaktion der Stufe c), d) und l) auch an einer Verbindung der Stufe n) durchführt, so daß man eine Verbindung der Formel I erhält, bei der
$R^1$ die anfangs genannte Bedeutung hat und
$R^2$ eine Struktur der Formel II oder IV ist, bei der
$R^5$ bis $R^8$ die anfangs genannte Bedeutung mit den Ausnahmen Hydroxymethyl, Hydroxy und $NH_2$ haben,
$R^3$ Wasserstoff oder $R^2$ oder $R^4$ ist und
$R^4$ eine Struktur der allgemeinen Formel II oder IV ist, bei der

$R^5$ bis $R^8$ die anfangs genannte Bedeutung mit den Ausnahmen Hydroxymethyl, Hydroxy, $NH_2$, N-$(CH_2CN)_2$ und$NH(CH_2CN)$ haben und gegebenenfalls

s) diese Verbindung der Stufe q) oder r) wieder wie bei Stufe g) beschrieben entblockiert wird, bei der eine Verbindung der Formel I entsteht, bei der

$R^1$ die anfangs genannte Bedeutung hat und

$R^2$, $R^2$ und $R^4$ unabhängig voneinander Wasserstoff oder eine Struktur der Formel II oder IV sind, bei der $R^5$ bis $R^8$ die anfangs genannte Bedeutung mit den Ausnahmen Acyloxymethyl, Acyloxy, Benzoyloxy oder substituiertes Benzoyloxy, NHAcyl, und gegebenenfalls

t) die Verbindung der Stufe s) entsprechend der Stufe h) mittels Reduktions-Aminierung in das entsprechende Derivat überführt wird, bei dem der Rest $R^7$, der in der Stufe s) eine $NH_2$ Gruppe war in $N(Alkyl)_2$ gewandelt ist, oder

u) des weiteren eine Verbindung der Stufe s) gemäß den Bedingungen der Stufe i) in das entsprechende Cyanomethyl-Derivat überführt wird, bei dem der Rest $R^7$, der in der Stufe s) eine $NH_2$ Gruppe war, in $NH(CH_2CN)$ gewandelt ist,

v) und man gegebenenfalls Verbindung der Formel I, bei der $R^1$,$R^2$ und $R^3$ die genannte Bedeutung haben und

$R^4$ Trimethylsilyl ist,

in einem organischen Lösungsmittel wie THF, Diethylether, Dioxan oder deren Gemische bei Temperaturen zwischen -40 °C und der Siedetemperatur des Lösungsmittels mit Tetrabutylammoniumfluorid umsetzt zu einer Verbindung der Formel I, bei der

$R^1$, $R^2$ und $R^3$ die oben genannte Bedeutung haben und

$R^4$ Hydroxyl ist,

w) und man gegebenenfalls Verbindungen der Formel I, bei der $R^1$, $R^2$, $R^3$, $R^4$ die genannte Bedeutung haben und $R^7$ $NH_2$, $N(Alkyl)_2$ (C1-C8), $N(CH_2CN)_2$ oder $NH(CH_2CN)$ ist in das Salz einer anorganischen oder organischen Säure überführt.

Gegenstand der Erfindung sind auch Arzneimittel, die eine oder mehrere Verbindungen der Formel I, oder deren Salze mit einer anorganischen Säure wie HCl oder einer organischen Säure wie Glutaminsäure oder Glucoronsäure als Wirkstoff enthalten.

Eine solche Verbindung kann zusammen mit den üblichen pharmazeutischen Konfektionierungs- und/oder Verdünnungsmitteln zu einem Arzneimittel verarbeitet werden, das besonders in der Krebstherapie Anwendung findet.

Die Dosierungs- und Anwendungsweise entspricht dabei im wesentlichen denjenigen für die bekannten Anthracycline, wie Adriamycin, Daunomycin oder Aclacinomycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Gegenstand der Erfindung ist auch eine Zusammensetzung enthaltend eine Verbindung der Formel I mit den angegebenen Definitionen und Ausnahmen und eine Trägersubstanz.

Gegenstand der Erfindung ist weiterhin die Verwendung einer Verbindung der Formel I mit den angegebenen Definitionen und Ausnahmen als Arzneimittel.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen nach Inkubation von 1 Stunde oder über mehrere Generationen. Bei einer Zelzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des Testverfahrens ergeben sich aus der nachfolgenden Verfahrensweise zur Ermittlung der Koloniebildung.

Verfahrensweise zur Ermittlung der Koloniebildung von L1210 Leukämiezellen in Soft-Agar

500 Leukämiezellen pro Platte wurden mit unterschiedlichen Konzentrationen der Testsubstanz 1 Stunde bei 37 °C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37 °C inkubiert (5 Vol.-% $CO_2$, 95 % relative Luftfeuchtigkeit).

Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskruve wurde die $IC_{50}$ als Maß für die Wirksamkeit der Substanz ermittelt. Die Ergebnisse für die hier beschriebenen Verbindungen im Vergleich zu Adriamycin sind in der nachfolgenden Tabelle 1 zusammengefaßt.

Tabelle 1, Teil 1:

| Substanz Nr. (Beispiel) | Dauerinkubation $IC_{50}$ (µg/ml) | 1h-Inkubation $IC_{50}$ (µg/ml) |
|---|---|---|
| Adriamycin | 0.02 | 0.04 |
| 8 | >1 | >1 |
| 9 | 0.6 | >10 |
| 10 | 0.9 | 6.5 |
| 11 | >1 | >1 |
| 12 | 0.5 | >1 |
| 13 | 0.03 | >1 |
| 14 | 0.024 | >1 |
| 15 | 0.004 | 0.02 |
| 16 | 0.006 | 0.02 |
| 17 | 0.004 | 0.09 |
| 18 | 0.13 | >1 |
| 19 | 0.11 | >1 |
| 21 | 0.5 | >1 |
| 24 | >1 | >1 |
| 25 | >1 | >1 |
| 27 | >1 | >1 |
| 28 | >1 | >1 |
| 29 | >10 | >1 |
| 30 | 0.5 | >1 |

Tabelle 1, Teil 2:

| Substanz Nr. (Beispiel) | Dauerinkubation $IC_{50}$ (µg/ml) | 1h-Inkubation $IC_{50}$ (µg/ml) |
|---|---|---|
| 33 | >1 | >1 |
| 34 | 0.026 | 0.09 |
| 35 | >1 | >1 |
| 37 | >1 | >1 |
| 38 | >1 | >1 |
| 40 | 0.85 | >1 |
| 42 | 0.16 | >1 |
| 44 | | >1 |
| 45 | | 0.29 |
| 46 | | 0.11 |
| 48 | | 0.095 |
| 49 | | >1 |
| 50 | 0.1 | |
| 51 | 0.4 | 0.68 |
| 52 | | 0.66 |
| 53 | 0.04 | 0.05 |

## Beispiele

Die Strukturen der hergestellten Verbindungen wurden mittels $^1H$- und $^{13}C$-NMR-Spektroskopie, unter Einbeziehung zweidimensionaler NMR-Methoden und andere Multi-Puls-Techniken sowie MS- und IR-Spektroskopie ermittelt. Der Verlauf der Reaktionen sowie die resultierenden Verbindungen wurden dünnschichtchromatographisch oder mit der HPLC-Technik untersucht.

Die folgenden Beispiele erläutern die Erfindung näher, ohne sie zu beschränken:

### Beispiel 1:

7,9-O-Phenyl-boronyl-β-rhodomycinon (1)

(Verbindung der Formel I mit $R^1 = R^4 = H$, $R^2$ und $R^3 = X$ mit X = Phenylboronyl)

Eine Lösung von β-Rhodomycinon (740 mg = 1.92 mmol) und Phenylboronsäure (360 mg = 2.95 mmol) in Toluol (150 ml) wurde zusammen mit aktiviertem 4Å Molekularsieb (3g) 9 Stunden am Rückfluß erhitzt, wobei die Reaktion dünnschichtchromatographisch verfolgt wurde (Laufmittel: Toluol/Methanol = 10:1).

Nach Abkühlen der Lösung wurde sie filtriert und das Lösungsmittel am Hochvakuum abgezogen. Das Präzipitat wurde in Chloroform gelöst und mit Petrolether auskristallisiert.
Ausbeute: 800 mg (1.7 mmol = 88%)

Beispiel 2:

7,9-O-Isopropyliden-β-rhodomycinon (2)

(Verbindung der Formel I mit $R^1 = R^4 = H$, $R^2$ und $R^3 = X$ mit X = Isopropyliden)

Eine Lösung von β-Rhodomycinon (300 mg = 0.78 mmol) in 30 ml trockenem N,N-Dimethylformamid (130 ml) und 2,2-Dimethoxypropan (36 ml) wurde mit para-Toluolsulfonsäure (150 mg) versetzt und 60 Stunden bei 50 °C und 320mbar am Rotationsverdampfer gerührt wobei weiteres 2,2-Dimethoxypropan (35 ml) zugegeben wurde. Nach dem Einengen der Lösung wurde mit Dichlormethan aufgenommen, mit wässriger Natriumhydrogencarbonatlösung ausgeschüttelt, getrocknet und eingeengt. Nach säulenchromatographischer Trennung (Laufmittel: Chloroform/Aceton/ Essigsäure/Wasser/Triethylamin = 95:5:1:0.25:0.1) wurde das Produkt kristallin erhalten.
Ausbeute: 150 mg (0.35 mmol = 45%)

Beispiel 3:

7-9-O-Benzyliden-β-rhodomycinon (3)

(Verbindung der Formel I mit $R^1 = R^4 = H$, $R^2$ und $R^3 = X$ mit X = Benzyliden)

Eine Lösung von β-Rhodomycinon (30 mg = 0,08 mmol) in trockenem N,N-Dimethylformamid (5 ml) und Benzaldehyddimethylacetal (2 ml) wurde mit para-Toluolsulfonsäure (10 mg) versetzt und eine Stunde bei 50 °C und 50 mbar am Rotationsverdampfer gerührt. Nach dem Einengen der Lösung wurde mit Dichlormethan aufgenommen, mit wässriger Natriumhydrogencarbonatlösung ausgeschüttelt, getrocknet und eingeengt.
Ausbeute: 35 mg (0.07 mmol = 90 %)
Die zwei exo/endo-Isomeren können chromatographisch (Laufmittel: Toluol/Methanol = 10:1) getrennt werden.

Beispiel 4:

10-0-Trifluoracetyl-β-rhodomycinon (4)

(Verbindung der Formel I mit $R^1 = R^2 = R^3 = H$, $R^4$ = Trifluoracetyl)

Eine Lösung von 7,9-0-Phenylboronyl-β-rhodomycinon (Verbindung 1) (230 mg = 0.49 mmol) in trockenem Dichlormethan (50 ml) wurde bei 0 °C mit Trifluoressigsäureanhydrid (4 ml = 283 mmol) und Triethylamin (0.2 ml) versetzt und 30 Minuten bei 0 °C gerührt.

Nach Abziehen des Lösungsmittels und mehrfachem Einengen mit Toluol wurde der Rückstand in Methanol gelöst und mit Salzsäure auf pH 2.5 gebracht, wobei nach 4-stündigem Rühren das Produkt ausfiel. Der Niederschlag wurde in Dichlormethan gelöst, mit Wasser gewaschen und nach Trocknen der organischen Phase eingeengt.
Ausbeute: 150 mg (0.31 mmol = 63 %)

Beispiel 5:

10-Tetrahydropyranyl-β-rhodomycinon (5)

(Verbindung der Formel I mit $R^1 = R^2 = R^3 = H$, $R^4$ = Struktur II mit $R^5 = R^6 = R^7 = R^8 = H$)

Zu einer Lösung aus 7,9-0-Phenylboronyl-β-rhodomycinon (500 mg = 1.06 mmol) wurde 3,4-Dihydro-2H-pyran (2.5 ml) in trockenem Dichlormethan (125 ml) mit 4Å Molekularsieb wurde eine katalytische Menge para-Toluolsulfonsäure (20 mg) gegeben und eine halbe Stunde bei Raumtemperatur gerührt. Nach Abfiltrieren des Molekularsiebes wurde das Filtrat mit wässriger Natriumhydrogencarbonatlösung gewaschen, mit Natriumsulfat getrocknet und das Lösungsmittel abdestilliert.

Dies Rohprodukt wurde am Hochvakuum getrocknet und dann zu einer Lösung von 2-Methyl-2,4-pentandiol (5 ml) in trokkenem Dichlormethan (40 ml) gegeben und mit katalytischen Mengen Eisessig (0.2

ml) 48 Stunden bei Raumtemperatur gerührt.

Anschließend wurde mit wässriger Natriumhydrogencarbonatlösung und mit Wasser ausgeschüttelt und über Natriumsulfat getrocknet. Eine säulenchromatographische Trennung (Laufmittel: Toluol/Methanol = 10:1) ergab das reine Produkt.
Ausbeute: 210 mg (0.45 mmol = 42%)

Beispiel 6:

10-0-Trimethylsilyl-β-rhodomycinon (6)

(Verbindung der Formel I mit $R^1 = R^2 = R^3 = H$, $R^4$ = Trimethylsilyl)

Zu einer Lösung von 7,9-0-Phenylboronyl-β-rhodomycinon (100 mg = 0.21 mmol) und Pyridin (85µl) in trockenem Dichlormethan und 4Å Molekularsieb wurde bei -40 °C Trifluormethansulfonsäure-trimethylsilylester (110µl) zugegeben und 1.5 Stunden gerührt.

Das Reaktionsgemisch wurde filtriert, mit wässriger Natriumhydrogencarbonatlösung ausgeschüttelt, über Natriumsulfat getrocknet und eingeengt.

Das Rohprodukt wurde zur Abspaltung des Boronylesters in trockenem Dichlormethan (10 ml) gelöst, mit 2-Methyl-2,4-pentandiol (0.55 ml) und Eisessig (0.1 ml) versetzt und 48h bei Raumtemperatur gerührt. Das Reaktionsgemisch wurde mit Wasser ausgeschüttelt, über Natriumsulfat getrocknet, eingeengt und säulenchromatographisch (Laufmittel: Dichlormethan/Essigsäure/Ameisensäure = 20:1:01) getrennt.
Ausbeute: 57 mg( 0.11 mmol = 52%)

Beispiel 7:

10-0-(4-0-Paranitrobenzoyl-3-N-trifluoracetyl-α-L-acosaminyl)-7,9-0-phenylboronyl-β-rhodomycinon (7)

(Verbindung der Formel I mit $R^1 = H$, $R^2$ und $R^3$ = Phenylboronyl, $R^4$ = Struktur II = α-L-Acosaminyl mit $R^5 = CH_3$, $R^6$ = OpNBz, $R^7$ = NHTFA, $R^8 = H$)

Zu einer Lösung aus 7,9-0-Phenylboronyl-β-rhodomycinon (Verbindung 1) (100 mg = 0.21 mmol) in trockenem Dichlormethan (8 ml) und 4Å Molekularsieb wurde eine Lösung aus 1,5-Anhydro-4-0-paranitrobenzoyl-2,3,6-tridesoxy-3-N-trifluoracetyl-L-arabino-hex-1-enit (87 mg = 0.23 mmol) in trockenem Dichlormethan (4 ml) gegeben und bei -40 °C unter Schutzgasatmosphäre (Argon) mit Trifluormethansulfonsäure-trimethylsilylester (40µl = 0.22 mmol) versetzt. Nach Aufwärmen auf -20 °C wurde noch 4 Stunden bei dieser Temperatur gerührt und anchließend die Reaktion beendet, indem die Lösung mit Triethylamin (80µl) versetzt wurde. Nach Filtration wurde die Lösung mit wässriger Natriumhydrogencarbonatlösung gewaschen, über Natriumsulfonat getrocknet, eingeengt und mehrfach mit Toluol abgezogen.
Roh-Ausbeute: 180 mg (quantitativ)

Beispiel 8:

10-0-(4-0-Paranitrobenzoyl-3-N-trifluor-acetyl-α-L-acosaminyl)-β-rhodomycinon (8)

(Verbindung der Formel I mit $R^1 = R^2 = R^3 = H$, $R^4$ = Struktur II = α-L-Acosaminyl mit $R^5 = CH_3$, $R^6$ = OpNBz, $R^7$ = NHTFA, $R^8 = H$)

Eine Lösung aus dem Rohgemisch von Verbindung 7 (180 mg = 0.21 mmol) in trockenem Dichlormethan (20 ml) wurde mit 2-Methyl-2,4-pentandiol (3.2 ml) und Eisessig (0.2 ml) 24 Stunden bei Raumtemperatur gerührt. Zur Aufarbeitung wurde mehrfach mit Toluol abgezogen und aus Diethylether/Petrolether 150 mg Rohprodukt auskristallisiert, welches säulenchromatographisch (Laufmittel: Dichlormethan/Aceton/Ameisensäure = 20:1:0.2) gereinigt wurde.
Ausbeute: 120 mg (0.16 mmol = 75%)

Beispiel 9:

10-0-(4-0-Paranitrobenzoyl-3-N-trifluoracetyl-α-L-daunosaminyl)-β-rhodomycinon (9)

(Verbindung der Formel I mit $R^1 = R^2 = R^3 = H$, $R^4$ = Struktur II = α-L-Daunosaminyl mit $R^5 = CH_3$, $R^6$ = OpNBz, $R^7$ = NHTFA, $R^8$ = H)

Entsprechend der Vorschrift von Beispiel 7 wurden 7,9-0-Phenyl-boronyl-β-rhodomycinon (Verbindung 1) (150 mg = 0.318 mmol) und 1,4-Di-0-paranitrobenzoyl-3-N-trifluoracetyl-L-daunosamin (190 mg = 0.35 mmol) mit Trifluormethansulfonsäure-trimethylsilylester (0.11 ml = 0.61 mmol) umgesetzt.

Das dabei entstandene Rohprodukt wurde zwecks Abspaltung des Boronsäureesters gemäß der Vorschrift von Beispiel 8 entblockiert.
Ausbeute: 180 mg (0.24 mmol = 75%)

Beispiel 10:

10-0-α-L-Acosaminyl-β-rhodomycinon (10)

(Verbindung der Formel I mit $R^1 = R^2 = R^3 = H$, $R^4$ = Struktur II = α-L-Acosaminyl mit $R^5 = CH_3$, $R^6$ = OH, $R^7 = NH_2$, $R^8$ = H)

Eine Lösung aus Verbindung 8 (36 mg = 47 µmol) in Methanol (2 ml) und 0.5n Natronlauge (2 ml) wurde 30 Minuten bei Raumtemperatur gerührt und anschließend mit Salzsäure auf pH 2.5 gebracht. Sodann wurde mehrfach mit Chloroform ausgeschüttelt. Die wässrige Phase wurde mit Natriumhydrogencarbonatlösung neutralisiert und mehrfach mit Chloroform ausgeschüttelt. Diese Chloroformphase wurde mit Natriumsulfat getrocknet und eingeengt.
Ausbeute: 17.5 mg (34 µmol = 72 %)

Beispiel 11:

10-0-α-L-Daunosaminyl-β-rhodomycinon (11)

(Verbindung der Formel I mit $R^1 = R^2 = R^3 = H$, $R^4$ = Struktur II = α-L-Daunosaminyl mit $R^5 = CH_3$, $R^6$ = OH, $R^7$ = NHz, $R^8$ = H

Gemäß den bei Beispiel 10 beschriebenen Bedingungen wurde Verbindung 9 (53 mg = 70 µmol) entblockiert und aufgearbeitet.
Ausbeute: 23 mg (45 µmol = 64%)

Beispiel 12:

10-0-(3-N,N-Dimethyl-α-L-acosaminyl)-β-rhodomycinon (12)

(Verbindung der Formel I mit $R^1 = R^2 = R^3 = H$, $R^4$ = Struktur II = α-L-Acosaminyl mit $R^5 = CH_3$, $R^6$ = OH, $R^7 = N(CH_3)_2$, $R^8$ = H)

Eine Lösung von Verbindung 10 (23 mg = 42 µmol) in Methanol (2 ml) wird mit Natriumcyanoborhydrid (16 mg = 250 µmol) und Formaldehyd (63µl, 37%ig = 860 µmol) zwei Stunden bei Raumtemperatur gerührt. Mit Salzsäure wird anschließend neutralisiert und chromatographisch gereinigt (Laufmittel: Chloroform/Methanol/Essigsäure/Wasser/Triethylamin = 80:20:10: 4:0.2).
Ausbeute: 15 mg (28 µmol = 67%)

Beispiel 13:

7-0-(4-0-Paranitrobenzoyl-3-N-trifluoracetyl-α-L-acosaminyl)-10-0-trifluoracetyl-β-rhodomycinon (13)

(Verbindung der Formel I mit $R^1 = R^3 = H$, $R^4$ = Trifluoracetyl, $R^2$ = Struktur II = α-L-Acosaminyl mit $R^5 = CH_3$, $R^6$ = OpNBz, $R^7$ = NHTFA, $R^8$ = H)

Verbindung 4 (90 mg = 0.19 mmol) wurde unter den bei Beispiel 7 beschriebenen Bedingungen umgesetzt.
Roh-Ausbeute: 150 mg (0.18 mmol = 95%)

Beispiel 14:

7-0-(4-0-Paranitrobenzoyl-3-N-trifluoracetyl-α-L-acosaminyl)-β-rhodomycinon (14)

(Verbindung der Formel I mit $R^1 = R^3 = R^4 = H$, $R^2$ = Struktur II = α-L-Acosaminyl mit $R^5 = CH_3$, $R^6$ = OpNBz, $R^7$ = NHTFA, $R^8$ = H)

Eine Lösung aus Verbindung 13 (100 mg = 0.12 mmol) in Methanol (5 ml) wurde mit 0.1n Natronlauge auf pH = 10 gebracht und 10 Minuten gerührt.
Das Reaktionsgemisch wurde mit verdünnter Salzsäure neutralisiert und das Lösungsmittel wurde abdestilliert. Der Rückstand wurde mit Dichlormethan aufgenommen, mit Wasser gewaschen, über Natriumsulfat getrocknet und eingeengt.
Roh-Ausbeute: 85 mg (0.11 mmol = 92%)

Beispiel 15:

7-0-α-L-Acosaminyl-β-rhodomycinon (15)

(Verbindung der Formel I mit $R^1 = R^3 = R^4 = H$, $R^2$ = Struktur II = α-L-Acosaminyl mit $R^5$-$CH_3$, $R^6$ = OH, $R^7 = NH_2$, $R^8$ = H)

Gemäß den bei Beispiel 10 beschriebenen Bedingungen wurde Verbindung 14 (27 mg = 35 µmol) entblockiert und aufgearbeitet.
Ausbeute: 15 mg (29 µmol = 83%)

Beispiel 16:

7-0-(3-N,N-Dimethyl-α-L-acosaminyl)-β-rhodomycinon (16)

(Verbindung der Formel I mit $R^1 = R^3 = R^4 = H$, $R^2$ = Struktur II = α-L-Acosaminyl mit $R^5 = CH_3$, $R^6$ = OH, $R^7 = N(CH_3)_2$, $R^8$ = H)

Verbindung 15 (15 mg - 29 µmol) wurde nach den Bedingungen von Beispiel 12 umgesetzt.
Ausbeute: 13 mg (24 µmol = 83%)

Beispiel 17:

7-O-(3-N-Cyanomethyl-α-L-daunosaminyl)-β-rhodomycinon (17)

(Verbindung der Formel I mit $R^1 = R^3 = R^4 = H$, $R^2$ = Struktur II = α-L-Daunosaminyl mit $R^5 = CH_3$, $R^6$ = OH, $R^7 = NH(CH_2CN)$, $R^8$ = H)

7-O-α-L-Daunosaminyl-β-rhodomycinon (200 mg = 0.39 mmol) wurde in trockenem N,N-Dimethylformamid (30 ml) gelöst und nach der Zugabe von Triethylamin (0.16 ml) und Jodacetonitril (0.283 ml) bei Raumtemperatur 15 h gerührt. Anschließend wurde die Reaktionsmischung im Hochvakuum eingedampft und säulenchromatographisch unter 50 g Kieselgel gereinigt (Elutionsmittel: Dichlormethan/Aceton/ Essigsäure = 5:2:1)

Ausbeute: 157 mg (0.28 mmol = 73 %)
MS: $M + H^+$ = 555

Beispiel 18:

7-O-(4-O-Paranitrobenzoyl-3-N-trifluoracetyl-α-L-daunosaminyl)-10-O-trifluoracetyl-β-rhodomycinon (18)

(Verbindung der Formel I mit $R^1 = R^3 = H$, $R^4$ = Trifluoracetyl, $R^2$ = Struktur II = α-L-Daunosaminyl mit $R^5 = CH_3$, $R^6$ = OpNBz, $R^7$ = NHTFA, $R^8$ = H)

10-O-Trifluoracetyl-β-rhodomycinon (500 mg = 1.03 mmol) wurde in einer Mischung aus Dichlormethan und Aceton (1:1; 40 ml) gelöst. Die Lösung wurde mit Molekularsieb (4Å, getrocknetes Pulver, 500 mg) und 1,4-Di-O-paranitrobenzoyl-3-N-trifluoracetyl-L-daunosamin (1.1g = 2.06 mmol) versetzt und unter Luftfeuchtigkeitsausschluß auf -30 °C abgekühlt. Zu der gerührten Suspension wurde Trifluormethansulfonsäure-trimethylsilylester (0.9 ml = 5.15 mmol) zugetropft. Nach 2 h wurde Triethylamin (0.8 ml) zugegeben. Die Mischung wurde bei Raumtemperatur filtriert und das Filtrat dreimal mit Eiswasser ausgewaschen. Die organische Phase wurde über Natriumsulfat getrocknet und im Wasserstrahlpumpenvakuum eingedampft. Das resultierende Rohprodukt wurde säulenchromatographisch (Kieselgel 60/35-70 Fa. Amicon, Elutionsmittel: Dichlormethan/ Petrolether/ Aceton = 5:5:1) gereinigt.
Ausbeute: 750 mg (0.88 mmol = 85 %)

Beispiel 19:

7-O-(4-O-Paranitrobenzoyl-3-N-trifluoracetyl-α-L-daunosaminyl)-β-rhodomycinon (19)

(Verbindung der Formel I mit $R^1 = R^3 = R^4 = H$, $R^2$ = Struktur II = α-L-Daunosaminyl mit $R^5 = CH_3$, $R^6$ = OpNBz, $R^7$ = NHTFA, $R^8$ = H)

Verbindung 19 (660 mg = 0.77 mmol) wurde in 30 ml einer Mischung aus Chloroform und Methanol 1:1 gelöst. Anschließend wurde tropfenweise 0.01 n wässrige Natronlauge (30 ml) zugegeben (pH 7.5). Nach 3 h wurde die Lösung mit 0.1 n wässriger Salzsäure neutralisiert und es wurde in Vakuum eingedampft. Das dünnschichtchromatographisch einheitliche Produkt wurde säulenchromatographisch über 200 g Kieselgel 60/35-70 Fa. Amicon (Laufmittel: Dichlormethan/Aceton = 15:1 bis 5:1) gereinigt.
Ausbeute: 570 mg (0.75 mmol = 97 %))

Beispiel 20:

7-O-(4-O-Paranitrobenzoyl-3-N-trifluoracetyl-α-L-daunosaminyl)-10-O-tetrahydropyranyl-β-rhodomycinon (20)

(Verbindung der Formel I mit $R^1 = R^3 = H$, $R^2$ = Struktur II = α-L-Daunosaminyl mit $R^5 CH_3$, $R^6$ = OpNBz, $R^7$ = NHTFA, $R^8$ = H; $R^4$ = Struktur II mit $R^5 = R^6 = R^7 = R^8 = H$)

Verbindung 19 (150 mg = 0.2 mmol) wurde in Dichlormethan (20 ml) gelöst. Unter Rühren wurde Paratoluolsulfonsäure (10 mg) und 3,4-Dihydro-2H-pyran (2 ml) bei Raumtemperatur zugegeben. Nach 24 h wurde die Reaktionsmischung dreimal mit insgesamt 100 ml Eiswasser ausgewaschen. Die organische Phase wurde über Natriumsulfat getrocknet und eingedampft. Das Rohprodukt wurde über Kieselgel gereinigt (Elutionsmittel: Dichlormethan/Petrolether/ Aceton = 5:5:1)
Ausbeute: 150 mg (0.178 mmol = 89 %)

Beispiel 21:

7-O-α-L-Daunosaminyl-10-O-tetrahydropyranyl-β-rhodomycinon (21)

(Verbindung der Formel I mit $R^1 = R^3 = H$, $R^2$ = Struktur II = α-L-Daunosaminyl mit $R^5 = CH_3$, $R^6 = OH$, $R^7 = NH_2$, $R^8 = H$; $R^4$ = Struktur II mit $R^5 = R^6 = R^7 = R^8 = H$)

Verbindung 20 (150 mg = 0.17 mmol) wurde in Chloroform/Methanol 1:1 (20 ml) bei Raumtemperatur gelöst und mit 1 n wässriger NaOH-Lösung (1 ml) versetzt. Nach 1 h Rühren wurde die Lösung mit 1 n Salzsäure neutralisiert und eingedampft. Der Rückstand wurde noch säulenchromatographisch (Kieselgel, Elutionsmittel: Dichlormethan/Methanol 5:1) gereinigt.
Ausbeute: 87 mg (0.145 mmol = 82 %)

Beispiel 22:

7-O-(3-Azido-4-O-paranitrobenzoyl-2,3,6-tridesoxy-α-L-arabinohexopyranosyl)-10-O-trifluoracetyl-β-rhodomycinon (22)

(Verbindung der Formel I mit $R^1 = R^3 = H$, $R^2$ = Struktur II = α-L-2,3,6-Tridesoxyarabinohexopyranosyl mit $R^5 = CH_3$, $R^6$ = OpNBz, $R^7 = N_3$, $R^8 = H$; $R^4$ = Trifluoracetyl)

Verbindung 4 (100 mg = 0.21 mmol) wurde in einer Mischung aus Dichlormethan und Aceton 5:1 (10 ml) gelöst und mit Molekularsieb 4Å (100 mg) als getrocknetes Pulver versetzt. Nach der Zugabe von 3-Azido-2,3,6-tridesoxy-4-O-paranitrobenzoyl-L-arabino-1-hex-1-enit (127 mg = 0.42 mmol) gelöst in Dichlormethan (5 ml) wurde die Mischung auf -30 °C gekühlt und mit Trifluormethansulfonsäure-trimethylsilylester (46 mg = 0.21 mmol) versetzt.
Die Reaktionstemperatur wurde nach 4 h auf -10 °C heraufgesetzt. Dann wurde weiteres 3-Azido-2,3,6-tridesoxy-4-O-paranitrobenzoyl-L-arabino-1-hex-1-enit (36 mg = 0.21 mmol) gelöst in Dichlormethan (25 ml) zugegeben. Nach weiteren 16 h wurde die Reaktionsmischung mit Triethylamin neutralisiert, abfiltriert und eingedampft. Das erhaltene Rohprodukt wurde säulenchromatographisch gereinigt (Kieselgel: Elutionsmittel: Dichlormethan)
Ausbeute: 127 mg (0.16 mmol = 77 %)

Beispiel 23:

7-O-(3-Azido-2,3,6-tridesoxy-α-L-arabinohexopyranosyl)-β-rhodomycinon (23)

(Verbindung der Formel I mit $R^1 = R^3 = R^4 = H$, $R^2$ = Struktur II = α-L-2,3,6-tridesoxy-arabinohexopyranosyl mit $R^5 = CH_3$, $R^6 = OH$, $R^7 = N_3$, $R^8 = H$)

Verbindung 22 (100 mg = 0.13 mmol) wurde in Chloroform/Methanol 1:1 (10 ml) gelöst und mit 1 n NaOH-Lösung (0.5 ml) unter Rühren bei Raumtemperatur versetzt. Nach 1 h wird der Reaktionsansatz mit 1 n HCl neutralisiert. Nach Abdampfen der Lösungsmittel wird das Produkt mit Toluol versetzt und bis zur Trockne wieder eingedampft. Das resultierende Produkt wird in Chloroform/Methanol 3:1 gelöst und die unlöslichen Bestandteile werden abfiltriert. Nach dem Einengen des Filtrates wird das Rohprodukt säulenchromatographisch über 50 g Kieselgel gereinigt (Dichlormethan/Aceton 10:1)
Ausbeute: 53 mg (0.098 mmol = 75 %)

Beispiel 24:

7-O-(3,4-Di-O-acetyl-2,6-didesoxy-2-iodo-α-L-talopyranosyl)-10-O-tetrahydropyranyl-β-rhodomycinon (24)

(Verbindung der Formel I mit $R^1 = R^3 = H$, $R^2$ = Struktur II = 2,6-Didesoxy-α-L-Talopyranosyl mit $R^5 = CH_3$, $R^6 = R^7 = OAc$, $R^8 = J$; $R^4$ = Struktur II mit $R^5 = R^6 = R^7 = R^8 = H$)

Zu einer Lösung aus Verbindung 5 (30 mg = 0.06 mmol) und 3,4-Di-O-acetyl-1,5-anhydro-2,6-didesoxy-L-lyxo-hex-1-enit (26 mg = 0.12 mmol) in 3 ml trockenem Acetonitril mit aktiviertem 3 Å Molekularsieb wurde unter Schutzgasatmosphäre (Argon) N-Jodsuccinimid (36 mg = 0.16 mmol) bei -10 °C

gegeben und während der Reaktionszeit von 4 Tagen auf Raumtemperatur gebracht. Nach Abfiltrieren wurde mit Dichlormethan verdünnt und die organische Phase zunächst mit wässriger Natriumthiosulfatlösung, dann mit Natriumhydrogencarbonatlösung und dann mit Wasser ausgeschüttelt, getrocknet und chromatographisch (Laufmittel: Toluol/Methanol = 10:1) gereinigt.
Nicht optimierte Ausbeute: 10 mg (0.013 mmol = 20 %)

Beispiel 25:

7-O-(3,4-Di-O-acetyl-2,6-didesoxy-2-iodo-α-L-mannopyranosyl)-10-O-tetrahydropyranyl-β-rhodomycinon (25)

(Verbindung der Formel I mit $R^1 = R^3$, $R^2$ = Struktur II = 2,6-Didesoxy-α-L-Mannopyranosyl mit $R^5 = CH_3$, $R^6 = R^7$ = OAc, $R^8$ = J; $R^4$ = Struktur II mit $R^5 = R^6 = R^7 = R^8$ = H)

Verbindung 5 (70 mg = 0.15 mmol) wurde analog zu Beispiel 24 mit 3,4-Di-O-acetyl-1,5-anhydro-2,6-didesoxy-L-arabino-hex-1-enit umgesetzt und aufgearbeitet.
Nicht optimierte Ausbeute: 45 mg (0,056 mmol = 37 %)

Beispiel 26:

7-O-(3,4-Di-O-acetyl-2,6-didesoxy-2-iodo-α-L-mannopyranosyl)-β-rhodomycinon (26)

(Verbindung der Formel I mit $R^1 = R^3 = R^4$ = H, $R^2$ = Struktur II = 2,6-Didesoxy-α-L-Mannopyranosyl mit $R^5 = CH_3$, $R^6 = R^7$ = OAc, $R^8$ = J)

Eine Lösung aus Verbindung 25 (34 mg = 0.04 mmol) in 2 ml Methanol wurde mit sauren Ionenaustauscher (Dowex 50 WX8) versetzt und 24 Stunden gerührt. Nach Abfiltrieren des Ionenaustauschers wurde chromatographisch gereinigt (Laufmittel: Dichlormethan/Aceton/Ameisensäure = 20:1:01.)
Nicht optimierte Ausbeute: 13 mg (0,018 mmol = 45 %)

Beispiel 27:

7,10-Di-O-(4-O-paranitrobenzoyl-3-N-trifluoracetyl-α-L-daunosaminyl)-β-rhodomycinon (27)

(Verbindung der Formel I mit $R^1 = R^3$ = H, $R^2 = R^4$ = Struktur II = α-L-Daunosaminyl mit $R^5 = CH_3$, $R^6$ = OpNBz, $R^7$ = NHTFA, $R^8$ = H)

Verbindung 9 wurde analog zu Beispiel 7 mit 1,4-Di-O-paranitrobenzoyl-3-N-trifluoracetyl-L-daunosamin und Trifluormethansulfonsäuretrimethylsilylester umgesetzt und aufgearbeitet.

Beispiel 28:

7,10-Di-O-(4-O-acetyl-3-N-trifluoracetyl-α-L-daunosaminyl)-β-rhodomycinon (28)

(Verbindung der Formel I mit $R^1 = R^3$ = H, $R^2 = R^4$ = Struktur II = α-L-Daunosaminyl mit $R^5 = CH_3$, $R^6$ = OAc, $R^7$ = NHTFA, $R^8$ = H)

Verbindung 1 wurde analog zu Beispiel 7 mit 1,4-Di-O-acetyl-3-N-trifluoracetyl-L-daunosamin und Trifluormethansulfonsäure-trimethylsilylester umgesetzt, dann analog Beispiel 8 an Position 7 und 9 entblokkiert und erneut analog zu Beispiel 7 mit 1,4-Di-O-acetyl-3-N-trifluoracetyl-L-daunosamin und Trifluormethansulfonsäuretrimethylsilylester umgesetzt.

Beispiel 29:

7,10-Di-O-α-L-daunosaminyl-β-rhodomycinon (29)

(Verbindung der Formel I mit $R^1 = R^3 = H$, $R^2 = R^4$ = Struktur II = α-L-Daunosaminyl mit $R^5 = CH_3$, $R^6 = OH$, $R^7 = NH_2$, $R^8 = H$)

Gemäß den bei Verbindung 10 beschriebenen Bedingungen wurde Verbindung 27 entblockiert und aufgearbeitet.

Beispiel 30:

7,10-O-Di-(3-N-Cyanomethyl-α-L-daunosaminyl)-β-rhodomycinon (30)

(Verbindung der Formel I mit $R^1 = R^3 = H$, $R^2 = R^4$ = Struktur II = α-L-Daunosaminyl mit $R^5 = CH_3$, $R^6 = OH$, $R^7 = NH(CH_2CN)$, $R^8 = H$)

Die im Titel beschriebene Verbindung wurde ausgehend von Verbindung 29 (132 mg = 0.2 mmol) und Jodacetonitril 0.15 ml, wie bereits in Beispiel 17 beschrieben, analog hergestellt.
Ausbeute: 76,6 mg (0.11 mmol = 53 % )
MS: $M + H^+$ = 723

Beispiel 31:

7,10-Di-O-(4-0-paranitrobenzoyl-3-N-trifluoracetyl-α-L-acosaminyl)-β-rhodomycinon (31)

(Verbindung der Formel I mit $R^1 = R^3 = H$, $R^2 = R^4$ = Struktur II = α-L-Acosaminyl mit $R^5 = CH_3$, $R^6$ = OpNBz, $R^7$ = NHTFA, $R^8 = H$)

Verbindung 8 wurde analog zu Beispiel 7 umgesetzt und aufgearbeitet.

Beispiel 32:

7,10-Di-O-(4-O-acetyl-3-N-trifluoracetyl-α-L-acosaminyl)-β-rhodomycinon (32)

(Verbindung der Formel I mit $R^1 = R^3 = H$, $R^2 = R^4$ = Struktur II = α-L-Acosaminyl mit $R^5 = CH_3$, $R^6$ = OAc, $R^7$ = NHTFA, $R^8 = H$)

Verbindung 1 wurde analog dem Beispiel 28 mit 1,4-Di-O-acetyl-3-N-trifluoracetyl-L-acosamin umgesetzt.

Beispiel 33:

7,10-Di-O-α-L-acosaminyl-β-rhodomycinon (33)

(Verbindung der Formel I mit $R^1 = R^3 = H$, $R^2 = R^4$ = Struktur II = α-L-Acosaminyl mit $R^5 = CH_3$, $R^6 = OH$, $R^7 = NH_2$, $R^8 = H$)

Verbindung 31 wurde analog den bei Verbindung 10 beschriebenen Bedingungen entblockiert und aufgearbeitet.

Beispiel 34:

7,10-Di-O-(3-N,N-dimethyl-α-L-acosaminyl-β-rhodomycinon (34)

(Verbindung der Formel I mit $R^1 = R^3 = H$, $R^2 = R^4$ = Struktur II mit $R^5 = CH_3$, $R^6 = OH$, $R^7 = N(CH_3)_2$, $R^8 = H$)

Verbindung 33 wurde analog den Bedingungen von Beispiel 12 umgesetzt.

Beispiel 35:

7-0-(4-0-Paranitrobenzoyl-3-N-trifluoracetyl-α-L-acosaminyl)-10-0-(4-0-paranitrobenzoyl-3-N-trifluoracetyl-α-L-daunosaminyl)-β-rhodomycinon (35)

(Verbindung der Formel I mit $R^1 = R^3 = H$, $R^2$ = Struktur II = α-L-Acosaminyl mit $R^5 = CH_3$, $R^6$ = OpNBz, $R^7$ = NHTFA, $R^8$ = H; $R^3$ = Struktur II = α-L-Daunosaminyl mit $R^5 = CH_3$, $R^6$ = OpNBz, $R^7$ NHTFA, $R^8$ = H)

Verbindung 9 wurde analog zu Beispiel 7 umgesetzt und aufgearbeitet.

Beispiel 36:

7-O-(4-O-Acetyl-3-N-trifluoracetyl-α-L-acosaminyl-10-0-(4-0-paranitrobenzoyl-3-N-trifluoracetyl-α-L-daunosaminyl)-β-rhodomycinon (36)

(Verbindung der Formel I mit $R^1 = R^3 = H$, $R^2$ = Struktur II = α-L-Acosaminyl mit $R^5 = CH_3$, $R^6$ = OAc, $R^7$ = NHTFA, $R^8$ = H; $R^4$ = Struktur II = α-L-Daunosaminyl mit $R^5 = CH_3$, $R^6$ = OpNBz, $R^7$ = NHTFA, $R^8$ = H)

Verbindung 9 wurde analog zu Beispiel 7 mit 1,4-Di-O-acetyl-3-N-trifluoracetyl-L-acosamin umgesetzt und aufgearbeitet.

Beispiel 37:

7-O-α-L-Acosaminyl-10-0-α-L-daunosaminyl-β-rhodomycinon (37)

(Verbindung der Formel I mit $R^1 = R^3 = H$, $R^2$ = Struktur II = α-L-Acosaminyl mit $R^5 = CH_3$, $R^6$ = OH, $R^7 = NH_2$, $R^8$ = H; $R^4$ = Struktur II = α-L-Daunosaminyl mit $R^5 = CH_3$, $R^6$ = OH, $R^7 = NH_2$, $R^8$ = H)

Gemäß den bei Verbindung 10 beschriebenen Bedingungen wurde Verbindung 35 entblockiert und aufgearbeitet.

Beispiel 38:

7-0-(4-0-Benzoyl-α-L-rhodosaminyl)-10-0-(4-0-paranitrobenzoyl-3-N-trifluoracetyl-α-L-acosaminyl)-β-rhodomycinon (38)

(Verbindung der Formel I mit $R^1 = R^3 = H$, $R^2$ = Struktur II = α-L-Rhodosaminyl mit $R^5 = CH_3$, $R^6$ = OBz, $R^7 = N(CH_3)_2$, $R^8$ = H, $R^4$ = Struktur II = α-L-Acosaminyl mit $R^5 = CH_3$, $R^6$ = OpNBz, $R^7$ NHTFA, $R^8$ = H)

7-0-(4-0-Benzoyl-α-L-rhodosaminyl)-β-rhodomycinon wurde analog dem Beispiel 7 umgesetzt und aufgearbeitet.

Beispiel 39:

10-0-(4-0-Paranitrobenzoyl-3-N-trifluoracetyl-α-L-acosaminyl)-7-0-α-L-rhodosaminyl)-β-rhodomycinon (39)

(Verbindung der Formel I mit $R^1 = R^3 = H$, $R^2$ = Struktur II = α-L-Rhodosaminyl mit $R^5 = CH_3$, $R^6$ = OH, $R^7$ = N-$(CH_3)_2$, $R^8$ = H; $R^4$ = Struktur II = α-L-Acosaminyl mit $R^5 = CH_3$, $R^6$ = OpNBz, $R^7$ NHTFA, $R^8$ = H)

7-0-α-L-Rhodosaminyl-β-rhodomycinon wurde analog dem Beispiel 7 umgesetzt und aufgearbeitet.

Beispiel 40:

10-0-α-L-Acosaminyl-7-0-α-L-rhodosaminyl-β-rhodomycinon (40)

(Verbindung der Formel I mit $R^1 = R^3 = H$, $R^2$ = Struktur II = α-L-Rhodosaminyl mit $R^5 = CH_3$, $R^6 = OH$, $R^7$ N-$(CH_3)_2$, $R^8 = H$; $R^4$ = Struktur II = α-L-Acosaminyl mit $R^5 = CH_3$, $R^6 = OH$, $R^7 = NH_2$, $R^8 = H$)

Verbindung 38 wurde analog der bei Verbindung 10 beschriebenen Bedingungen entblockiert und aufgearbeitet.

Beispiel 41:

10-0-(4-0-Acetyl-2,3,6-tridesoxy-α-L-erythro-hex-2-eno-pyranosyl)-7-0-(4-0-paranitrobenzoyl-3-N-trifluoracetyl-α-L-daunosaminyl)-β-rhodomycinon (41)

(Verbindung der Formel I mit $R^1 = R^3 = H$, $R^2$ = Struktur II = α-L-Daunosaminyl mit $R^5 = CH_3$, $R^6$ = OpNBz, $R^7$ = NHTFA, $R^8 = H$; $R^4$ = Struktur IV = α-L-erythro-hex-2-eno-pyranosyl mit $R^6$ = OAc)

Verbindung 19 (400 mg = 0.52 mmol) wurde in Dichlormethan/Aceton (50 ml) gelöst. Nach der Zugabe von 3,4-Di-0-acetyl-L-rhamnal (225 mg = 1.0 mmol) und Molekularsieb 4Å (getrocknetes Pulver) (400 mg) wurde die Suspension auf -40 °C abgekühlt. Anschließend wurden Trifluormethansulfonsäuretrimethylsilylester (60 mg = 2.6 mmol) zugetropft. Nach 2 h wurde der Suspension weiteres 3,4-Di-0-acetyl-L-rhamnal (225 mg) zugegeben. Die Reaktionsmischung wurde nach 24 h mit Triethylamin neutralisiert und wie üblich aufgearbeitet.
Ausbeute: 180 mg (0.2 mmol = 37 %)

Beispiel 42:

10-0-(2,3,6-Tridesoxy-α-L-erythro-hex-2-enopyranosyl)-7-0-α-L-daunosaminyl-β-rhodomycinon (42)

(Verbindung der Formel I mit $R^1 = R^3 = H$, $R^2$ = Struktur II = α-L-Daunosaminyl mit $R^5 = CH_3$, $R^6$ OH, $R^7 = NH_2$, $R^8 = H$; $R^4$ = Struktur IV = α-L-erythro-hex-2-eno-pyranosyl mit $R^6 = OH$)

Verbindung 41 (100 mg = 0.13 mmol) wurde in Methanol (10 ml) gelöst und mit 1 n wässriger NaOH-Lösung (10 ml) versetzt. Die Reaktionsmischung wurde 24 h bei Raumtemperatur gerührt und anschließend mit 1 n Salzsäure neutralisiert. Nach Abdampfen des Lösungsmittels wurde der Rückstand säulenchromatographisch über Kieselgel (Elutionsmittel: Chloroform/Methanol 5:1) gereinigt.
Ausbeute: 60 mg ((0.096 mmol = 74 %)
FAB-MS: m/z 628 = $M + H^+$

Beispiel 43:

7,9,10-Tri-O-(4-O-paranitrobenzoyl-3-N-trifluoracetyl-α-L-acosaminyl)-β-rhodomycinon (43)

(Verbindung der Formel I mit $R^1 = H$, $R^2 = R^3 = R^4$ = Struktur II = α-L-Acosaminyl mit $R^5 = CH_3$, $R^6$ = OpNBz, $R^7$ = NHTFA, $R^8 = H$)

Verbindung 8 wurde analog Beispiel 7, aber mit der doppelten equimolaren Menge an 1,5-Anhydro-4-O-paranitrobenzoyl-2,3,6-tridesoxy-3-N-trifluoracetyl-L-arabino-hex-1-enit bei 0 °C umgesetzt.

Beispiel 44:

7,9,10-Tri-O-$\alpha$-L-acosaminyl-$\beta$-rhodomycinon (44)

(Verbindung der Formel I mit $R^1 = H$, $R^2 = R^3 = R^4$ = Struktur II = $\alpha$-L-Acosaminyl mit $R^5 = CH_3$, $R^6 = OH$, $R^7 = NH_2$, $R^8 = H$)

Verbindung 43 wurde analog der bei der Verbindung 1O beschriebenen Bedingungen entblockiert und aufgearbeitet.
Schmelzpunkt: 225 ° C

Beispiel 45:

7-O-(3-N-Trifluoracetyl-$\alpha$-L-acosaminyl)-ß-rhodomycinon (45)

(Verbindung der Formel I mit $R^1 = R^3 = R^4 = H$, $R^2$ = Struktur II = $\alpha$-L-Acosaminyl mit $R^5 = CH_3$, $R^6 = OH$, $R^7$ = NHTFA, $R^8 = H$)

Eine Lösung aus Verbindung 13 (27 mg = 32 $\mu$mol) in trockenem Methanol (2 ml) und 2 Tropfen methanolischer Natriummethanolatlösung (33 %ig) wurde eine Stunde bei Raumtemperatur gerührt, mit saurem Ionenaustauscher (Dowex WX8) neutralisiert, filtriert, eingeengt und chromatographisch gereinigt (Laufmittel: Dichlormethan/Methanol/Ameisensäure = 10 : 1 : 0.2).
Ausbeute: 15 mg (25 $\mu$mol = 78 %)

Beispiel 46:

7-O-$\alpha$-L-Ristosaminyl-$\beta$-rhodomycinon (46)

(Verbindung der Formel I mit $R^1 = R^3 = R^4 = H$, $R^2$ = Struktur II = $\alpha$-L-Ristosaminyl mit $R^5 = CH_3$, $R^6 = OH$, $R^7 = NH_2$, $R^9 = H$)

Verbindung 4 wurde unter den bei Beispiel 7 beschriebenen Bedingungen mit 1,5-Anhydro-4-O-paranitrobenzoyl-2,3,6-tridesoxy-3-N-trifluoracetyl-L-ribo-hex-1-enit umgesetzt und unter den in Beispiel 10 beschriebenen Bedingungen entblockiert.
Schmelzpunkt: 217 ° C
MS: $M + H^+ = 516$

Beispiel 47:

7-O-(3-N-Trifluoracetyl-$\alpha$-L-ristosaminyl)-$\beta$-rhodomycinon (47)

(Verbindung der Formel I mit $R^1 = R^3 = R^4 = H$, $R^2$ = Struktur II = $\alpha$-L-Ristosaminyl mit $R^5 = CH_3$, $R^6 = OH$, $R^7$ = NHTFA, $R^8 = H$)

Verbindung 4 wurde unter den bei Beispiel 7 beschriebenen Bedingungen mit 1,5-Anhydro-4-O-paranitrobenzoyl-2,3,6-tridesoxy-3-N-trifluoracetyl-L-ribo-hex-1-enit umgesetzt und unter den in Beispiel 45 beschriebenen Bedingungen entblockiert.
Schmelzpunkt: 178 ° C
MS: $M + H^+ = 612$

Beispiel 48:

7-O-(4-O-Benzyl-α-L-acosaminyl)-β-rhodomycinon (48)

(Verbindung der Formel I mit $R^1 = R^3 = R^4 = H$, $R^2$ = Struktur II = α-L-Acosaminyl mit $R^5 = CH_3$, $R^6 = OBn$, $R^7 = NH_2$, $R^8 = H$)

Verbindung 4 wurde unter den bei Beispiel 7 beschriebenen Bedingungen mit 1,5-Anhydro-4-O-benzyl-2,3,6-tridesoxy-3-N-trifluoracetyl-L-arabino-hex-1-enit umgesetzt und unter den in Beispiel 10 beschriebenen Bedingungen entblockiert.
MS: $M + H^+ = 606$

Beispiel 49:

7-O-(4-O-Benzyl-3-N-trifluoracetyl-α-L-acosaminyl)-β-rhodomycinon (49)

(Verbindung der Formel I mit $R^1 = R^3 = R^4 = H$, $R^2$ = Struktur II = α-L-Acosaminyl mit $R^5 = CH_3$, $R^6 = OBn$, $R^7$ = NHTFA, $R^8 = H$)

Verbindung 4 wurde unter den bei Beispiel 7 beschriebenen Bedingungen mit 1,5-Anhydro-4-O-benzyl-2,3,6-tridesoxy-3-N-trifluoracetyl-L-arabino-hex-1-enit umgesetzt und unter den in Beispiel 45 beschriebenen Bedingungen entblockiert.

Beispiel 50:

7-O-(2,6-Didesoxy-2-iodo-α-L-mannopyranosyl)-β-rhodomycinon (50)

(Verbindung der Formel I mit $R^1 = R^3 = R^4 = H$, $R^2$ = Struktur II = 2,6-Didesoxy-α-L-Mannopyranosyl mit $R^5 = CH_3$, $R^6 = OH$, $R^7 = OH$, $R^8 = I$)

Verbindung 26 wurde in trockenem Methanol und zwei Tropfen methanolischer Natriummethanolatlösung (33 %ig) gelöst, 2 Stunden bei Raumtemperatur gerührt, mit saurem Ionenaustauscher (Dowex WX8) neutralisiert, filtriert und eingeengt.
Schmelzpunkt: 158° C

Beispiel 51:

7,10-Di-O-(4-O-Benzyl-α-L-acosaminyl)-β-rhodomycinon (51)

(Verbindung der Formel I mit $R^1 = R^3 = H$, $R^2 = R^4$ = Struktur II = α-L-Acosaminyl mit $R^5 = CH_3$, $R^6 = OBn$, $R^7 = NH_2$, $R^8 = H$)

Verbindung 1 wurde analog zu Beispiel 7 mit 1,5-Anhydro-4-O-benzyl-2,3,6-tridesoxy-3-N-trifluoracetyl-L-hex-1-enit umgesetzt, dann analog zu Beispiel 8 am Aglycon entblokkiert und erneut analog Beispiel 7 mit 1,5-Anhydro-4-O-benzyl-2,3,6-tridesoxy-3-N-trifluoracetyl-L-hex-1-enit glycosidert und analog dem Beispiel 10 entblockiert.
MS: $M + H^+ = 826$

Beispiel 52:

7-O-(4-Desoxy-3-N-trifluoracetyl-α-L-daunosaminyl)-β-rhodomycinon (52)

(Verbindung der Formel I mit $R^1 = R^3 = H$, $R^2 = R^4$ = Struktur II = α-L-Daunosaminyl mit $R^5 = CH_3$, $R^6 = H$, $R^7$ = NHTFA, $R^8 = H$)

Verbindung 4 wurde unter den in Beispiel 7 beschriebenen Bedingungen mit 1-O-Paranitrobenzoyl-4-desoxy-3-N-trifluoracetyl-L-daunosamin umgesetzt und unter den in Beispiel 45 beschriebenen Bedingungen entblockiert.

Beispiel 53:

7-O-(4-Desoxy-α-L-daunosaminyl)-β-rhodomycinon (52)

(Verbindung der Formel I mit $R^1 = R^3 = H$, $R^2 = R^4$ = Struktur II = α-L-Daunosaminyl mit $R^5 = CH_3$, $R^6 = H$, $R^7 = NH_2$, $R^8 = H$)

Verbindung 4 wurde unter den in Beispiel 7 beschriebenen Bedingungen mit 1-O-Paranitrobenzoyl-4-desoxy-3-N-trifluoracetyl-L-daunosamin umgesetzt und unter den in Beispiel 10 beschriebenen Bedingungen entblockiert.

In den nachfolgenden Tabellen 2 und 3 sind die $^1$N-NMR-Daten einiger der vorstehend beschriebenen neuen Verbindungen 1-43 zusammengestellt.

Tabelle 2

$^1$H-NMR-Daten verschiedener Verbindungen der Formel I

Die Substanz-Nummern der ersten Zeile entsprechen den jeweiligen Beispielsnummern. Die chemische Verschiebung ist in ppm angegeben, wobei Tetramethylsilan als interner Standard dient. Soweit nicht anders angegeben wurde, sind die Spektren in $CDCl_3$ als Lösungsmittel gemessen.

Abkürzungen:

s: Singulett
d: Dublett
t: Triplett
q: Quartett
m: Multiplett
dd: Dublett eine Dubletts
ddd: Dublett eines Dubletts eines Dubletts
dq: Dublett eines Quartetts
a) gemessen bei 300 MHz
b) B-Phenyl 7.4-7.27m
c) gemessen bei 270 MHz
d) Verbindung 3a und 3b sind exo-endo-Isomere
e) Benzyliden-CH 5.41s, aromat. Benzyliden 7.36-7.20m
f) Benzyliden-CH 6.13s, aromat. Benzyliden 7.36-7.17m
g) Tetrahydropyranylring H-1 4.91m und 4.99m,
Ringprotonen 4.0-3.1m,
da die Substanz als Diastereomerengemisch vorliegt (R/S am THP-Ring C-1), liegen einige Signale doppelt vor
h) gemessen bei 400 MHz
i) O-Trimethylsilyl 0.13s
k) B-Phenyl 7.35-7.17m
l) Lösungsmittel $CDCl_3$ + $CD_3OD$
m) Lösungsmittel $CDCl_3$ + $d_6$-DMSO
n) $N(CH_3)_2$ 2.086 s
o) Tetrahydropyranyl H-1 5.09dd
OAc 2.03s, 2.09s
p) Tetrahydropyranyl H-1 5.04dd;
Ringprotonen 4.2-3.0m
OAc 2.03s, 2.01s
q) OAc 2.04s, 2.01s
r) Lösungsmitel $CDCl_3$ + Dioxan
s) gemessen bei 200 MHz
t) Benzyl $CH_2$ 4.77d, 4.66d, arom. Benzyl 7.37m
u) Benzyl $CH_2$ 4.46d, 4.33d, arom. Benzyl 7.29-7.12m

v) gemessen bei 90 MHz

Tabelle 2, Teil 1:

| | 1[a,b)] | 2[c)] | 3a[a,de)] | 3b[a,d,f)] |
|---|---|---|---|---|
| H-1 | 7.907dd | 7.821d | 7.85dd | 7.82dd |
| H-2 | 7.698t | 7.645t | 7.67t | 7.64t |
| H-3 | 7.774dd | 7.262d | 7.29dd | 7.25dd |
| H-7 | 5.672t | 5.279t | 5.67dd | 5.54t |
| H-8a | 2.293dd | 2.39dd | 2.31dd | 2.67dd |
| H-8b | 2.19dd | 2.13dd | 1.79dd | 2.16dd |
| H-10 | 4.973d | 4.782d | 4.94d | 4.84d |
| H-13a | 2.22-1.79m | 1.95m | 1.98-1.71m | 1.99-1.89m |
| H-13b | 2.22-1.79m | 1.76m | 1.98-1.71m | 1.99-1.89m |
| $H_3$-14 | 1.236t | 1.048t | 1.07t | 1.11t |
| H-1' | - | - | - | - |
| H-2a' | - | - | - | - |
| H-2e' | - | - | - | - |
| H-3' | - | - | - | - |
| H-4' | - | - | - | - |
| H-5' | - | - | - | - |
| $H_3$-6' | - | - | - | - |
| OH-4 | 12.145s | 12.151s | 12.09s | 11.96s |
| OH-6 | 12.78s | 12.683s | 12.67s | 12.66s |
| OH-11 | 13.575s | 13.498s | 13.70s | 13.51s |
| OH-7 | - | - | - | - |
| OH-9 | - | - | - | - |
| H-10 | | 2.68d | 3.18d | 2.80d |
| NHTFA | - | - | - | - |
| pNBz | - | - | - | - |

Tabelle 2, Teil 2:

| | 4[a)] | 5[c,g)] | 6[h,i)] | 7[a,k)] |
|---|---|---|---|---|
| H-1 | 7.902dd | 7.78d | 7.891dd | 7.77d |
| H-2 | 7.745t | 7.64t | 7.715t | 7.599t |
| H-3 | 7.347dd | 7.24d | 7.312dd | 7.70dd |
| H-7 | 5.345dt | 5.19dd/5.14dd | 5.231t | 5.680 |
| H-8a | 2.408dt | 2.30dd | 2.228dd | 2.45dd |
| H-8b | 2.054ddd | 2.05d | 2.146dt | 2.18d |
| H-10 | 6.344d | 4.94s/4.81s | 4.822d | 4.956s |
| H-13a | 1.73m | 1.75m | 1.718m | 1.83m |
| H-13b | 1.56m | 1.75m | 1.718m | 1.83m |
| $H_3$-14 | 1.103t | 1.03t | 1.075t | 1.18t |
| H-1' | - | - | - | 5.64d |
| H-2a' | - | - | - | 1.83m |
| H-2e' | - | - | - | 2.12m |
| H-3' | - | - | - | 4.44m |
| H-4' | - | - | - | 4.786t |
| H-5' | - | - | - | 4.18dq |
| $H_3$-6' | - | - | - | 1.27d |
| OH-4 | 12.010s | 11.97s | 12.106s | 12.032s |
| OH-6 | 12.806s | 12.81s | 12.897s | 12.714s |
| OH-11 | 13.345d | 13.66s/13.6s | 13.626s | 13.694s |
| OH-7 | 3.452d | | 3.478d | - |
| OH-9 | 3.756s | | 3.148s | |
| OH-10 | - | - | - | |
| NHTFA | - | 6.45d | 6.44d | |
| pNBz | - | 8.28-8.08m | 8.34-8.15m | |

Tabelle 2, Teil 3:

| | 8[c)] | 9[h)] | 10[a,l)] | 11[h,m)] |
|---|---|---|---|---|
| H-1 | 7.91dd | 7.905dd | 7.774d | 7.812d |
| H-2 | 7.73t | 7.726t | 7.621t | 7.653t |
| H-3 | 7.34dd | 7.344dd | 7.209d | 7.243d |
| H-7 | 5.31d | 5.279t | 5.087d | 5.089d |
| H-8a | 2.36dd | 2.308dd | 2.154d | 2.125dd |
| H-8b | 2.22d | 2.195d | 2.06d | 2.04d |
| H-10 | 4.98s | 5.020s | 4.87s | 4.886s |
| H-13a | 1.95-1.72m | 1.88-1.38m | 1.82m | 1.75m |
| H-13b | 1.95-1.72m | 1.88-1.38m | 1.82m | 1.70m |
| $H_3$-14 | 1.16t | 1.135t | 1.02t | 1.018t |
| H-1' | 5.57d | 5.659d | 5.31d | 5.302d |
| H-2a' | 1.8m | 2.16-1.89m | 1.49m | 1.60m |
| H-2e' | 2.09dd | 2.16-1.89m | 2.09m | 2.01m |
| H-3' | 4.48d | 4.493m | 2.87m | 2.9m |
| H-4' | 4.82t | 5.409s | 2.87m | 3.317s |
| H-5' | 4.21dq | 4.314q | 3.66dq | 3.881q |
| $H_3$-6' | 1.29d | 1.228d | 1.236d | 1.202d |
| OH-4 | 12.06s | 12.041s | | |
| OH-6 | 12.89s | 12.894s | | |
| OH-11 | 13.72s | 13.735s | | |
| OH-7 | 3.54d | 3.491d | | |
| OH-9 | 3.34s | 3.248s | | |
| OH-10 | - | - | | |
| NHTFA | - | | | - |
| pNBz | - | | | - |

Tabelle 2, Teil 4:

| | 12h,n) | 13h) | 14h) | 15h,m) |
|---|---|---|---|---|
| H-1 | 7.821d | 7.908d | 7.800d | 7.78d |
| H-2 | 7.644t | 7.738t | 7.622t | 7.64t |
| H-3 | 7.245d | 7.334d | 7.229d | 7.21d |
| H-7 | 5.167d | 5.246t | 5.063t | 4.995t |
| H-8a | 2.2-2.09m | 2.40dd | 2.24-1.99m | 2.105m |
| H-8b | 2.2-2.09m | 2.45d | 2.24-1.99m | 2.105m |
| H-10 | 4.908s | 6.414d | 4.854d | 4.70s |
| H-13a | 1.85-1.66m | 1.75m | 1.86-1.69m | 1.75-1.60m |
| H-13b | 1.85-1.66m | 1.75m | 1.86-1.69m | 1.75-1.60m |
| $H_3$-14 | 1.039t | 1.125t | 1.073t | 0.99t |
| H-1' | 5.417d | 5.520d | 5.419 | 5.29d |
| H-2a' | 1.489ddd | 1.86ddd | 2.24-1.99m | 1.49m |
| H-2e' | 2.194ddd | 2.01ddd | 2.24-1.99m | 1.94m |
| H-3' | 2.602ddd | 4.3m | 4.38m | 2.8m |
| H-4' | 3.012t | 4.84t | 4.891t | 2.8m |
| H-5' | 3.699dq | 4.3m | 4.278dq | 3.77dq |
| $H_3$-6' | 1.268d | 1.330d | 1.238d | 1.23d |
| OH-4 | | 12.002s | 12.023s | |
| OH-6 | | 12.813s | 12.796s | |
| OH-11 | | 13.380s | 13.551s | |
| OH-7 | | | - | - |
| OH-9 | | 3.819s | 3.575s | |
| OH-10 | | - | 3.615d | |
| NHTFA | - | 6.47d | | - |
| pNBz | - | 8.25-8.9m | 8.22-8.09m | - |

Tabelle 2, Teil 5:

| | 16[a)] | 18[h)] | 19[h,l)] | 23[h,r)] |
|---|---|---|---|---|
| H-1 | 7.79dd | 7.74d | 7.70d | 7.78dd |
| H-2 | 7.634t | 7.65t | 7.62t | 7.59t |
| H-3 | 7.229dd | 7.22d | 7.17d | 7.21dd |
| H-7 | 5.059dd | 5.25d | 5.06s | 4.96dd |
| H-8a | 2.22-2.02m | 2.47d | 2.13m | 2.10m |
| H-8b | 2.22-2.02m | 2.08dd | 2.13m | 2.10m |
| H-10 | 4.857d | 6.33s | 4.78s | 4.74s |
| H-13a | 1.90-1.52m | 1.77m | 1.88m | 1.75m |
| H-13b | 1.90-1.52m | 1.53m | 1.68m | 1.65m |
| $H_3$-14 | 1.054t | 1.10t | 1.05t | 1.02t |
| H-1' | 5.472d | 5.65s | 5.53s | 5.23dd |
| H-2a' | 1.90-1.52m | | 1.90ddd | 1.92m |
| H-2e' | 2.22-2.02m | | 2.13m | 2.10m |
| H-3' | 2.578ddd | 4.48m | 4.34m | 4.04m |
| H-4' | 3.101t | 5.49s | 5.42s | 3.45m |
| H-5' | 3.857dq | 4.48m | 4.44q | 3.55dq |
| $H_3$-6' | 1.320d | 1.27d | 1.16d | 1.25d |
| OH-4 | | | 11.84s | |
| OH-6 | | 12.68s | | |
| OH-11 | | 13.22s | | |
| OH-7 | - | - | - | |
| OH-9 | 3.881s | 3.85s | | |
| OH-10 | | - | | |
| NHTFA | - | 6.61d | | |
| pNBz | - | 8.25m | 8.23m | |

Tabelle 2, Teil 6:

| | 24 h,o) | 25 h,p) | 26 a,q) |
|---|---|---|---|
| H-1 | 7.88d | 7.90d | 7.83d |
| H-2 | 7.69t | 7.69t | 7.69t |
| H-3 | 7.30d | 7.31d | 7.28d |
| H-7 | 5.12t | 5.07t | 5.02dd |
| H-8a | | | 2.22d |
| H-8b | | | 2.11dd |
| H-10 | 4.98s | 5.04s | 4.88s |
| H-13a | | | 1.91-1.70m |
| H-13b | | | 1.91-1.70m |
| $H_3$-14 | 1.13t | 1.17t | 1.10t |
| H-1' | 5.79s | 5.37s | 5.71d |
| H-2a' | - | - | - |
| H-2e' | 4.56dd | 4.61dd | 4.66dd |
| H-3' | 4.27d | 4.29dd | 4.32dd |
| H-4' | 4.86s | 5.16t | 5.18t |
| H-5' | 4.08q | 4.04dq | 4.17dq |
| $H_3$-6' | 1.21d | 1.24d | 1.29d |
| OH-4 | 12.12s | 12.12s | 12.04s |
| OH-6 | 12.89s | 12.90s | 12.79s |
| OH-11 | 13.78s | 13.81s | 13.51s |
| OH-7 | - | - | - |
| OH-9 | | | 3.37s |
| OH-10 | - | - | 2.80s |
| NHTFA | - | - | - |
| pNBz | - | - | - |

Tabelle 2, Teil 7:

| | 44 a) | 45 s) | 46 a) | 47 a) |
|---|---|---|---|---|
| H-1 | 7.83dd | 7.80d | 7.75dd | 7.88d |
| H-2 | 7.65t | 7.69t | 7.64t | 7.73t |
| H-3 | 7.26dd | 7.24d | 7.15dd | 7.32d |
| H-7 | 5.22m | 5.02t | 4.87m | 4.96d |
| H-8a | | 2.20m | 2.08m | |
| H-8b | | 2.20m | 2.08m | |
| H-10 | 5.11s | 4.82m | 4.60s | 4.81s |
| H-13a | | 1.72m | 1.78m | - |
| H-13b | | 1.72m | 1.78m | - |
| $H_3$-14 | 0.90t | 1.06t | 1.04t | 1.09t |
| H-1' | | 5.35d | 5.42d | 5.35s |
| H-2a' | | 2.20m | 2.08m | |
| H-2e' | | 2.20m | 2.08m | |
| H-3' | | 3.80m | 3.66m | 4.39ddd |
| H-4' | | 3.16t | 3.52dd | 3.50dd |
| H-5' | | 3.96dq | 4.14dq | 4.15dq |
| $H_3$-6' | | 1.32d | 1.37d | 1.34d |
| OH-4 | | 12.08s | | |
| OH-6 | | 12.79s | | |
| OH-11 | | 13.59s | | |
| OH-7 | | - | - | - |
| OH-9 | | | | |
| OH-10 | | | | |
| NHTFA | | 7.75d | - | 8.01d |
| pNBz | | - | - | - |

Tabelle 2, Teil 8:

| | 48[a,t] | 49[h,u] | 50[a] | 52[v] |
|---|---|---|---|---|
| H-1 | 7.88d | 7.86d | 7.91dd | 7.92dd |
| H-2 | 7.72t | 7.70t | 7.76t | 7.71t |
| H-3 | 7.32d | 7.31d | 7.35dd | 7.28dd |
| H-7 | 5.13m | 5.24s | 5.07d | 5.12t |
| H-8a | 2.22d | 2.37d | | |
| H-8b | 2.16dd | 2.10m | | |
| H-10 | 4.91s | 5.09s | 4.81d | 4.95d |
| H-13a | 1.9-1.5m | 1.98m | 1.7m | |
| H-13b | 1.9-1.5m | 1.98m | 1.7m | |
| $H_3$-14 | 1.11t | 1.03t | 1.08t | 1.13t |
| H-1' | 5.43d | 5.27d | 5.71s | 5.52d |
| H-2a' | 2.2-1.5m | 2.10m | - | |
| H-2e' | 2.2-1.5m | 2.19ddd | 4.45dd | |
| H-3' | 3.09ddd | 3.93m | 3.97dd | 4.2m |
| H-4' | 2.91t | 3.09t | | |
| H-5' | 3.98dq | 3.39dq | | 4.2m |
| $H_3$-6' | 1.41d | 0.98d | 1.38d | 1.28d |
| OH-4 | | 12.11s | 12.18s | 12.14s |
| OH-6 | | 12.88s | 12.93s | 12.77s |
| OH-11 | | 13.65s | 13.72s | 13.52s |
| OH-7 | - | - | - | - |
| OH-9 | | | | |
| OH-10 | | | | 2.68d |
| NHTFA | | 6.09d | | 5.92d |
| pNBz | | - | | - |

Tabelle 3

$^1$H-NMR-Daten verschiedener Verbindungen der Formel I

Die Substanz-Nummern der ersten Zeile entsprechend den jeweiligen Beispielsnummern. Die chemische Verschiebung ist in ppm angegeben, wobei Tetramethylsilan als interner Standard dient. Soweit nicht anders angegeben wurde, sind die Spektren in $CDCl_3$ als Lösungsmittel gemessen.

Abkürzungen:

| | |
|---|---|
| s: | Singulett |
| d: | Dublett |
| t: | Triplett |
| q: | Quartett |
| m: | Multiplett |
| dd: | Dublett eines Dublett |
| ddd: | Dublett eines Dublett eines Dublett |
| dq: | Dublett eines Quartetts |

Die einfach gestrichenen Kohlenhydratprotonen (H-1′ usw.) beziehen sich auf die Kohlenhydrate als $R^2$, die zweifach gestrichenen Kohlenhydratprotonen (H-1" usw.) beziehen sich auf die Kohlenhydrate als $R^4$ in der allgemeinen Formel I

a) gemessen bei 270 MHz
b) die Werte sind paarweise austauschbar
c) gemessen bei 300 MHz
d) OAc 2.21s
e) gemessen bei 400 MHz
f) Lösungsmittel $COCl_3$ + $d_6$-DMSO
g) OAc 2.01s, 1.99s
h) Lösungmittel $D_2O$
i) $N(CH_3)_2$ 2.10s, 2.07s
k) OBz 7.72-7.56m;
$N(CH_3)_2$ 2.28s
l) Bei den Protonen der drei Kohlenhydratreste ist die Zuordnung der Kohlenhydratringprotonen zu den einzelnen Kohlenhydratresten unbestimmt.

| | |
|---|---|
| H-1 | 5.66d, 5.59d, 5.43d |
| H-3 | 4.74-4.65m, 2 x 4.52-4.35m |
| H-4 | 3 x 4.99-4.76m |
| H-5 | 2 x 4.20-4.08m, 3.47dq |
| $H_3$-6 | 1.37d, 1.27d, 0.84d |
| NHTFA | 2 x 6.67d, 6.45d |

Tabelle 3, Teil 1:

| | 27[a)] | 28[c,d)] | 29[e,f)] |
|---|---|---|---|
| H-1 | 7.795d | 7.905d | 7.74d |
| H-2 | 7.651t | 7.718t | 7.59t |
| H-3 | 7.241d | 7.314d | 7.18d |
| H-7 | 5.157t | 5.138t | 4.96t |
| H-8a | 2.24-1.94m | | 1.69-1.48m |
| H-8b | 2.24-1.94m | | 1.69-1.48mm |
| H-10 | 5.071s | 4.873s | 4.78s |
| H-13a | 1.85-1.66m | | 1.69-1.48m |
| H-13b | 1.85-1.66m | | 1.69-1.48m |
| $H_3$-14 | 1.107t | 1.065t | 0.92t |
| H-1' | 5.431d[b)] | 5.507d[b)] | 5.25d[b)] |
| H-2a' | 1.85-1.66m | | 1.69-1.48m |
| H-2e' | 2.24-1.94m | | 1.69-1.48m |
| H-3' | 4.47-4.42m | 4.53m[b)] | |
| H-4' | 5.595s[b)] | 5.14s[b)] | 3.29s[b)] |
| H-5' | 4.47-4.42m[b)] | 4.34q[b)] | |
| $H_3$-6' | 1.232d[b)] | 1.26d[b)] | 1.16d[b)] |
| H-1'' | 5.347d[b)] | 5.34d[b)] | 5.19d[b)] |
| H-2a'' | 1.85-1.66m | | 1.69-1.48m |
| H-2e'' | 2.24-1.94m | | 1.69-1.48m |
| H-3'' | 4.47-4.42m | 4.53m[b)] | |
| H-4'' | 5.589s[b)] | 5.14s[b)] | 3.26s[b)] |
| H-5'' | 4.309q[b)] | 4.20q[b)] | 3.79q[b)] |
| $H_3$-6'' | 1.147d[b)] | 1.21d[b)] | 1.12d[b)] |
| OH-4 | 11.902s | 12.158s | |
| OH-6 | 12.804s | 12.901s | |
| OH-11 | 13.683s | 13.674s | |
| OH-9 | 3.287s | | |
| NHTFA | 6.29d/6.24d | 6.54d/6.30d | - |
| pNBz | 8.30-8.18m | - | - |

Tabelle 3, Teil 2:

| | 31[c)] | 32[e,g)] | 33[e,h)] | 34[a,f,i)] |
|---|---|---|---|---|
| H-1 | 7.85d | 7.81dd | 7.50d | 7.81dd |
| H-2 | 7.66t | 7.63t | 7.35t | 7.64t |
| H-3 | 7.27d | 7.24dd | 7.09d | 7.24dd |
| H-7 | 5.15m | 5.07d | | 5.07t |
| H-8a | 2.29m | 2.33-1.92m | | 1.83-1.39m |
| H-8b | 2.29m | 2.33-1.92m | | 1.83-1.39m |
| H-10 | 4.98s | 4.93s | | 4.92s |
| H-13a | 1.93-1.68m | 1.82-1.6m | | 1.83-1.39m |
| H-13b | 1.93-1.68m | 1.82-1.6m | | 1.83-1.39m |
| $H_3$-14 | 1.13t | 1.07s | 1.03t | 1.04t |
| H-1' | 5.45d | 5.39d[b)] | 5.47d[b)] | 5.46d[b)] |
| H-2a' | 1.85ddd | 1.82-1.6m | | 1.83-1.39m |
| H-2e' | 2.35ddd | 2.33-1.92m | | 1.83-1.39m |
| H-3' | 4.28m | 4.15m[b)] | | 2.8-2.5m |
| H-4' | 4.83t | 4.55t[b)] | | 3.09t[b)] |
| H-5' | 4.30dq | 4.11dq[b)] | | 3.83dq[b)] |
| $H_3$-6' | 1.27d | 1.21d[b)] | 1.33d[b)] | 1.31d[b)] |
| H-1'' | 5.50d | 5.38d[b)] | 5.35d[b)] | 5.42d[b)] |
| H-2a'' | 1.76ddd | 1.82-1.6m | | 1.83-1.39m |
| H-2e'' | 2.02ddd | 2.33-1.92m | | 1.83-1.39m |
| H-3'' | 4.34m | 4.15m | | 2.8-2.5m |
| H-4'' | 4.74t | 4.47t[b)] | | 3.01t[b)] |
| H-5'' | 4.12dq | 3.96dq[b)] | | 3.70dq[b)] |
| $H_3$-6'' | 1.23d | 1.17d[b)] | 1.33d[b)] | 1.27d[b)] |
| OH-4 | 12.01s | 11.98s | | 12.07s |
| OH-6 | 12.84s | 12.78s | | 12.84s |
| OH-11 | 13.71s | 13.65s | | 13.60s |
| OH-9 | 3.30s | 3.26s | | 3.53s |
| NHTFA | 6.39d/6.33d | 6.62d/6.55d | - | - |
| pNBz | 8.02-8.36m | - | - | - |

Tabelle 3, Teil 3:

| | 35[e)] | 36[e)] | 37[e)] |
|---|---|---|---|
| H-1 | 7.94dd | 7.82dd | 7.90dd |
| H-2 | 7.76t | 7.64t | 7.71t |
| H-3 | 7.36dd | 7.25dd | 7.32dd |
| H-7 | 5.22t | 5.08t | 5.10m |
| H-8a | | 2.36-1.60m | |
| H-8b | | 2.36-1.60m | |
| H-10 | 5.12s | 4.99s | 4.96s |
| H-13a | 2.08-1.79m | 2.36-1.60m | |
| H-13b | 2.08-1.79m | 2.36-1.60m | |
| $H_3$-14 | 1.21t | 1.09t | 1.09t |
| H-1' | 5.55d[b)] | 5.59d[b)] | 5.38s[b)] |
| H-2a' | 2.08-1.79m | 2.36-1.60m | |
| H-2e' | 2.08-1.79m | 2.36-1.60m | |
| H-3' | 4.55-4.32m | 4.12ddd | 2.78m |
| H-4' | 4.93t | 4.54t | 2.92t |
| H-5' | 4.55-4.32m | 4.12dq | 3.83dq |
| $H_3$-6' | 1.37d[b)] | 1.22d[b)] | 1.34d[b)] |
| H-1'' | 5.45d[b)] | 5.39d[b)] | 5.37s[b)] |
| H-2a'' | 2.08-1.79m | 2.36-1.60m | |
| H-2e'' | 2.08-1.79m | 2.36-1.60m | |
| H-3'' | 4.55-4.32m | 4.412ddd | 3.04m |
| H-4'' | 5.71s | 5.34s | 3.37s |
| H-5'' | 4.55-4.32m | 4.24q | 3.96q |
| $H_3$-6'' | 1.28d[b)] | 1.16d[b)] | 1.28d[b)] |
| OH-4 | 12.09s | 11.97s | |
| OH-6 | 12.93s | 12.79s | |
| OH-11 | 13.83s | 13.70s | |
| OH-9 | 3.41s | 3.29s | |
| NHTFA | 6.54d/6.25d | 6.25d/6.13d | |
| pNBz | 8.36-8.2m | 8.26-8.09m | |

Tabelle 3, Teil 4:

| | 38[e,k)] | 39[e)] | 40[e)] | 43[a,l)] |
|---|---|---|---|---|
| H-1 | 7.87d | 7.83d | 7.89d | 7.92d |
| H-2 | 7.46t | 7.65t | 7.71t | 7.74t |
| H-3 | 7.31d | 7.26d | 7.32d | 7.36d |
| H-7 | 5.23s | 5.14m | 5.13t | 5.29t |
| H-8a | 2.05-2.02m | | 1.9-1.65m | |
| H-8b | 2.05-2.02m | | 1.9-1.65m | |
| H-10 | 4.99s | 4.91s | 4.95s | 5.35s |
| H-13a | 1.93-1.74m | | 1.9-1.65m | |
| H-13b | 1.93-1.74m | | 1.9-1.65m | |
| $H_3$-14 | 1.15t | 1.14t | 1.09t | 1.29t |
| H-1' | 5.54s | 5.49s[b)] | 5.46d[b)] | |
| H-2a' | 1.93-1.74m | | | |
| H-2e' | 2.21m | | | |
| H-3' | 2.69ddd | 2.23m | | |
| H-4' | 5.54s | 3.62s | 3.67s | |
| H-5' | 4.27q | 3.97q | 4.02q | |
| $H_3$-6' | 1.26d | 1.34d | 1.37d[b)] | |
| H-1'' | 5.68d | 5.44s[b)] | 5.37d[b)] | |
| H-2a'' | 1.93-1.74m | | | |
| H-2e'' | 2.21m | | | |
| H-3'' | 4.44ddd | 4.37m | 2.76m | |
| H-4'' | 4.79t | 4.73t | 2.85t | |
| H-5'' | 4.16dq | 4.13dq | | |
| $H_3$-6'' | 1.21d | 1.19d | 1.30d[b)] | |
| OH-4 | 12.07s | 12.08s | | 12.14s |
| OH-6 | 12.88s | 12.80s | | 13.13s |
| OH-11 | 13.78s | 13.74s | | 13.92s |
| OH-9 | | 3.42s | | |
| NHTFA | 6.58d | 6.28d | | |
| pNBz | 8.27-8.08m | 8.24-8.08m | | 8.34-7.96m |

**Patentansprüche**

**Patentansprüche für folgende Vertragsstaaten : AT, BE, CH, DE, FR, GB, IT, LI, LU, NL, SE**

**1.** Verbindung der Formel I, deren Salze einer anorganischen oder organischen Säure,

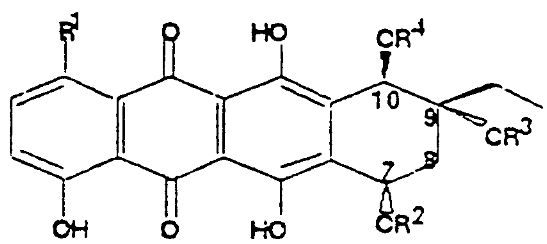

I

für welche gilt:

$R^1$ ist Wasserstoff oder eine Hydroxygruppe

$R^2$ ist Wasserstoff, eine Struktur der Formel II oder IV oder zusammen mit $R^3$, der Formel III,

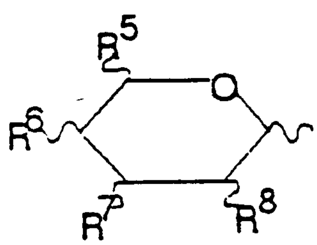

II

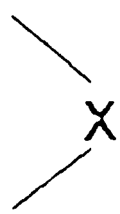

III

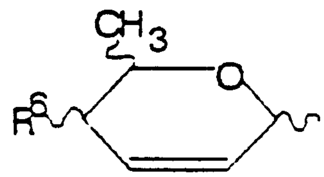

IV

$R^3$ ist Wasserstoff, eine Struktur der Formel II oder IV, oder zusammen mit $R^2$ der Formel III,

$R^4$ ist Wasserstoff, Trimethylsilyl, eine in der Kohlenhydratchemie übliche Schutzgruppe, vorzugsweise Acetyl, Trifluoracetyl, Benzoyl oder substituiertes Benzoyl wie para-Nitrobenzoyl, eine Struktur der Formel II oder eine Struktur der Formel IV,

$R^5$ ist Wasserstoff, Methyl, Hydroxymethyl, Acyloxymethyl (C1-C8) oder Alkyloxymethyl (C1-C8),

$R^6$,$R^7$ und $R^8$ sind unabhängig voneinander, Wasserstoff, Hydroxy, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy oder Halogen, wobei $R^7$ weiterhin $NH_2$, NHAcyl (C1-C8), $N(Alkyl)_2$ (C1-C8), $N(CH_2CN)_2$, $NH(CH_2CN)$ oder Azido sein kann, und

X ist eine in der Kohlenhydratchemie übliche zweizähnige Schutzgruppe, vorzugsweise Alkylboronyl, Phenylboronyl, ein Ortho-Alkyl-Carbonsäurealkylester, vorzugsweise Orthoameisensäuremethylester oder Orthoessigsäureethylester, ein Ketal oder Acetal, vorzugsweise Isopropyliden oder Benzyliden,

wobei die Verbindungen mit, $R^1 = R^2 = R^3 = R^4 = H$, $R^1 = OH$ und $R^2 = R^3 = R^4 = H$, $R^1 = R^3 = R^4 = H$ und $R^2 = \alpha$-L-Daunosaminyl oder $\alpha$-L-Rhodosaminyl oder 4'-Acyl-$\alpha$-L-rhodosaminyl, $R^1 = OH$ oder H, $R^3 = H$ und $R^2 = R^4 = \alpha$-L-Rhodosaminyl oder 4'-Acyl$\alpha$-L-rhodosaminyl und $R^1 = R^2 = R^3 = H$ und $R^4 = \alpha$-L-Rhodosaminyl oder $\alpha$-L-Daunosaminyl sowie $R^1 =$ OH, $R^3 = R^4 = H$ und $R^2 =$ Rhodosaminyl oder Daunosaminyl ausgenommen sind.

**2.** Verbindung nach Anspruch 1,
**dadurch gekennzeichnet**,
daß $R^2$ und $R^3$ unabhängig voneinander Wasserstoff, eine Struktur der Formel II oder IV mit der in Anspruch 1 angegebenen Bedeutung für $R^5$ bis $R^8$ sind.

**3.** Verbindung nach Anspruch 1,
**dadurch gekennzeichnet**,
daß $R^2$ und $R^3$ unabhängig voneinander Wasserstoff, eine Struktur der Formel II oder IV sind, wobei $R^5$ Methyl ist, $R^6$ und $R^7$ die in Anspruch 1 angegebene Bedeutung haben und $R^8$ Wasserstoff oder Halogen ist.

**4.** Verbindung nach Anspruch 1,
**dadurch gekennzeichnet**,
daß $R^2$ und $R^3$ unabhängig voneinander Wasserstoff, eine Struktur der Formel II oder IV sind, wobei $R^5$ Methyl ist, $R^6$ die in Anspruch 1 angegebene Bedeutung hat, $R^7$ $NH_2$, NHAcyl (C1-C8), $N(Alkyl)_2$ (C1-C8), $N(CH_2CN)_2$, $NH(CH_2CN)$ oder Azido ist, $R^8$ Wasserstoff oder Halogen ist.

**5.** Verbindung nach Anspruch 1,
**dadurch gekennzeichnet,**
daß $R^2$ und $R^3$ unabhängig voneinander Wasserstoff, eine Struktur der Formel II oder IV sind, wobei $R^5$ Methyl ist, $R^6$ und $R^7$ die in Anspruch 1 angegebene Bedeutung haben und $R^8$ Wasserstoff oder Halogen ist und die Struktur der Formel II oder IV der L-Reihe der Kohlenhydrate angehören.

**6.** Verbindung nach Anspruch 1,
**dadurch gekennzeichnet,**
daß $R^2$ und $R^3$ unabhängig voneinander Wasserstoff, eine Struktur der Formel II oder IV sind, wobei $R^5$ Methyl ist, $R^6$ die in Anspruch 1 angegebene Bedeutung hat, $R^7$ $NH_2$, NHAcyl (C1-C8), $N(Alkyl)_2$ (C1-C8), $N(CH_2CN)_2$, $NH(CH_2CN)$ oder Azido ist, $R^8$ Wasserstoff oder Halogen ist und die Strukturen der Formel II oder IV oder L-Reihe der Kohlenhydrate angehören.

**7.** Verfahren zur Herstellung einer Verbindung nach einem der Ansprüche 1 bis 6, dadurch gekennzeichnet, daß man

a) eine Verbindung der Formel I, in welcher $R^1$ die anfangs genannte Bedeutung hat und $R^2$ bis $R^4$ Wasserstoff sind, in einem geeigneten organischen Lösungsmittel wie Toluol oder DMF oder deren Mischungen, mit einem Katalysator wie einer Mineral-, Carbon- oder Sulfonsäure, bei einer Temperatur zwischen 0 °C und der Siedetemperatur des Lösungsmittels, mit einer Boronsäure wie Phenylboronsäure oder mit einem Keton wie Aceton oder einem Ketal wie 2,2-Dimethoxypropan oder einem Acetal wie Benzaldehyddimethylacetal, gegebenenfalls unter Zugabe eines Wasserfängers wie einem 4Å Molekularsieb zur Reaktion bringt, worauf man eine Verbindung der Formel I erhält, bei der $R^1$ die anfangs genannte Bedeutung hat und $R^2$ zusammen mit $R^3$ eine Verbindung der Formel III bilden, welche man durch Abfiltrieren und Entfernen des Lösungsmittels isoliert, und aus einem organischen Lösungsmittel wie Petrolether, auskristallisiert, woraufhin die Hydroxylgruppe an der Position 10 in geeigneter Weise derivatisiert wird,

b) gegebenenfalls durch eine Acylierung mit einem Carbonsäureanhydrid wie Essigsäureanhydrid, Trifluoressigsäureanhydrid oder einen Phenylcarbonsäureanhydrid oder einem Carbonsäurehalogenid oder durch Umsetzung mit Trifluormethansulfonsäure-trimethylsilylester in einem geeigneten organischen Lösungsmittel wie Chloroform, Dichlormethan oder Toluol oder deren Mischungen, bei einer Temperatur zwischen -40 °C und der Siedetemperatur des Lösungsmittels und in Gegenwart einer Base wie Triethylamin oder Pyridin, wobei eine Verbindung der Formel I erhalten wird, bei der
$R^1$ die anfangs genannte Bedeutung hat,
$R^2$ zusammen mit $R^3$ eine Struktur der Formel III und
$R^4$ Acyl oder Trimethylsilyl ist,

c) oder beispielsweise durch Umsetzung der unter a) erhaltenen Verbindung mit 3,4-Dihydro-2H-pyran in einem geeigneten organischen Lösungsmittel wie Chloroform, Dichlormethan, DMF oder Toluol in Gegenwart eines Katalysators wie para-Toluolsulfonsäure und eines Trockenmittels wie 4Å Molekularsieb, bei einer Temperatur zwischen -30 °C und der Siedetemperatur des Lösungsmittels, wobei eine Verbindung der Formel I entsteht, bei der
$R^1$ die anfangs genannte Bedeutung hat,
$R^2$ zusammen mit $R^3$ eine Struktur der Formel III
$R^4$ eine Struktur der Formel II mit $R^5$ bis $R^8$ Wasserstoff ist,

d) oder beispielsweise durch Umsetzung der unter a) erthaltenen Verbindung unter den in der Kohlenhydratchemie üblichen Bedingungen mit einem Kohlenhydratderivat der Formel V,

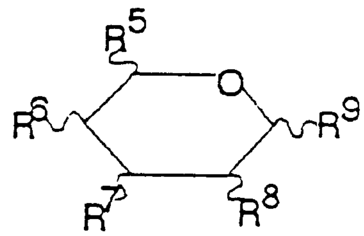

V

wobei

$R^5$ bis $R^8$ die anfangs genannte Bedeutung als geeignete Schutzgruppen haben und

$R^9$ Halogen wie Cl oder Br, O-Acyl- oder eine andere bei Glycosidierungsreaktionen übliche Abgangsgruppe ist,

wobei eine Verbindung der Formel I entsteht, bei der

$R^1$ die anfangs genannte Bedeutung hat,

$R^2$ zusammen mit $R^3$ eine Struktur der Formel III und

$R^4$ eine Struktur der Formel II ist,

e) oder beispielsweise, indem man die unter a) erhaltene Verbindung mit einem funktionalisierten Kohlenhydrat der allgemeinen Formel V oder VI,

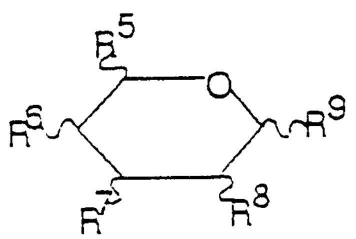

V

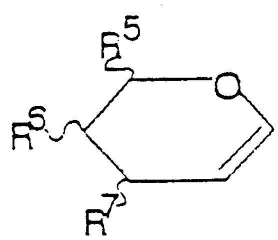

VI

worin

$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben und

$R^9$ über Sauerstoff gebundenes Acyl wie aliphatisches Acyloxy (C1-C8) wie Acetyl, Benzoyloxy, oder substituiertes Benzyloxy wie para-Nitrobenzoyloxy ist,

in Gegenwart eines organischen Lösungsmittels wie Chloroform, Dichlormethan, Toluol, Ether, DMF, Aceton, Acetonitril oder Nitromethan oder deren Mischungen, eines Katalysators, wie para-Toluolsulfonsäure oder eines Trifluormethansulfonsäuretrialkylsilylesters und gegebenenfalls eines Säurefängers und eines Trockenmittels wie einem Molekularsieb, bei einer Reaktionstemperatur von -70 °C bis +30 °C unter einer Schutzgasatmosphäre wie Stickstoff oder Argon, zu einer Verbindung der Formel I umsetzt,

wobei

$R^1$ die genannte Bedeutung hat,

$R^2$ zusammen mit $R^3$ eine Struktur der Formel III und

$R^4$ eine Struktur der Formel II oder IV ist, bei der

$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben,

f) und gegebenenfalls man die Verbindung der Stufen b) bis e) an den Positionen 7 und 9 entblockieren kann, indem man diese Verbindung in einem geeigneten organischen Lösungsmittel wie Chloroform, Dichlormethan, DMF, Toluol oder Methanol mit einem Katalysator wie verdünnte wässrige Lösungen von Carbonsäure oder para-Toluolsulfonsäure und gegebenenfalls mit einem Diol wie 2-Methyl-2,4-pentandiol, bei einer Temperatur zwischen 0 °C und der Siedetemperatur des Lösungsmittels zur Reaktion bringt, woraufhin man eine Verbindung der allgemeinen Formel I erhält,

bei der
$R^1$ die genannte Bedeutung hat,
$R^2$ und $R^3$ Wasserstoff sind und
$R^4$ die genannte Bedeutung außer Wasserstoff hat,
g) woraufhin man gegebenenfalls die Verbindung der Stufe f) bei der $R^4$ eine Struktur der Formel II oder IV mit einer der in der Kohlenhydratchemie üblichen Schutzgruppen als Reste $R^5$ bis $R^8$ ist, unter in der Kohlenhydratchemie üblichen Bedingungen selektiv an den geschützten Hydroxyfunktionen und/oder an den geschützten Aminofunktionen in an sich bekannter Weise mittels einer anorganischen oder organischen Base wie Alkali- oder Erdalkalihydroxiden, Natriumcarbonat und Triethylamin in einem Lösungsmittel wie Wasser, Methanol, Ethanol oder THF oder deren Mischungen partiell oder vollständig entblockiert kann, wobei eine Verbindung der Formel I entsteht, bei der
$R^1$ Wasserstoff oder Hydroxyl,
$R^2$ und $R^3$ Wasserstoff und
$R^4$ eine Struktur der Formel II oder IV sind, in welchen
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ $N(Alkyl)_2$, Azido, Hydroxy oder Alkyloxy sind und
$R^8$ die gleiche Bedeutung wie $R^6$ hat, aber unabhängig davon ist,
h) und man gegebenenfalls die Verbindung der Stufe g) der Formel I, bei der
$R^1$ bis $R^3$ die unter g) genannte Bedeutung haben und
$R^4$ eine Struktur der Formel II, wobei
$R^5$, $R^6$ und $R^8$ unter g) angegebene Bedeutung haben und
$R^7$ $NH_2$ sind,
unter den an sich bekannten Bedingungen der reduktiven Aminierung in die entsprechend Verbindung der Formel I umsetzt, wobei
$R^1$ bis $R^3$ die unter g) genannte Bedeutung haben und
$R^4$ eine Struktur der Formel II ist,
bei der
$R^5$, $R^6$ und $R^8$ die unter g) genannte Bedeutung haben und $R^6$ $N(Alkyl)_2$ ist, oder
i) weiterhin eine Verbindung der Stufe g) der allgemeinen Formel I, bei der
$R^1$ bis $R^3$, die unter g) genannte Bedeutung haben und
$R^4$ eine Struktur der Formel II mit den für
$R^5$, $R^6$ und $R^7$ unter g) angegebenen Bedeutungen und
$R^7$ $NH_2$ sind,
durch Umsetzung mit Jodacetontril oder Bromacetonitril in einem geeigneten Lösungsmittel, beispielsweise Dimethylformamid, in Gegenwart einer geeigneten Base wie Triethylamin in eine Verbindung der Formel I überführt, bei der $R^1$ bis $R^3$ die unter g) genannte Bedeutung haben und $R^4$ eine Struktur der Formel II ist, bei der
$R^5$, $R^6$ und $R^8$ die unter g) genannten Bedeutungen haben und
$R^7$ $N(CH_2CN)_2$ oder $NH(CH_2CN)$ ist, und
k) gegebenenfalls die bei der Stufe f), h) oder i) entstandene Verbindung nach den bereits bei der Stufe e) genannten Bedingungen glycosidiert, wobei je nach Menge des benutzten Glycosyldonors entweder nur Position 7 oder gleichzeitig Position 7 und 9 glycosidiert werden, was zu Produkten führt, die der allgemeinen Formel I entsprechen, bei der
$R^1$ die genannte Bedeutung hat,
$R^2$ eine Struktur der Formel II oder IV ist, bei der
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben,
$R^3$ Wasserstoff ist oder $R^2$ entspricht und
$R^4$ Acyl, Trimethylsilyl oder eine Struktur der allgemeinen Formel II oder IV, wobei
$R^5$ bis $R^8$ die anfangs benannten Bedeutungen mit den Ausnahmen Hydroxymethylen, Hydroxy und $NH_2$ haben, ist, und gegebenenfalls
l) eine der bei der Stufe f), h) oder i) entstandenen Verbindungen in Gegenwart eines organischen Lösungsmittels wie Chloroform, Dichlormethan, Toluol, Ether, Aceton und Acetonitril oder deren Mischungen mit einem Glycal der Formel VI, wobei $R^5$ bis $R^7$ die bei Stufe e) genannte Bedeutung haben, mit N-Jodsuccinimid und gegebenenfalls mit einem Trockenmittel wie einem Molekularsieb bei einer Temperatur von -40 °C bis +40 °C unter einer Schutzgasatmosphäre wie Stickstoff oder Argon, zu einer Verbindung der Formel I überführen bei der

$R^1$ die genannte Bedeutung hat und
$R^2$ eine Struktur der Formel II ist, bei der
$R^5$ Wasserstoff, Methyl, Acyloxymethyl (C1-C8), Alkyloxymethyl (C1-C8),
$R^6$ Acyloxy, Alkyloxy, Allyloxy oder Benzyloxy,
$R^7$ Acyloxy, Alkyloxy, Allyloxy, Benzyloxy, NHAcyl, $N(Alkyl)_2$, Azido und
$R^8$ Jod ist, und
$R^3$ und $R^4$ die bei Stufe k) genannte Bedeutung haben, und
m) gegebenenfalls die bei der Stufe k) oder l) entstandene Verbindung gemäß den Bedingungen der Stufe g) entblockiert, wobei man eine Verbindung der Formel I erhält, bei der
$R^1$ die genannte Bedeutung hat, und
$R^2$ eine Struktur der Formel II oder IV ist, bei der
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ oder $N(Alkyl)_2$, Azido, Hydroxy oder Alkyloxy ist und
$R^8$ die gleiche Bedeutung wie $R^6$ hat, aber unabhängig davon ist,
$R^3$ Wasserstoff ist oder $R^2$ entspricht und
$R^4$ Waserstoff, Trimethylsilyl oder eine Struktur der allgemeinen Formel II oder IV ist, wobei
$R^5$ Wasserstoff, Methyl, Hydroxymethylen oder Alkyloxymethyl,
$R^6$ Wasserstoff, Hydroxy, Alkyloxy oder Halogen,
$R^7$ $NH_2$, $N(Alkyl)_2$, $N(CH_2CN)_2$ oder $NH(CH_2CN)$, Azido, Hydroxy oder Alkyloxy ist und
$R^8$ die gleiche Bedeutung wie $R^6$ hat, aber unabhängig davon ist, oder
n) die bei der Stufe k) oder l) entstandene Verbindung unter den Bedingungen der Stufe g) auch so entblockiert, daß selektiv nur die Position 10 entblockiert wird und man eine Verbindung der Formel I erhält, bei der
$R^1$ die genannte Bedeutung hat,
$R^2$ eine Struktur der Formel II oder IV ist, bei denen $R^5$ bis $R^8$ die anfangs genannten Bedeutungen mit den Ausnahmen Hydroxymethyl, Hydroxy und $NH_2$ haben,
$R^3$ Wasserstoff ist oder $R^2$ entspricht und
$R^4$ Wasserstoff ist,
o) woraufhin sich gegebenenfalls die Verbindung der Stufe m), bei der $R^2$ eine Struktur der Formel II mit $R^7$ gleich $NH_2$ und $R^3$ Wasserstoff oder $R^2$ ist, wiederum in der für Stufe h) beschriebenen Art und Weise der reduktiven Aminierung in die ensprechenden Verbindungen umwandelt, bei der $R^6$ N-$(Alkyl)_2$, ist, oder gegebenenfalls
p) eine Verbindung der Stufe m), bei der $R^2$ eine Struktur der Formel II mit $R^7$ gleich $NH_2$ und $R^3$ Wasserstoff oder $R^2$ ist, gemäß den Bedingungen der Stufe i) in die entsprechenden Cyanomethyl-Derivate überführt, bei der
$R^7$ $N(CH_2CN)_2$ oder $NH(CH_2CN)$ ist, und gegebenenfalls
q) eine Verbindung der Stufe n) nach den bereits bei der Stufe e) genannten Bedingungen glycosidiert, wobei je nach Menge des benutzten Glycosyldonors entweder nur Position 10, oder gleichzeitig Positionen 9 und 10 glycosidiert werden, was zu einer Verbindung der Formel I führt, bei der
$R^1$ Wasserstoff oder Hydroxy ist und
$R^2$ eine Struktur der allgemeinen Formel II oder IV sind, bei der
$R^5$ bis $R^8$ die anfangs genannte Bedeutung mit den Ausnehmen von Hydroxymethyl, Hydroxy und $NH_2$ haben,
$R^3$ Wasserstoff oder $R^2$ oder $R^4$ ist und
$R^4$ eine Struktur der allgemeinen Formel II oder IV ist, bei der
$R^5$ bis $R^8$ die anfangs genannte Bedeutung mit den Ausnahmen von Hydroxymethyl, Hydroxy, $NH_2$, $N(CH_2CN)_2$, $NH(CH_2CN)$ haben, und gegebenenfalls
r) die Reaktion der Stufe c), d) und l) auch an einer Verbindung der Stufe n) durchführt, so daß man eine Verbindung der Formel I erhält, bei der
$R^1$ die anfangs genannte Bedeutung hat und
$R^2$ eine Struktur der Formel II oder IV ist, bei der
$R^5$ bis $R^8$ die anfangs genannte Bedeutung mit den Ausnahmen Hydroxymethyl, Hydroxy und $NH_2$ haben,
$R^3$ Wasserstoff oder $R^2$ oder $R^4$ ist und
$R^4$ eine Struktur der allgemeinen Formel II oder IV ist, bei der
$R^5$ bis $R^8$ die anfangs genannte Bedeutung mit den Ausnahmen Hydroxymethyl, Hydroxy, $NH_2$, N-

$(CH_2CN)_2$ und $NH(CH_2CN)$ haben und gegebenenfalls
s) diese Verbindung der Stufe q) oder r) wieder wie bei Stufe g) beschrieben entblockiert wird, bei der eine Verbindung der Formel I entsteht, bei der
$R^1$ die anfangs genannte Bedeutung hat und
$R^2$, $R^3$ und $R^4$ unabhängig voneinander Wasserstoff oder eine Struktur der Formel II oder IV sind, bei der
$R^5$ bis $R^8$ die anfangs genannte Bedeutung mit den Ausnahmen Acyloxymethyl, Acyloxy, Benzoyloxy oder substituiertes Benzoyloxy, NHAcyl, und gegebenenfalls
t) die Verbindung der Stufe s) entsprechend der Stufe h) mittels Reduktions-Aminierung in das entsprechende Derivat überführt wird, bei dem der Rest $R^7$, der in der Stufe s) eine $NH_2$ Gruppe war in $N(Alkyl)_2$ gewandelt ist, oder
u) des weiteren eine Verbindung der Stufe s) gemäß den Bedingungen der Stufe i) in das entsprechende Cyanomethyl-Derivat überführt wird, bei dem der Rest $R^7$, der in der Stufe s) eine $NH_2$ Gruppe war, in $NH(CH_2CN)$ gewandelt ist,
v) und man gegebenenfalls Verbindung der Formel I, bei der
$R^1$,$R^2$ und $R^3$ die genannte Bedeutung haben und
$R^4$ Trimethylsilyl ist,
in einem organischen Lösungsmittel wie THF, Diethylether, Dioxan oder deren Gemische bei Temperaturen zwischen -40 °C und der Siedetemperatur des Lösungsmittels mit Tetrabutylammoniumfluorid umsetzt zu einer Verbindung der Formel I, bei der $R^1$, $R^2$ und $R^3$ die oben genannte Bedeutung haben und $R^4$ Hydroxyl ist,
w) und man gegebenenfalls Verbindungen der Formel I, bei der $R^1$, $R^2$, $R^3$, $R^4$ die genannte Bedeutung haben und $R^7$ $NH_2$, $N(Alkyl)_2$ (C1-C8), $N(CH_2CN)_2$ oder $NH(CH_2CN)$ ist, in das Salz einer anorganischen oder organischen Säure überführt.

**8.** Verfahren nach Anspruch 7,
**dadurch gekennzeichnet,**
daß man eine Verbindung der Formel I, in welcher $R^1$ die anfangs genannte Bedeutung, hat, $R^2$ zusammen mit $R^3$ eine Verbindung der Formel III bilden und $R^4$ Wasserstoff ist, durch eine Acylierung mit einem Carbonsäureanhydrid wie Essigsäureanhydrid, Trifluoressigsäureanhydrid oder einem Phenylcarbonsäureanhydrid oder einem Carbonsäurehalogenid oder durch Umsetzung mit Trifluormethansulfonsäure-trimethylsilylester in einem organischen Lösungsmittel wie Chloroform, Dichlormethan oder Toluol oder deren Mischungen, bei einer Temperatur zwischen -40 °C und der Siedetemperatur des Lösungsmittels und in Gegenwart einer Base wie Triethylamin oder Pyridin zu einer Verbindung der Formel I umsetzt, bei der
$R^1$ die anfangs genannte Bedeutung hat,
$R^2$ zusammen mit $R^3$ eine Struktur der Formel III und
$R^4$ Acyl oder Trimethylsilyl ist, oder
daß man eine Verbindung der Formel I, in welcher $R^1$ die anfangs genannte Bedeutung hat, $R^2$ zusammen mit $R^3$ eine Verbindung der Formel III bilden und $R^4$ Wasserstoff ist, durch Umsetzung mit 3,4-Dihydro-2H-pyran in einem geeigenten organischen Lösungsmittel wie Chloroform, Dichlormethan, DMF oder Toluol in Gegenwart eines Katalysators wie para-Toluolsulfonsäure und eines Trockenmittels wie 4Å-Molekularsieb, bei einer Temperatur zwischen -30 °C und der Siedetemperatur des Lösungsmittels in eine Verbindung der Formel I überführt, bei der
$R^1$ die anfangs genannte Bedeutung hat,
$R^2$ zusammen mit $R^3$ eine Struktur der Formel III
$R^4$ eine Struktur der Formel II mit $R^5$ bis $R^8$ Wasserstoff ist.

**9.** Verfahren nach Anspruch 7,
**dadurch gekennzeichnet,**
daß man eine Verbindung der Formel I, in welcher $R^1$ die anfangs genannte Bedeutung hat, $R^2$ zusammen mit $R^3$ eine Verbindung der Formel III bilden und $R^4$ die genannte Bedeutung außer Wasserstoff hat, an den Positionen 7 und 9 entblockieren kann, indem man diese Verbindung in einem geeigneten organischen Lösungsmittel wie Chloroform, Dichlormethan, DMF, Toluol oder Methanol mit einem Katalysator, wie verdünnte wässrige Lösungen von Carbonsäuren oder para-Toluolsulfonsäure und gegebenenfalls mit einem Diol wie 2-Methyl-2,4-pentandiol, bei einer Temperatur zwischen 0 °C und der Siedetemperatur des Lösungsmittels zur Reaktion bringt, woraufhin man eine Verbindung der allgemeinen Formel I erhält, bei der

$R^1$ die genannte Bedeutung hat,
$R^2$ und $R^3$ Wasserstoff sind und
$R^4$ die genannte Bedeutung außer Wasserstoff hat.

**10.** Verfahren nach Anspruch 7,
**dadurch gekennzeichnet,**
daß man eine Verbindung der Formel I, in welcher $R^1$ bis $R^4$ die genannte Bedeutung haben, aber $R^2$ oder $R^4$ Wasserstoff sein müssen und $R^5$ bis $R^8$ nicht Hydroxy, Hydroxymethyl oder $NH_2$ sein können mit einem funktionalisierten Kohlenhydrat der allgemeinen Formel V oder VI,

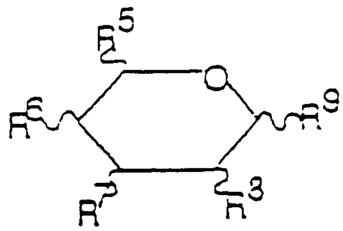

V

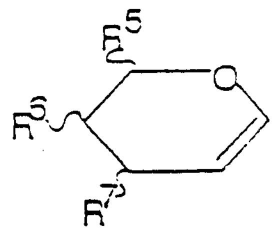

VI

worin
$R^5$ bis $R^8$ die anfangs genannte Bedeutungen mit den Ausnahmen Hydroxymethyl, Hydroxy, $NH_2$, $N(CH_2CN)_2$ und $NH(CH_2CN)$ haben und
$R^9$ über Sauerstoff gebundene Acylschutzgruppe wie aliphatisches Acyloxy (C1-C8) wie Acetyl, Benzoyloxy, oder substituiertes Benzyloxy wie para-Nitrobenzoyloxy ist,
in Gegenwart eines organischen Lösungsmittels wie Chloroform, Dichlormethan, Toluol, Ether, DMF, Aceton, Acetonitril oder Nitromethan oder deren Mischungen, eines Katalysators, wie para-Toluolsulfonsäure oder eines Trifluormethansulfonsäure-trialkylsilylesters und gegebenenfalls eines Säurefängers und eines Trockenmittels wie einem Molekularsieb, bei einer Reaktionstemperatur von -70 °C bis +30 °C unter einer Schutzgasatmosphäre wie Stickstoff oder Argon, zu einer Verbindung der Formel I umsetzt, wobei
$R^1$ die genannte Bedeutung hat,
$R^2$ zusammen mit $R^3$ eine Struktur der Formel III oder $R^2$, $R^3$ oder $R^4$ eine Struktur der Formel II oder IV ist, bei der
$R^5$ bis $R^8$ die anfangs genannte Bedeutungen mit den Ausnahmen Hydroxymethyl, Hydroxy, $NH_2$ haben, oder
daß man eine Verbindung der Formel I, in welcher $R^1$ bis $R^4$ die genannte Bedeutungen haben, aber $R^2$ oder $R^4$ Wasserstoff sein müssen und $R^5$ bis $R^8$ nicht Hydroxy, Hydroxymethyl oder $NH_2$ sein können in Gegenwart eines organischen Lösungsmittels wie Chloroform, Dichlormethan, Toluol, Ether, Aceton oder Acetonitril oder deren Mischungen mit einem Glycal oder Formel VI, wobei $R^5$ bis $R^7$ die anfangs genannten Bedeutungen mit den Ausnahmen Hydroxymethyl, Hydroxy, $NH_2$, $N(CH_2CN)_2$ und $NH(CH_2CN)$ haben, mit N-Jodsuccinimid und gegebenenfalls mit einem Trockenmittel wie einem Molekularsieb bei einer Temperatur von -40 °C bis +40 °C unter einer Schutzgasatmosphäre wie Stickstoff oder Argon, zu einer Verbindung der Formel I überführen, wobei
$R^1$ die genannte Bedeutung hat,
$R^2$ zusammen mit $R^3$ eine Struktur der Formel III oder $R^2$, $R^3$ oder
$R^4$ eine Struktur der Formel II oder IV ist, bei der
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben.

**11.** Verwendung einer Verbindung nach einem der Ansprüche 1 bis 10 zur Herstellung eines Arzneimittels.

**Patentansprüche für folgende Vertragsstaaten: ES, GR**

**1.** Verfahren zur Herstellung einer Verbindung der Formel I und gegebenenfalls einem ihrer Salze mit einer anorganischen oder organischen Säure,

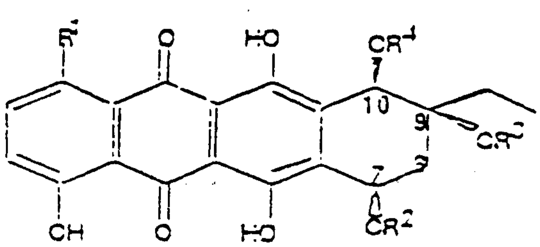

für welche gilt:
$R^1$ ist Wasserstoff oder eine Hydroxygruppe
$R^2$ ist Wasserstoff, eine Struktur der Formel II oder IV oder zusammen mit $R^3$, der Formel III,

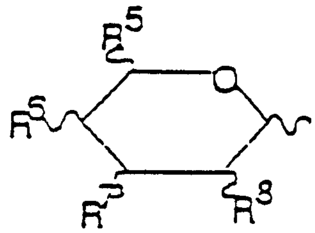

II

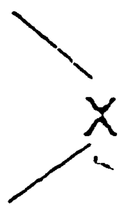

III

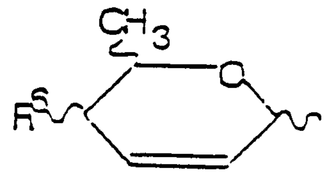

IV

$R^3$ ist Wasserstoff, eine Struktur der Formel II oder IV, oder zusammen mit $R^2$ der Formel III,
$R^4$ ist Wasserstoff, Trimethylsilyl, eine in der Kohlenhydratchemie übliche Schutzgruppe, vorzugsweise Acetyl, Trifluoracetyl, Benzoyl oder substituiertes Benzoyl wie para-Nitrobenzoyl, eine Struktur der Formel II oder eine Struktur der Formel IV,
$R^5$ ist Wasserstoff, Methyl, Hydroxymethyl, Acyloxymethyl (C1-C8) oder Alkyloxymethyl (C1-C8),
$R^6$,$R^7$ und $R^8$ sind unabhängig voneinander, Wasserstoff, Hydroxy, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy oder Halogen, wobei $R^7$ weiterhin $NH_2$, NHAcyl (C1-C8), $N(Alkyl)_2$ (C1-C8), $N(CH_2CN)_2$, $NH(CH_2CN)$ oder Azido sein kann, und
X ist eine in der Kohlenhydratchemie übliche zweizähnige Schutzgruppe, vorzugsweise Alkylboronyl, Phenylboronyl, ein Ortho-Alkyl-Carbonsäurealkylester, vorzugsweise Orthoameisensäuremethylester oder Orthoessigsäureethylester, ein Ketal oder Acetal, vorzugsweise Isopropyliden oder Benzyliden,
wobei die Verbindungen mit, $R^1 = R^2 = R^3 = R^4 = H$, $R^1 = OH$ und $R^2 = R^3 = R^4 = H$, $R^1 = R^3 = R^4 = H$ und $R^2 = \alpha$-L-Daunosaminyl oder $\alpha$-L-Rhodosaminyl oder 4'-Acyl-$\alpha$-L-rhodosaminyl, $R^1 = OH$ oder H, $R^3 = H$ und $R^2 = R^4 = \alpha$-L-Rhodosaminyl oder 4'-Acyl-$\alpha$-L-rhodosaminyl und $R^1 = R^2 = R^3 = H$ und $R^4 = \alpha$-L-Rhodosaminyl sowie $R^1$ = OH, $R^3$ = $R^4$ = H und $R^2$ = Rhodosaminyl oder Daunosaminyl ausgenommen sind, dadurch gekennzeichnet sind, daß man

a) eine Verbindung der Formel I, in welcher $R^1$ die anfangs genannte Bedeutung hat und $R^2$ bis $R^4$ Wasserstoff sind, in einem geeigneten organischen Lösungsmittel wie Toluol oder DMF oder deren Mischungen, mit einem Katalysator wie einer Mineral-, Carbon- oder Sulfonsäure, bei einer Temperatur zwischen 0 °C und der Siedetemperatur des Lösungsmittels, mit einer Boronsäure wie Phenylboronsäure oder mit einem Keton wie Aceton oder einem Ketal wie 2,2-Dimethoxypropan oder einem Acetal wie Benzaldehyddimethylacetal, gegebenenfalls unter Zugabe eines Wasserfängers wie einem 4Å Molekularsieb zur Reaktion bringt, worauf man eine Verbindung der Formel I erhält, bei der $R^1$ die anfangs genannte Bedeutung hat und $R^2$ zusammen mit $R^3$ eine Verbindung der Formel III bilden, welche man durch Abfiltrieren und Entfernen des Lösungsmittels isoliert, und aus einem organischen Lösungsmittel wie Petrolether, auskristallisiert, woraufhin die Hydroxylgruppe an der Position 10 in geeigneter Weise derivatisiert wird,

b) gegebenenfalls durch eine Acylierung mit einem Carbonsäureanhydrid wie Essigsäureanhydrid, Trifluoressigsäureanhydrid oder einen Phenylcarbonsäureanhydrid oder einem Carbonsäurehalogenid oder durch Umsetzung mit Trifluormethansulfonsäure-trimethylsilylester in einem geeigneten organischen Lösungsmittel wie Chloroform, Dichlormethan oder Toluol oder deren Mischungen, bei einer Temperatur zwischen -40 °C und der Siedetemperatur des Lösungsmittels und in Gegenwart einer Base wie Triethylamin oder Pyridin, wobei eine Verbindung der Formel I erhalten wird, bei der

$R^1$ die anfangs genannte Bedeutung hat,
$R^2$ zusammen mit $R^3$ eine Struktur der Formel III und
$R^4$ Acyl oder Trimethylsilyl ist,
c) oder beispielsweise durch Umsetzung der unter a) erhaltenen Verbindung mit 3,4-Dihydro-2H-pyran in einem geeigneten organischen Lösungsmittel wie Chloroform, Dichlormethan, DMF oder Toluol in Gegenwart eines Katalysators wie para-Toluolsulfonsäure und eines Trockenmittels wie 4Å Molekularsieb, bei einer Temperatur zwischen -30 °C und der Siedetemperatur des Lösungsmittels, wobei eine Verbindung der Formel I entsteht, bei der
$R^1$ die anfangs genannte Bedeutung hat,
$R^2$ zusammen mit $R^3$ eine Struktur der Formel III
$R^4$ eine Struktur der Formel II mit $R^5$ bis $R^8$ Wasserstoff ist.
d) oder beispielsweise durch Umsetzung der unter a) erhaltenen Verbindung unter den in der Kohlenhydratchemie üblichen Bedingungen mit einem Kohlenhydratderivat der Formel V,

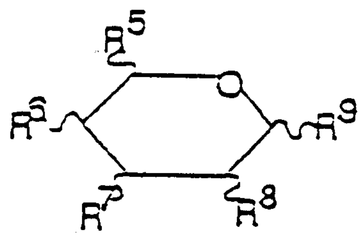

V

wobei
$R^5$ bis $R^8$ die anfangs genannte Bedeutung als geeignete Schutzgruppen haben und
$R^9$ Halogen wie Cl oder Br, O-Acyl- oder eine andere bei Glycosidierungsreaktionen übliche Abgangsgruppe ist,
wobei eine Verbindung der Formel I entsteht, bei der
$R^1$ die anfangs genannte Bedeutung hat,
$R^2$ zusammen mit $R^3$ eine Struktur der Formel III und
$R^4$ eine Struktur der Formel II ist,
e) oder beispielsweise, indem man die unter a) erhaltene Verbindung mit einem funktionalisierten Kohlenhydrat der allgemeinen Formel V oder VI,

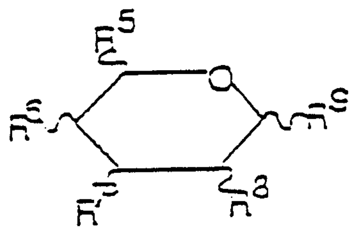

V

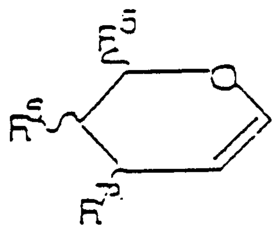

VI

worin
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben und
$R^9$ über Sauerstoff gebundenes Acyl wie aliphatisches Acyloxy (C1-C8) wie Acetyl, Benzoyloxy, oder substituiertes Benzyloxy wie para-Nitrobenzoyloxy ist,
in Gegenwart eines organischen Lösungsmittels wie Chloroform, Dichlormethan, Toluol, Ether, DMF, Aceton, Acetonitril oder Nitromethan oder deren Mischungen, eines Katalysators, wie para-Toluolsulfonsäure oder eines Trifluormethansulfonsäuretrialkylsilylesters und gegebenenfalls eines Säurefängers und eines Trockenmittels wie einem Molekularsieb, bei einer Reaktionstemperatur von -70 °C bis +30 °C unter einer Schutzgasatmosphäre wie Stickstoff oder Argon, zu einer Verbindung der

Formel I umsetzt,
wobei
$R^1$ die genannte Bedeutung hat,
$R^2$ zusammen mit $R^3$ eine Struktur der Formel III und
$R^4$ eine Struktur der Formel II oder IV ist, bei der
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben,
f) und gegebenenfalls man die Verbindung der Stufen b) bis e) an den Positionenen 7 und 9 entblockieren kann, indem man diese Verbindung in einem geeigneten organischen Lösungsmittel wie Chloroform, Dichlormethan, DMF, Toluol oder Methanol mit einem Katalysator wie verdünnte wässrige Lösungen von Carbonsäure oder para-Toluolsulfonsäure und gegebenenfalls mit einem Diol wie 2-Methyl-2,4-pentandiol, bei einer Temperatur zwischen 0 °C und der Siedetemperatur des Lösungsmittels zur Reaktion bringt, woraufhin man eine Verbindung der allgemeinen Formel I erhält, bei der
$R^1$ die genannte Bedeutung hat,
$R^2$ und $R^3$ Wasserstoff sind und
$R^4$ die genannte Bedeutung außer Wasserstoff hat,
g) woraufhin man gegebenenfalls die Verbindung der Stufe f) bei der $R^4$ eine Struktur der Formel II oder IV mit einer der in der Kohlenhydratchemie üblichen Schutzgruppen als Reste $R^5$ bis $R^8$ ist, unter in der Kohlenhydratchemie üblichen Bedingungen selektiv an den geschützten Hydroxyfunktionen und/oder an den geschützten Aminofunktionen in an sich bekannter Weise mittels einer anorganischen oder organischen Base wie Alkali- oder Erdalkalihydroxiden, Natriumcarbonat und Triethylamin in einem Lösungsmittel wie Wasser, Methanol, Ethanol oder THF oder deren Mischungen partiell oder vollständig entblockiert kann, wobei eine Verbindung der Formel I entsteht, bei der
$R^1$ Wasserstoff oder Hydroxyl,
$R^2$ und $R^3$ Wasserstoff und
$R^4$ eine Struktur der Formel II oder IV sind, in welchen
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ $N(Alkyl)_2$, Azido, Hydroxy oder Alkyloxy sind und
$R^8$ die gleiche Bedeutung wie $R^6$ hat, aber unabhängig davon ist,
h) und man gegebenenfalls die Verbindung der Stufe g) der Formel I, bei der
$R^1$ bis $R^3$ die unter g) genannte Bedeutung haben und
$R^4$ eine Struktur der Formel II, wobei
$R^5$, $R^6$ und $R^8$ unter g) angegebene Bedeutung haben und
$R^7$ $NH_2$ sind,
unter den an sich bekannten Bedingungen der reduktiven Aminierung in die entsprechend Verbindung der Formel I umsetzt, wobei
$R^1$ bis $R^3$ die unter g) genannte Bedeutung haben und
$R^4$ eine Struktur der Formel II ist,
bei der
$R^5$, $R^6$ und $R^8$ die unter g) genannte Bedeutung haben und $R^6$ $N(Alkyl)_2$ ist, oder
i) weiterhin eine Verbindung der Stufe g) der allgemeinen Formel I, bei der
$R^1$ bis $R^3$, die unter g) genannte Bedeutung haben und
$R^4$ eine Struktur der Formel II mit den für
$R^5$, $R^6$ und $R^7$ unter g) angegebenen Bedeutungen und
$R^7$ $NH_2$ sind,
durch Umsetzung mit Jodacetonitril oder Bromacetonitril in einem geeigneten Lösungsmittel, beispielsweise Dimethylformamid, in Gegenwart einer geeigneten Base wie Triethylamin in eine Verbindung der Formel I überführt, bei der $R^1$ bis $R^3$ die unter g) genannte Bedeutung haben und $R^4$ eine Struktur der Formel II ist, bei der
$R^5$, $R^6$ und $R^8$ die unter g) genannten Bedeutungen haben und
$R^7$ $N(CH_2CN)_2$ oder $NH(CH_2CN)$ ist, und
k) gegebenenfalls die bei der Stufe f), h) oder i) entstandene Verbindung nach den bereits bei der Stufe e) genannten Bedingungen glycosidiert, wobei je nach Menge des benutzten Glycosyldonors entweder nur Position 7 oder gleichzeitig Position 7 und 9 glycosidiert werden, was zu Produkten führt, die der allgemeinen Formel I entsprechen, bei der
$R^1$ die genannte Bedeutung hat,

$R^2$ eine Struktur der Formel II oder IV ist, bei der
$R^5$ bis $R^8$ die anfangs genannten Bedeutungen mit den Ausnahmen Hydroxymethyl, Hydroxy, $NH_2$, $N(CH_2CN)_2$ und $NH(CH_2CN)$ haben,
$R^3$ Wasserstoff ist oder $R^2$ entspricht und
$R^4$ Acyl, Trimethylsilyl oder eine Struktur der allgemeinen Formel II oder IV, wobei
$R^5$ bis $R^8$ die anfangs benannten Bedeutungen mit den Ausnahmen Hydroxymethylen, Hydroxy und $NH_2$ haben, ist, und gegebenenfalls
l) eine der bei der Stufe f), h) oder i) entstandenen Verbindungen in Gegenwart eines organischen Lösungsmittels wie Chloroform, Dichlormethan, Toluol, Ether, Aceton und Acetonitril oder deren Mischungen mit einem Glycal der Formel VI, wobei $R^5$ bis $R^7$ die bei Stufe e) genannte Bedeutung haben, mit N-Jodsuccinimid und gegebenenfalls mit einem Trockenmittel wie einem Molekularsieb bei einer Temperatur von -40 °C bis +40 °C unter einer Schutzgasatmosphäre wie Stickstoff oder Argon, zu einer Verbindung der Formel I überführen bei der
$R^1$ die genannte Bedeutung hat und
$R^2$ eine Struktur der Formel II ist, bei der
$R^5$ Wasserstoff, Methyl, Acyloxymethyl (C1-C8), Alkyloxymethyl (C1-C8),
$R^6$ Acyloxy, Alkyloxy, Allyloxy oder Benzyloxy,
$R^7$ Acyloxy, Alkyloxy, Allyloxy, Benzyloxy, NHAcyl, $N(Alkyl)_2$, Azido und
$R^8$ Jod ist, und
$R^3$ und $R^4$ die bei Stufe k) genannte Bedeutung haben, und
m) gegebenenfalls die bei der Stufe k) oder l) entstandene Verbindung gemäß den Bedingungen der Stufe g) entblockiert, wobei man eine Verbindung der Formel I erhält, bei der
$R^1$ die genannte Bedeutung hat, und
$R^2$ eine Struktur der Formel II oder IV ist, bei der
$R^5$ Wasserstoff, Methyl, Hydroxymethyl oder Alkyloxymethyl,
$R^6$ Wasserstoff, Hydroxy, Alkyloxy oder Halogen,
$R^7$ $NH_2$ oder $N(Alkyl)_2$, Azido, Hydroxy oder Alkyloxy ist und
$R^8$ die gleiche Bedeutung wie $R^6$ hat, aber unabhängig davon ist,
$R^3$ Wasserstoff ist oder $R^2$ entspricht und
$R^4$ Waserstoff, Trimethylsilyl oder eine Struktur der allgemeinen Formel II oder IV ist, wobei
$R^5$ Wasserstoff, Methyl, Hydroxymethylen oder Alkyloxymethyl,
$R^6$ Wasserstoff, Hydroxy, Alkyloxy oder Halogen,
$R^7$ $NH_2$, $N(Alkyl)_2$, $N(CH_2CN)_2$ oder $NH(CH_2CN)$, Azido, Hydroxy oder Alkyloxy ist und
$R^8$ die gleiche Bedeutung wie $R^6$ hat, aber unabhängig davon ist, oder
n) die bei der Stufe k) oder l) entstandene Verbindung unter den Bedingungen der Stufe g) auch so entblockiert, daß selectiv nur die Position 10 entblockiert wird und man eine Verbindung der Formel I erhält, bei der
$R^1$ die genannte Bedeutung hat,
$R^2$ eine Struktur der Formel II oder IV ist, bei denen $R^5$ bis $R^8$ die anfangs genannten Bedeutungen mit den Ausnahmen Hydroxymethyl, Hydroxy und $NH_2$ haben,
$R^3$ Wasserstoff ist oder $R^2$ entspricht und
$R^4$ Wasserstoff ist,
o) woraufhin sich gegebenenfalls die Verbindung der Stufe m), bei der $R^2$ eine Struktur der Formel II mit $R^7$ gleich $NH_2$ und $R^3$ Wasserstoff oder $R^2$ ist, wiederum in der für Stufe h) beschriebenen Art und Weise der reduktiven Aminierung in die ensprechenden Verbindungen umwandelt, bei der $R^6$ N-$(Alkyl)_2$, ist, oder gegebenenfalls
p) eine Verbindung der Stufe m), bei der $R^2$ eine Struktur der Formel II mit $R^7$ gleich $NH_2$ und $R^3$ Wasserstoff oder $R^2$ ist, gemäß den Bedingungen der Stufe i) in die entsprechenden Cyanomethyl-Derivate überführt, bei der
$R^7$ $N(CH_2CN)_2$ oder $NH(CH_2CN)$ ist, und gegebenenfalls
q) eine Verbindung der Stufe n) nach den bereits bei der Stufe e) genannten Bedingungen glycosidiert, wobei je nach Menge des benutzten Glycosyldonors entweder nur Position 10, oder gleichzeitig Positionen 9 und 10 glycosidiert werden, was zu einer Verbindung der Formel I führt, bei der
$R^1$ Wasserstoff oder Hydroxy ist und
$R^2$ eine Struktur der allgemeinen Formel II oder IV sind, bei der
$R^5$ bis $R^8$ die anfangs genannte Bedeutung mit den Ausnehmen von Hydroxymethyl, Hydroxy und $NH_2$ haben,

$R^3$ Wasserstoff oder $R^2$ oder $R^4$ ist und
$R^4$ eine Struktur der allgemeinen Formel II oder IV ist, bei der
$R^5$ bis $R^8$ die anfangs genannte Bedeutung mit den Ausnahmen von Hydroxymethyl, Hydroxy, $NH_2$, $N(CH_2CN)_2$, $NH(CH_2CN)$ haben, und gegebenenfalls
r) die Reaktion der Stufe c), d) und l) auch an einer Verbindung der Stufe n) durchführt, so daß man eine Verbindung der Formel I erhält, bei der
$R^1$ die anfangs genannte Bedeutung hat und
$R^2$ eine Struktur der Formel II oder IV ist, bei der
$R^5$ bis $R^8$ die anfangs genannte Bedeutung mit den Ausnahmen Hydroxymethyl, Hydroxy und $NH_2$ haben,
$R^3$ Wasserstoff oder $R^2$ oder $R^4$ ist und
$R^4$ eine Struktur der allgemeinen Formel II oder IV ist, bei der
$R^5$ bis $R^8$ die anfangs genannte Bedeutung mit den Ausnahmen Hydroxymethyl, Hydroxy, $NH_2$, $N(CH_2CN)_2$ und $NH(CH_2CN)$ haben und gegebenenfalls
s) diese Verbindung der Stufe q) oder r) wieder wie bei Stufe g) beschrieben entblockiert wird, bei der eine Verbindung der Formel I entsteht, bei der
$R^1$ die anfangs genannte Bedeutung hat und
$R^2$, $R^3$ und $R^4$ unabhängig voneinander Wasserstoff oder eine Struktur der Formel II oder IV sind, bei der
$R^5$ bis $R^8$ die anfangs genannte Bedeutung mit den Ausnahmen Acyloxymethyl, Acyloxy, Benzoyloxy oder substituiertes Benzoyloxy, NHAcyl, und gegebenenfalls
t) die Verbindung der Stufe s) entsprechend der Stufe h) mittels Reduktions-Aminierung in das entsprechende Derivat überführt wird, bei dem der Rest $R^7$, der in der Stufe s) eine $NH_2$ Gruppe war in $N(Alkyl)_2$ gewandelt ist, oder
u) des weiteren eine Verbindung der Stufe s) gemäß den Bedingungen der Stufe i) in das entsprechende Cyanomethyl-Derivat überführt wird, bei dem der Rest $R^7$, der in der Stufe s) eine $NH_2$ Gruppe war, in $NH(CH_2CN)$ gewandelt ist,
v) und man gegebenenfalls Verbindung der Formel I, bei der
$R^1$,$R^2$ und $R^3$ die genannte Bedeutung haben und
$R^4$ Trimethylsilyl ist,
in einem organischen Lösungsmittel wie THF, Diethylether, Dioxan oder deren Gemische bei Temperaturen zwischen -40 °C und der Siedetemperatur des Lösungsmittels mit Tetrabutylammoniumfluorid umsetzt zu einer Verbindung der Formel I, bei der $R^1$, $R^2$ und $R^3$ die oben genannte Bedeutung haben und $R^4$ Hydroxyl ist,
w) und man gegebenenfalls Verbindungen der Formel I, bei der $R^1$, $R^2$, $R^3$, $R^4$ die genannte Bedeutung haben und $R^7$ $NH_2$, $N(Alkyl)_2$ (C1-C8), $N(CH_2CN)_2$ oder $NH(CH_2CN)$ ist, in das Salz einer anorganischen oder organischen Säure überführt.

**2.** Verfahren zur Herstellung einer Verbindung nach Anspruch 1,
**dadurch gekennzeichnet,**
daß man eine Verbindung der Formel I, in welcher $R^1$ die anfangs genannte Bedeutung, hat, $R^2$ zusammen mit $R^3$ eine Verbindung der Formel III bilden und $R^4$ Wasserstoff ist, durch eine Acylierung mit einem Carbonsäureanhydrid wie Essigsäureanhydrid, Trifluoressigsäureanhydrid oder einem Phenylcarbonsäureanhydrid oder einem Carbonsäurehalogenid oder durch Umsetzung mit Trifluormethansulfonsäure-trimethylsilylester in einem organischen Lösungsmittel wie Chloroform, Dichlormethan oder Toluol oder deren Mischungen, bei einer Temperatur zwischen -40 °C und der Siedetemperatur des Lösungsmittels und in Gegenwart einer Base wie Triethylamin oder Pyridin zu einer Verbindung der Formel I umsetzt, bei der
$R^1$ die anfangs genannte Bedeutung hat,
$R^2$ zusammen mit $R^3$ eine Struktur der Formel III und
$R^4$ Acyl oder Trimethylsilyl ist, oder
daß man eine Verbindung der Formel I, in welcher $R^1$ die anfangs genannte Bedeutung hat, $R^2$ zusammen mit $R^3$ eine Verbindung der Formel III bilden und $R^4$ Wasserstoff ist, durch Umsetzung mit 3,4-Dihydro-2H-pyran in einem geeigenten organischen Lösungsmittel wie Chlorofoprm, Dichlormethan, DMF oder Toluol in Gegenwart eines Katalysators wie para-Toluolsulfonsäure und eines Trockenmittels wie 4Å-Molekularsieb, bei einer Temperatur zwischen -30 °C und der Siedetemperatur des Lösungsmittels in eine Verbindung der Formel I überführt, bei der
$R^1$ die anfangs genannte Bedeutung hat,

$R^2$ zusammen mit $R^3$ eine Struktur der Formel III
$R^4$ eine Struktur der Formel II mit $R^5$ bis $R^8$ Wasserstoff ist.

**3.** Verfahren zur Herstellung einer Verbindung nach Anspruch 1,
**dadurch gekennzeichnet,**
daß man eine Verbindung der Formel I, in welcher $R^1$ die anfangs genannte Bedeutung hat, $R^2$ zusammen mit $R^3$ eine Verbindung der Formel III bilden und $R^4$ die genannte Bedeutung außer Wasserstoff hat, an den Positionen 7 und 9 entblockieren kann, indem man diese Verbindung in einem geeigneten organischen Lösungsmittel wie Chloroform, Dichlormethan, DMF, Toluol oder Methanol mit einem Katalysator, wie verdünnte wässrige Lösungen von Carbonsäuren oder para-Toluolsulfonsäure und gegebenenfalls mit einem Diol wie 2-Methyl-2,4-pentandiol, bei einer Temperatur zwischen 0 °C und der Siedetemperatur des Lösungsmittels zur Reaktion bringt, woraufhin man eine Verbindung der allgemeinen Formel I erhält, bei der
$R^1$ die genannte Bedeutung hat,
$R^2$ und $R^3$ Wasserstoff sind und
$R^4$ die genannte Bedeutung außer Wasserstoff hat.

**4.** Verfahren zur Herstellung einer Verbindung nach Anspruch 1,
**dadurch gekennzeichnet,**
daß man eine Verbindung der Formel I, in welcher $R^1$ bis $R^4$ die genannte Bedeutung haben, aber $R^2$ oder $R^4$ Wasserstoff sein müssen und $R^5$ bis $R^8$ nicht Hydroxy, Hydroxymethyl oder $NH_2$ sein können mit einem funktionalisierten Kohlenhydrat der allgemeinen Formel V oder VI,

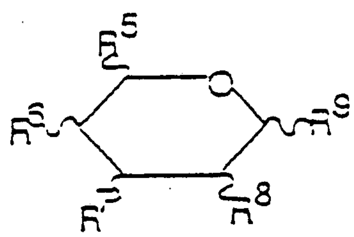

V

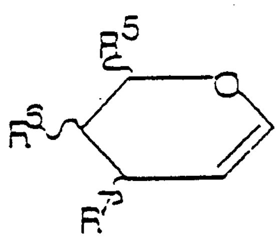

VI

worin
$R^5$ bis $R^8$ die anfangs genannte Bedeutungen mit den Ausnahmen Hydroxymethyl, Hydroxy, $NH_2$, $N(CH_2CN)_2$ und $NH(CH_2CN)$ haben und
$R^9$ über Sauerstoff gebundene Acylschutzgruppe wie aliphatisches Acyloxy (C1-C8) wie Acetyl, Benzoyloxy, oder substituiertes Benzyloxy wie para-Nitrobenzoyloxy ist,
in Gegenwart eines organischen Lösungsmittels wie Chloroform, Dichlormethan, Toluol, Ether, DMF, Aceton, Acetonitril oder Nitromethan oder deren Mischungen, eines Katalysators, wie para-Toluolsulfonsäure oder eines Trifluormethansulfonsäure-trialkylsilylesters und gegebenenfalls eines Säurefängers und eines Trockenmittels wie einem Molekularsieb, bei einer Reaktionstemperatur von -70 °C bis +30 °C unter einer Schutzgasatmosphäre wie Stickstoff oder Argon, zu einer Verbindung der Formel I umsetzt, wobei
$R^1$ die genannte Bedeutung hat,
$R^2$ zusammen mit $R^3$ eine Struktur der Formel III oder $R^2$, $R^3$ oder $R^4$ eine Struktur der Formel II oder IV ist, bei der
$R^5$ bis $R^8$ die anfangs genannte Bedeutungen mit den Ausnahmen Hydroxymethyl, Hydroxy, $NH_2$ haben, oder
daß man eine Verbindung der Formel I, in welcher $R^1$ bis $R^4$ die genannte Bedeutungen haben, aber $R^2$ oder $R^4$ Wasserstoff sein müssen und $R^5$ bis $R^8$ nicht Hydroxy, Hydroxymethyl oder $NH_2$ sein können in Gegenwart eines organischen Lösungsmittels wie Chloroform, Dichlormethan, Toluol, Ether, Aceton oder Acetonitril oder deren Mischungen mit einem Glycal oder Formel VI, wobei $R^5$ bis $R^7$ die anfangs genannten Bedeutungen mit den Ausnahmen Hydroxymethyl, Hydroxy, $NH_2$, $N(CH_2CN)_2$ und $NH(CH_2CN)$ haben, mit N-Jodsuccinimid und gegebenenfalls mit einem Trockenmittel wie einem Molekularsieb bei einer Temperatur von -40 °C bis +40 °C unter einer Schutzgasatmosphäre wie Stickstoff

oder Argon, zu einer Verbindung der Formel I überführen, wobei
$R^1$ die genannte Bedeutung hat,
$R^2$ zusammen mit $R^3$ eine Struktur der Formel III oder $R^2$, $R^3$ oder
$R^4$ eine Struktur der Formel II oder IV ist, bei der
$R^5$ bis $R^8$ die anfangs genannten Bedeutungen mit den Ausnahmen Hydroxymethyl, Hydroxy, $NH_2$, N-$(CH_2CN)_2$ und $NH(CH_2CN)$ haben.

## Claims

## Claims for the following Contracting States : AT, BE, CH, DE, FR, GB, IT, LI, LU, NL, SE

**1.** A compound of the formula I or a salt thereof with an inorganic or organic acid

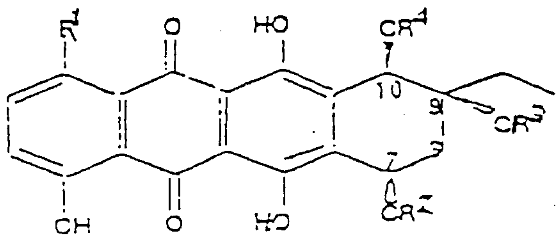

I

in which:
$R^1$ is hydrogen or a hydroxyl group
$R^2$ is hydrogen or a structure of the formula II or IV or, together with $R^3$, of the formula III

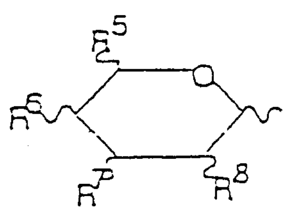

II

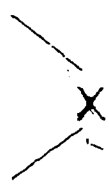

III

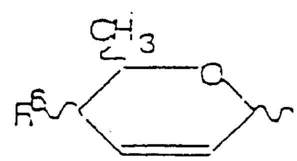

IV

$R^3$ is hydrogen or a structure of the formula II or IV or, together with $R^2$, of the formula III,
$R^4$ is hydrogen, trimethylsilyl, a protective group which is customary in carbohydrate chemistry, preferably acetyl, trifluoroacetyl, benzoyl or substituted benzoyl, such as para-nitrobenzoyl, a structure of the formula II or a structure of the formula IV,
$R^5$ is hydrogen, methyl, hydroxymethyl, acyloxymethyl, (C1-C8) or alkoxymethyl (C1-C8),
$R^6$, $R^7$ and $R^8$ independently of one another are hydrogen, hydroxyl, aliphatic acyloxy (C1-C8), benzoyloxy or substituted benzoyloxy, such as para-nitrobenzoyloxy, alkoxy (C1-C8), allyloxy, benzyloxy or substituted benzyloxy or halogen, and $R^7$ can furthermore be $NH_2$, NHacyl (C1-C8), $N(alkyl)_2$ (C1-C8), $N(CH_2CN)_2$, $NH(CH_2CN)$ or azido, and
X is a bidentate protective group which is customary in carbohydrate chemistry, preferably alkylboronyl, phenylboronyl, an alkyl orthoalkanoate, preferably methyl orthoformate or ethyl orthoacetate, or a ketal or acetal, preferably isopropylidene or benzylidene,
the compounds where $R^1 = R^2 = R^3 = R^4 = H$, $R^1 = OH$ and $R^2 = R^3 = R^4 = H$, $R^1 = R^3 = R^4 = H$ and $R^2 = \alpha$-L-daunosaminyl or $\alpha$-L-rhodosaminyl or 4'-acyl-$\alpha$-L-rhodosaminyl, $R^1 = OH$ or H, $R^3 = H$ and $R^2 = R^4 = \alpha$-L-rhodosaminyl or 4'-acyl-$\alpha$-L-rhodosaminyl and $R^1 = R^2 = R^3 = H$ and $R^4 = \alpha$-L-rhodosaminyl or $\alpha$-L-daunosaminyl and $R^1 = OH$, $R^3 = R^4 = H$ and $R^2$ = rhodosaminyl or daunosaminyl being excluded.

**2.** A compound as claimed in claim 1,
wherein
$R^2$ and $R^3$ independently of one another are hydrogen or a structure of the formula II or IV with the meanings of $R^5$ to $R^8$ given in claim 1.

**3.** A compound as claimed in claim 1, wherein $R^2$ and $R^3$ independently of one another are hydrogen or a structure of the formula II or IV in which $R^5$ is methyl, $R^6$ and $R^7$ have the meanings given in claim 1 and $R^8$ is hydrogen or halogen.

**4.** A compound as claimed in claim 1, wherein $R^2$ and $R^3$ independently of one another are hydrogen or a structure of the formula II or IV in which $R^5$ is methyl, $R^6$ has the meaning given in claim 1, $R^7$ is $NH_2$, NHacyl (C1-C8), $N(alkyl)_2$ (C1-C8), N-$(CH_2CN)_2$, $NH(CH_2CN)$ or azido and $R^8$ is hydrogen or halogen.

**5.** A compound as claimed in claim 1, wherein $R^2$ and $R^3$ independently of one another are hydrogen or a structure of the formula II or IV, in which $R^5$ is methyl, $R^6$ and $R^7$ have the meanings given in claim 1 and $R^8$ is hydrogen or halogen and the structure of the formula II or IV belong to the L-series of carbohydrates.

**6.** A compound as claimed in claim 1, wherein $R^2$ and $R^3$ independently of one another are hydrogen or a structure of the formula II or IV, in which $R^5$ is methyl, $R^6$ has the meaning given in claim 1, $R^7$ is $NH_2$, NHacyl (C1-C8), $N(alkyl)_2$ (C1-C8), N-$(CH_2CN)_2$, $NH(CH_2CH)$ or azido and $R^8$ is hydrogen or halogen, and the structures of the formula II or IV belong to the L-series of carbohydrates.

**7.** A process for the preparation of a compound as claimed in any one of claims 1 to 6, which comprises

a) reacting a compound of the formula I in which $R^1$ has the meaning initially given and $R^2$ to $R^4$ are hydrogen with a boric acid, such as phenylboric acid, or with a ketone, such as acetone, or a ketal, such as 2,2-dimethoxypropane, or an acetal, such as benzaldehyde dimethyl acetal, in a suitable organic solvent, such as toluene or dimethylformamide or mixtures thereof, with a catalyst, such as a mineral, carboxylic or sulfonic acid, at a temperature between 0 °C and the boiling point of the solvent, if appropriate with the addition of a dehydrating agent, such as 4Å molecular sieve, to give a compound of the formula I in which $R^1$ has the meaning initially given and $R^2$ together with $R^3$ forms a compound of the formula III, which is isolated by filtration and by removal of the solvent and crystallized out of an organic solvent, such as petroleum ether, after which the hydroxyl group on position. 10 is derivatized in a suitable manner,

b) if appropriate by acylating with a carboxylic acid anhydride, such as acetic anhydride or trifluoroacetic anhydride, or a phenylcarboxylic acid anhydride or a carboxylic acid halide or by reacting with trimethylsilyl trifluoromethanesulfonate in a suitable organic solvent, such as chloroform, methylene chloride, toluene or mixtures thereof, at a temperature between -40 °C and the boiling point of the solvent and in the presence of a base, such as triethylamine or pyridine, to give a compound of the formula I in which
$R^1$ has the meaning initially given,
$R^2$ together with $R^3$ is a structure of the formula III and
$R^4$ is acyl or trimethylsilyl,

c) or, for example, by reacting the compound obtained under a) with 3,4-dihydro-2H-pyran in a suitable organic solvent, such as chloroform, methylene chloride, dimethylformamide or toluene, in the presence of a catalyst, such as para-toluenesulfonic acid, and a desiccant, such as 4Å molecular sieve, at a temperature between -30 °C and the boiling point of the solvent, to give a compound of the formula I in which
$R^1$ has the meaning initially given
$R^2$ together with $R^3$ is a structure of the formula III and
$R^4$ is a structure of the formula II, where $R^5$ to $R^8$ is hydrogen,

d) or, for example, by reacting the compound obtained under a) with a carbohydrate derivative of the formula V

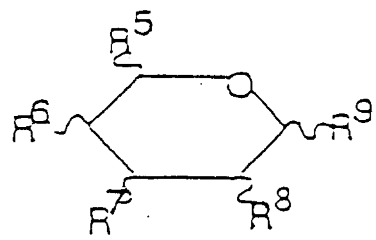

V

in which
$R^5$ to $R^8$, as suitable protective groups, have the meanings initially given and
$R^9$ is halogen, such as Cl or Br, O-acyl or another leaving group which is customary for glycosidation reactions,
under the conditions customary in carbohydrate chemistry, to give a compound of the formula I in which
$R^1$ has the meaning initially given
$R^2$ together with $R^3$ is a structure of the formula III and
$R^4$ is a structure of the formula II,
e) or, for example, by reacting the compound obtained under a) with a functionalized carbohydrate of the general formula V or VI

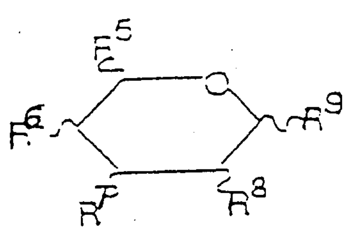

V

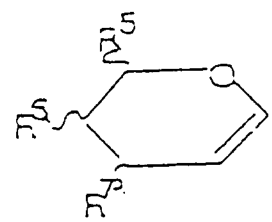

VI

in which
$R^5$ to $R^8$ have the meanings initially given, with the exception of hydroxymethyl, hydroxyl, $NH_2$, N-$(CH_2CN)_2$ and $NH(CH_2CN)$ and
$R^9$ is acyl which is bonded via oxygen, such as aliphatic acyloxy (C1-C8), such as acetyl, benzoyloxy or substituted benzoyloxy, such as paranitrobenzoyloxy,
in the presence of an organic solvent, such as chloroform, methylene chloride, toluene, ether, dimethylformamide, acetone, acetonitrile or nitromethane or mixtures thereof, a catalyst, such as para-toluenesulfonic acid or a trialkylsilyl trifluoromethanesulfonate, and if appropriate an acid-trapping agent and a desiccant, such as molecular sieve, at a reaction temperature of -70 ° C to + 30 ° C under a protective gas atmosphere, such as nitrogen or argon, to give a compound of the formula I
in which
$R^1$ has the meaning given
$R^2$ together with $R^3$ is a structure of the formula III and
$R^4$ is a structure of the formula II or IV, in which
$R^5$ to $R^8$ have the meanings initially given, with the exception of hydroxymethyl, hydroxyl, $NH_2$, N-$(CH_2CN)_2$ and $NH(CH_2CN)$,
f) and, if appropriate, possibly deblocking the compound of stages b) to e) on positions 7 and 9 by reacting this compound in a suitable organic solvent, such as chloroform, methylene chloride, dimethylformamide, toluene or methanol, with a catalyst, such as dilute aqueous solutions of carboxylic acids or paratoluenesulfonic acid, and if appropriate with a diol, such as 2-methyl-2,4-pentanediol, at a temperature between 0 ° C and the boiling point of the solvent, to give a compound of the formula I in which

$R^1$ has the meaning given,
$R^2$ and $R^3$ are hydrogen and
$R^4$ has the meaning given, with the exception of hydrogen,
g) and subsequently, if appropriate, possibly partly or completely deblocking the compound of stage f) in which $R^4$ is a structure of the formula II or IV with, as radicals $R^5$ to $R^8$, one of the protective groups customary in carbohydrate chemistry, in a manner which is known per se, selectively on the protected hydroxyl functions and/or on the protected amino functions under the conditions customary in carbohydrate chemistry by means of an inorganic or organic base, such as alkali metal or alkaline earth metal hydroxides, sodium carbonate and triethylamine, in a solvent, such as water, methanol, ethanol or tetrahydrofuran or mixtures thereof, to give a compound of the formula I in which
$R^1$ is hydrogen or hydroxyl,
$R^2$ and $R^3$ are hydrogen and
$R^4$ is a structure of the formula II or IV, in which
$R^5$ is hydrogen, methyl, hydroxymethyl or alkoxymethyl,
$R^6$ is hydrogen, hydroxyl, alkoxy or halogen,
$R^7$ is $NH_2$ $N(alkyl)_2$, azido, hydroxyl or alkoxy and
$R^8$ has the same meaning as $R^6$ but is independent thereof,
h) and, if appropriate, reacting the compound of stage g) of the formula I, in which
$R^1$ to $R^3$ have the meanings given under g) and
$R^4$ is a structure of the formula II, in which
$R^5$, $R^6$ and $R^8$ have the meanings given under g) and
$R^7$ is $NH_2$,
under the conditions which are known per se for reductive amination, to give the corresponding compound of the formula I in which
$R^1$ to $R^3$ have the meanings given under g) and
$R^4$ is a structure of the formula II,
in which
$R^5$, $R^6$ and $R^8$ have the meanings given under g) and
$R^6$ is $N(alkyl)_2$, or
i) furthermore converting a compound of stage g) of the formula I in which
$R^1$ to $R^3$ have the meanings given under g) and
$R^4$ is a structure of the formula II in which
$R^5$, $R^6$ and $R^7$ have the meanings given under g) and
$R^7$ is $NH_2$,
by reaction with iodoacetonitrile or bromoacetonitrile in a suitable solvent, for example dimethylformamide, in the presence of a suitable base, such as triethylamine, into a compound of the formula I in which $R^1$ to $R^3$ have the meanings given under g) and $R^4$ is a structure of the formula II, in which
$R^5$, $R^6$ and $R^8$ have the meanings given under g) and
$R^7$ is $N(CH_2CN)_2$, $NH(CH_2CN)$, and
k) if appropriate glycosidating the compound formed in stage f), h) or i) under the conditions already mentioned in stage e), either only position 7 or simultaneously positions 7 and 9 being glycosidated, depending on the amount of glycosyl donor used, to give products which correspond to the formula I in which
$R^1$ has the meaning given,
$R^2$ is a structure of the formula II or IV, in which
$R^5$ to $R^8$ have the meanings initially given, with the exception of hydroxymethyl, hydroxyl, $NH_2$, $N(CH_2CN)_2$, $NH(CH_2CN)$,
$R^3$ is hydrogen or corresponds to $R^2$ and
$R^4$ is acyl, trimethylsilyl or a structure of the formula II or IV in which
$R^5$ to $R^8$ have the meanings initially mentioned, with the exception of hydroxymethyl, hydroxyl and $NH_2$, and, if appropriate,
l) converting one of the compounds formed in stage f), h) or i) with a glycal of the formula VI, in which $R^5$ to $R^7$ have the meanings given in stage e), in the presence of an organic solvent, such as chloroform, methylene chloride, toluene, ether, acetone or acetonitrile or mixtures thereof, with N-iodosuccinimide and if appropriate with a desiccant, such as molecular sieve, at a temperature from -40 °C to +40 °C under a protective gas atmosphere, such as nitrogen or argon, into a compound of the formula I in which

$R^1$ has the meaning given and
$R^2$ is a structure of the formula II, in which
$R^5$ is hydrogen, methyl, acyloxymethyl (C1-C8) or alkoxymethyl (C1-C8),
$R^6$ is acyloxy, alkyloxy, allyloxy or benzyloxy,
$R^7$ is acyloxy, alkyloxy, allyloxy, benzyloxy, NHacyl, $N(alkyl)_2$ or azido and
$R^8$ is iodine, and
$R^3$ and $R^4$ have the meanings given in stage k), and
m) if appropriate deblocking the compound formed in stage k) or l) in accordance with the conditions of stage g), to give a compound of the formula I in which
$R^1$ has the meaning given and
$R^2$ is a structure of the formula II or IV, in which
$R^5$ is hydrogen, methyl, hydroxymethyl or alkyloxymethyl,
$R^6$ is hydrogen, hydroxyl, alkyloxy or halogen,
$R^7$ is $NH_2$ or $N(alkyl)_2$, azido, hydroxyl or alkoxy and
$R^8$ has the same meaning as $R^6$, but is independent thereof,
$R^3$ is hydrogen or corresponds to $R^2$ and
$R^4$ is hydrogen, trimethylsilyl or a structure of the formula II or IV, in which
$R^5$ is hydrogen, methyl, hydroxymethyl or alkoxymethyl,
$R^6$ is hydrogen, hydroxyl, alkoxy or halogen,
$R^7$ is $NH_2$, $N(alkyl)_2$, $N(CH_2CN)_2$, $NH(CH_2CN)$, azido, hydroxyl or alkoxy and
$R^8$ has the same meaning as $R^6$, but is independent thereof, or
n) also deblocking the compound formed in stage k) or l) under the conditions of stage g) so that only position 10 is selectively deblocked, to give a compound of the formula I in which
$R^1$ has the meaning given,
$R^2$ is a structure of the formula II or IV, in which
$R^5$ to $R^8$ have the meanings initially given, with the exception of hydroxymethyl, hydroxyl and $NH_2$,
$R^3$ is hydrogen or corresponds to $R^2$ and
$R^4$ is hydrogen,
o) and subsequently, if appropriate, converting the compound of stage m), in which $R^2$ is a structure of the formula II where $R^7$ is $NH_2$ and $R^3$ is hydrogen or $R^2$, into the corresponding compounds in which $R^6$ is $N(alkyl)_2$, again in the manner of reductive amination described for stage h), or, if appropriate,
p) converting a compound of stage m) in which $R^2$ is a structure of the formula II, where $R^7$ is $NH_2$ and $R^3$ is hydrogen or $R^2$, into the corresponding cyanomethyl drivatives in which
$R^7$ is $N(CH_2CN)_2$ or $NH(CH_2CN)$ in accordance with the conditions of stage i), and, if appropriate,
q) glycosidating a compound of stage n) under the conditions already given in stage e), either only position 10 or simultaneously positions 9 and 10 being glycosidated, depending on the amount of glycosyl donor used, to give a compound of the formula I in which
$R^1$ is hydrogen or hydroxyl and
$R^2$ is a structure of the formula II or IV, in which
$R^5$ to $R^8$ have the meanings initially given, with the exception of hydroxymethyl, hydroxyl and $NH_2$,
$R^3$ is hydrogen or $R^2$ or $R^4$ and
$R^4$ is a structure of the formula II or IV, in which
$R^5$ to $R^8$ have the meanings initially given, with the exception of hydroxymethyl, hydroxyl, $NH_2$, $N(CH_2CN)_2$ or $NH(CH_2CN)$, and, if appropriate,
r) also carrying out the reaction of stages c), d) and l) on a compound of stage n) so as to give a compound of the formula I in which
$R^1$ has the meaning initially given and
$R^2$ is a structure of the formula II or IV, in which
$R^5$ to $R^8$ have the meanings initially given, with the exception of hydroxymethyl, hydroxyl and $NH_2$,
$R^3$ is hydrogen or $R^2$ or $R^4$ and
$R^4$ is a structure of the formula II or IV in which
$R^5$ to $R^8$ have the meanings initially given, with the exception of hydroxymethyl, hydroxyl, $NH_2$, $N(CH_2CN)_2$ or $NH(CH_2CN)$, and, if appropriate,
s) deblocking this compound of stage q) or r) again as described in stage g), to give a compound of the formula I in which
$R^1$ has the meaning initially given and
$R^2$, $R^3$ and $R^4$ independently of one another are hydrogen or a structure of the formula II or IV, in

which
$R^5$ to $R^8$ have the meanings initially given, with the exception of acyloxymethyl, acyloxy, benzoyloxy or substituted benzoyloxy or NHacyl, and, if appropriate,
t) converting the compound of stage s) into the corresponding derivative in which the radical $R^7$, which in stage s) was an $NH_2$ group, is converted into $N(alkyl)_2$, by means of reduction amination in accordance with stage h), or
u) moreover converting a compound of stage s) into the corresponding cyanomethyl derivative in which the radical $R^7$, which in stage s) was an $NH_2$ group, into $NH(CH_2CN)$ in accordance with the conditions of stage i),
v) and, if appropriate, reacting a compound of the formula I in which
$R^1$, $R^2$ and $R^3$ have the meanings given and
$R^4$ is trimethylsilyl,
with tetrabutylammonium fluoride in an organic solvent, such as tetrahydrofuran, diethyl ether, dioxane or mixtures thereof, at temperatures between -40 °C and the boiling point of the solvent, to give a compound of the formula I in
which $R^1$, $R^2$ and $R^3$ have the meanings given above and
$R^4$ is hydroxyl,
w) and, if appropriate, converting compounds of the formula I in which $R^1$, $R^2$, $R^3$ and $R^4$ have the meanings given and $R^7$ is $NH_2$, $N(alkyl)_2$ (C1-C8), $N(CH_2CN)_2$ or $NH(CH_2CN)$ into the salt of an inorganic or organic acid.

**8.** The process as claimed in claim 7,
wherein
a compound of the formula I in which $R^1$ has the meaning initially given, $R^2$ together with $R^3$ forms a structure of the formula III and $R^4$ is hydrogen, is converted by acylation with a carboxylic acid anhydride, such as acetic anhydride or trifluoroacetic anhydride, or a phenylcarboxylic acid anhydride or a carboxylic acid halide or by reaction with trimethylsilyl trifluoromethanesulfonate in an organic solvent, such as chloroform, methylene chloride or toluene or mixtures thereof, at a temperature between -40 °C and the boiling point of the solvent and in the presence of a base, such as triethylamine or pyridine, into a compound of the formula I in which
$R^1$ has the meaning initially given,
$R^2$ together with $R^3$ is a structure of the formula III and
$R^4$ is acyl or trimethylsilyl, or
wherein a compound of the formula I in which $R^1$ has the meaning initially given, $R^2$ together with $R^3$ forms a structure of the formula III and $R^4$ is hydrogen, is converted by reaction with 3,4-dihydro-2H-pyran in a suitable organic solvent, such as chloroform, methylene chloride, dimethylformamide or toluene, in the presence of a catalyst, such as para-toluenesulfonic acid, and a desiccant, such as 4Å molecular sieve, at a temperature between -30 °C and the boiling point of the solvent, into a compound of the formula I in which
$R^1$ has the meaning initially given,
$R^2$ together with $R^3$ is a structure of the formula III and
$R^4$ is a structure of the formula II where $R^5$ to $R^8$ are hydrogen.

**9.** The process as claimed in claim 7,
wherein
A compound of the formula I in which $R^1$ has the meaning initially given, $R^2$ together with $R^3$ forms a structure of the formula III and $R^4$ has the meaning given, excluding hydrogen, can be deblocked at positions 7 and 9 by reacting this compound in a suitable organic solvent, such as chloroform, methylene chloride, dimethylformamide, toluene or methanol, with a catalyst, such as dilute aqueous solutions of carboxylic acids or para-toluenesulfonic acid, and if appropriate with a diol, such as 2-methyl-2,4-pentanediol, at a temperature between 0 °C and the boiling point of the solvent, to give a compound of the formula I in which
$R^1$ has the meaning given,
$R^2$ and $R^3$ are hydrogen and
$R^4$ has the meaning given, with the exception of hydrogen.

**10.** The process as claimed in claim 7,
wherein

a compound of the formula I in which $R^1$ or $R^4$ have the meanings given, but $R^2$ or $R^4$ must be hydrogen and $R^5$ to $R^8$ cannot be hydroxyl, hydroxymethyl or $NH_2$, is reacted with a functionalized carbohydrate of the formula V or VI

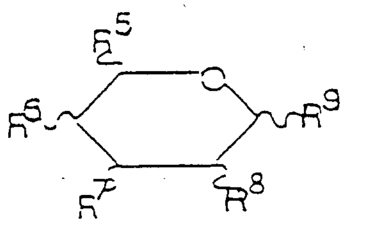

V

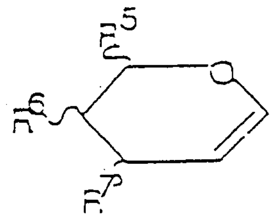

VI

in which
$R^5$ to $R^8$ have the meanings initially given, with the exception of hydroxymethyl, hydroxyl, $NH_2$, N-$(CH_2CN)_2$ and $NH(CH_2CN)$ and
$R^9$ is an acyl protective group bonded via oxygen, such as aliphatic acyloxy (C1-C8), such as acetyl, benzoyloxy or substituted benzoyloxy, such as paranitrobenzoyloxy,
in the presence of an organic solvent, such as chloroform, methylene chloride, toluene, ether, dimethylformamide, acetone, acetonitrile or nitromethane or mixtures thereof, a catalyst, such as para-toluenesulfonic acid or a trialkylsilyl trifluoromethanesulfonate, and if appropriate an acid-trapping agent and a desiccant, such as molecular sieve, at a reaction temperature of -70 °C to +30 °C under a protective gas atmosphere, such as nitrogen or argon, to give a compound of the formula I in which
$R^1$ has the meaning given,
$R^2$ together with $R^3$ is a structure of the formula III or $R^2$, $R^3$ or $R^4$ is a structure of the formula II or IV, in which
$R^5$ to $R^8$ have the meanings initially given, with the exception of hydroxymethyl, hydroxyl and $NH_2$, or wherein a compound of the formula I in which $R^1$ to $R^4$ have the meanings given, but $R^2$ or $R^4$ must be hydrogen and $R^5$ to $R^8$ cannot be hydroxyl, hydroxymethyl or $NH_2$, is converted in the presence of an organic solvent, such as chloroform, methylene chloride, toluene, ether, acetone or acetonitrile or mixtures thereof, with a glycal of the formula VI, in which $R^5$ to $R^7$ have the meanings initially given, with the exception of hydroxymethyl, hydroxyl, $NH_2$, $N(CH_2CN)_2$ and $NH(CH_2CN)$, with N-iodosuccinimide and if appropriate with a desiccant, such as molecular sieve, at a temperature of -40 °C to +40 °C under a protective gas atmosphere, such as nitrogen or argon, into a compound of the formula I in which
$R^1$ has the meaning given,
$R^2$ together with $R^3$ is a structure of the formula III or $R^2$, $R^3$ or $R^4$ is a structure of the formula II or IV, in which
$R^5$ to $R^8$ have the meanings initially given, with the exception of hydroxymethyl, hydroxyl, $NH_2$, N-$(CH_2CN)_2$ and $NH(CH_2CN)$.

**11.** The use of a compound as claimed in any one of claims 1 to 10 for the production of a pharmaceutical.

**Claims for the following Contracting States : ES, GR**

**1.** A process for the preparation of a compound of the formula I or if appropriate of a salt thereof with an inorganic or organic acid

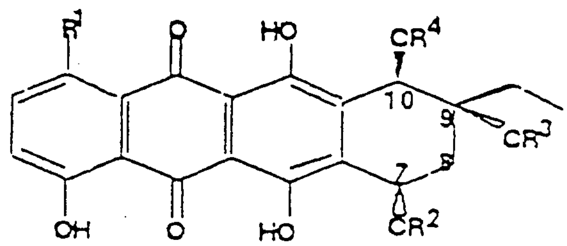

I

in which:
$R^1$ is hydrogen or a hydroxyl group
$R^2$ is hydrogen or a structure of the formula II or IV
or, together with $R^3$, of the formula III

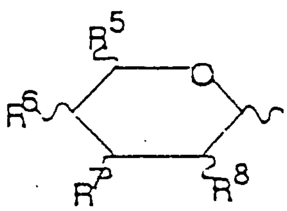

II

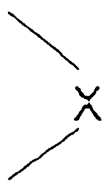

III

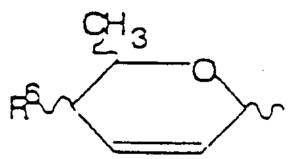

IV

$R^3$ is hydrogen or a structure of the formula II or IV or, together with $R^2$, of the formula III,
$R^4$ is hydrogen, trimethylsilyl, a protective group which is customary in carbohydrate chemistry, preferably acetyl, trifluoroacetyl, benzoyl or substituted benzoyl, such as para-nitrobenzoyl, a structure of the formula II or a structure of the formula IV,
$R^5$ is hydrogen, methyl, hydroxymethyl, acyloxymethyl, (C1-C8) or alkoxymethyl (C1-C8),
$R^6$, $R^7$ and $R^8$ independently of one another are hydrogen, hydroxyl, aliphatic acyloxy (C1-C8), benzoyloxy or substituted benzoyloxy, such as para-nitrobenzoyloxy, alkoxy (C1-C8), allyloxy, benzyloxy or substituted benzyloxy or halogen, and $R^7$ can furthermore be $NH_2$, NHacyl (C1-C8), $N(alkyl)_2$ (C1-C8), $N(CH_2CN)_2$, $NH(CH_2CN)$ or azido, and
X is a bidentate protective group which is customary in carbohydrate chemistry, preferably alkylboronyl, phenylboronyl, an alkyl orthoalkanoate, preferably methyl orthoformate or ethyl orthoacetate, or a ketal or acetal, preferably isopropylidene or benzylidene,
the compounds where $R^1 = R^2 = R^3 = R^4 = H$, $R^1 = OH$ and $R^2 = R^3 = R^4 = H$, $R^1 = R^3 = R^4 = H$ and $R^2 = \alpha$-L-daunosaminyl or $\alpha$-L-rhodosaminyl or 4'-acyl-$\alpha$-L-rhodosaminyl, $R^1 = OH$ or H, $R^3 = H$ and $R^2 = R^4 = \alpha$-L-rhodosaminyl or 4'-acyl-$\alpha$-L-rhodosaminyl and $R^1 = R^2 = R^3 = H$ and $R^4 = \alpha$-L-rhodosaminyl and $R^1 = OH$, $R^3 = R^4 = H$ and $R^2 =$ rhodosaminyl or daunosaminyl being excluded, which comprises

a) reacting a compound of the formula I in which $R^1$ has the meaning initially given and $R^2$ to $R^4$ are hydrogen with a boric acid, such as phenylboric acid, or with a ketone, such as acetone, or a ketal, such as 2,2-dimethoxypropane, or an acetal, such as benzaldehyde dimethyl acetal, in a suitable organic solvent, such as toluene or dimethylformamide or mixtures thereof, with a catalyst, such as a mineral, carboxylic or sulfonic acid, at a temperature between 0 °C and the boiling point of the solvent, if appropriate with the addition of a dehydrating agent, such as 4A molecular sieve, to give a compound of the formula I in which $R^1$ has the meaning initially given and $R^2$ together with $R^3$ forms a compound of the formula III, which is isolated by filtration and by removal of the solvent and crystallized out of an organic solvent, such as petroleum ether, after which the hydroxyl group on position 10 is derivatized in a suitable manner,

b) if appropriate by acylating with a carboxylic acid anhydride, such as acetic anhydride or trifluoroacetic anhydride, or a phenylcarboxylic acid anhydride or a carboxylic acid halide or by reacting with trimethylsilyl trifluoromethanesulfonate in a suitable organic solvent, such as chloroform, methylene chloride, toluene or mixtures thereof, at a temperature between -40 °C and the boiling point of the solvent and in the presence of a base, such as triethylamine or pyridine, to give a compound of the formula I in which
$R^1$ has the meaning initially given,

$R^2$ together with $R^3$ is a structure of the formula III and
$R^4$ is acyl or trimethylsilyl,
c) or, for example, by reacting the compound obtained under a) with 3,4-dihydro-2H-pyran in a suitable organic solvent, such as chloroform, methylene chloride, dimethylformamide or toluene, in the presence of a catalyst, such as paratoluenesulfonic acid, and a desiccant, such as 4Å molecular sieve, at a temperature between -30 °C and the boiling point of the solvent, to give a compound of the formula I in which
$R^1$ has the meaning initially given,
$R^2$ together with $R^3$ is a structure of the formula III and
$R^4$ is a structure of the formula II, where
$R^5$ to $R^8$ is hydrogen,
d) or, for example, by reacting the compound obtained under a) with a carbohydrate derivative of the formula V

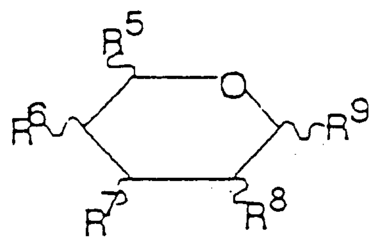

V

in which
$R^5$ to $R^8$, as suitable protective groups, have the meanings initially given and
$R^9$ is halogen, such as Cl or Br, O-acyl or another leaving group which is customary for glycosidation reactions,
under the conditions customary in carbohydrate chemistry, to give a compound of the formula I in which
$R^1$ has the meaning initially given,
$R^2$ together with $R^3$ is a structure of the formula III and
$R^4$ is a structure of the formula II,
e) or, for example, by reacting the compound obtained under a) with a functionalized carbohydrate of the general formula V or VI

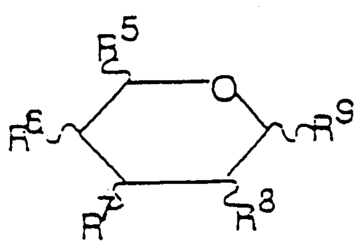

V

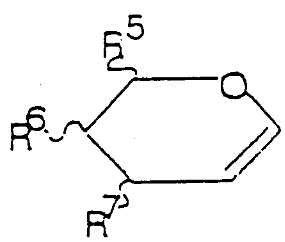

VI

in which
$R^5$ to $R^8$ have the meanings initially given, with the exception of hydroxymethyl, hydroxyl, $NH_2$, N-$(CH_2CN)_2$ and $NH(CH_2CN)$ and
$R^9$ is acyl which is bonded via oxygen, such as aliphatic acyloxy (C1-C8), such as acetyl, benzoyloxy or substituted benzoyloxy, such as para-nitrobenzoyloxy,
in the presence of an organic solvent, such as chloroform, methylene chloride, toluene, ether, dimethylformamide, acetone, acetonitrile or nitromethane or mixtures thereof, a catalyst, such as para-toluene-sulfonic acid or a trialkylsilyl trifluoromethane-sulfonate, and if appropriate an acid-trapping agent and a desiccant, such as molecular sieve, at a reaction temperature of -70°C to +30°C under a protective gas atmosphere, such as nitrogen or argon, to give a compound of the formula I
in which
$R^1$ has the meaning given,
$R^2$ together with $R^3$ is a structure of the formula III and
$R^4$ is a structure of the formula II or IV, in which
$R^5$ to $R^8$ have the meanings initially given, with the exception of hydroxymethyl, hydroxyl, $NH_2$, N-$(CH_2CN)_2$ and $NH(CH_2CN)$,
f) and, if appropriate, possibly deblocking the compound of stages b) to e) on positions 7 and 9 by reacting this compound in a suitable organic solvent, such as chloroform, methylene chloride, dimethylformamide, toluene or methanol, with a catalyst, such as dilute aqueous solutions of carboxylic acids or paratoluenesulfonic acid, and if appropriate with a diol, such as 2-methyl-2,4-pentanediol, at a temperature between 0°C and the boiling point of the solvent, to give a compound of the formula I in which
$R^1$ has the meaning given,
$R^2$ and $R^3$ are hydrogen and
$R^4$ has the meaning given, with the exception of hydrogen,
g) and subsequently, if appropriate, possibly partly or completely deblocking the compound of stage f) in which $R^4$ is a structure of the formula II or IV with, as radicals $R^5$ to $R^8$, one of the protective groups customary in carbohydrate chemistry, in a manner which is known per se, selectively on the protected hydroxyl functions and/or on the protected amino functions under the conditions customary in carbohydrate chemistry by means of an inorganic or organic base, such as alkali metal or alkaline earth metal hydroxides, sodium carbonate and triethylamine, in a solvent, such as water, methanol, ethanol or tetrahydrofuran or mixtures thereof, to give a compound of the formula I in which
$R^1$ is hydrogen or hydroxyl,
$R^2$ and $R^3$ are hydrogen and
$R^4$ is a structure of the formula II or IV, in which
$R^5$ is hydrogen, methyl, hydroxymethyl or alkoxymethyl,
$R^6$ is hydrogen, hydroxyl, alkoxy or halogen,
$R^7$ is $NH_2$ $N(alkyl)_2$, azido, hydroxyl or alkoxy and
$R^8$ has the same meaning as $R^6$ but is independent thereof,
h) and, if appropriate, reacting the compound of stage g) of the formula I, in which
$R^1$ to $R^3$ have the meanings given under g) and
$R^4$ is a structure of the formula II, in which
$R^5$, $R^6$ and $R^8$ have the meanings given under g) and
$R^7$ is $NH_2$,
under the conditions which are known per se for reductive amination, to give the corresponding compound of the formula I in which
$R^1$ to $R^3$ have the meanings given under g) and
$R^4$ is a structure of the formula II, in which
$R^5$, $R^6$ and $R^8$ have the meanings given under g) and
$R^6$ is $N(alkyl)_2$, or
i) furthermore converting a compound of stage g) of the formula I in which
$R^1$ to $R^3$ have the meanings given under g) and
$R^4$ is a structure of the formula II in which
$R^5$, $R^6$ and $R^7$ have the meanings given under g) and
$R^7$ is $NH_2$,
by reaction with iodoacetonitrile or bromoacetonitrile in a suitable solvent, for example dimethylformamide, in the presence of a suitable base, such as triethylamine, into a compound of the formula I in which $R^1$ to $R^3$ have the meanings given under g) and $R^4$ is a structure of the formula II, in which
$R^5$, $R^6$ and $R^8$ have the meanings given under g) and
$R^7$ is $N(CH_2CN)_2$ or $NH(CH_2CN)$, and
k) if appropriate glycosidating the compound formed in stage f), h) or i) under the conditions already mentioned in stage e), either only position 7 or simultaneously positions 7 and 9 being glycosidated, depending on the amount of glycosyl donor used, to give products which correspond to the formula I in which

$R^1$ has the meaning given,
$R^2$ is a structure of the formula II or IV, in which
$R^5$ to $R^8$ have the meanings initially given, with the exception of hydroxymethyl, hydroxyl, $NH_2$, N-$(CH_2CN)_2$ and $NH(CH_2CN)$,
$R^3$ is hydrogen or corresponds to $R^2$ and $R^4$ is acyl, trimethylsilyl or a structure of the formula II or IV in which
$R^5$ to $R^8$ have the meanings initially mentioned, with the exception of hydroxymethyl, hydroxyl and $NH_2$, and, if appropriate,
l) converting one of the compounds formed in stage f), h) or i) with a glycal of the formula VI, in which $R^5$ to $R^7$ have the meanings given in stage e), in the presence of an organic solvent, such as chloroform, methylene chloride, toluene, ether, acetone or acetonitrile or mixtures thereof, with N-iodosuccinimide and if appropriate with a desiccant, such as molecular sieve, at a temperature from -40 °C to +40 °C under a protective gas atmosphere, such as nitrogen or argon, into a compound of the formula I in which
$R^1$ has the meaning given and
$R^2$ is a structure of the formula II, in which
$R^5$ is hydrogen, methyl, acyloxymethyl (C1-C8) or alkoxymethyl (C1-C8),
$R^6$ is acyloxy, alkyloxy, allyloxy or benzyloxy,
$R^7$ is acyloxy, alkyloxy, allyloxy, benzyloxy, NHacyl, $N(alkyl)_2$ or azido and
$R^8$ is iodine, and
$R^3$ and $R^4$ have the meanings given in stage k), and
m) if appropriate deblocking the compound formed in stage k) or l) in accordance with the conditions of stage g), to give a compound of the formula I in which
$R^1$ has the meaning given and
$R^2$ is a structure of the formula II or IV, in which
$R^5$ is hydrogen, methyl, hydroxymethyl or alkyloxymethyl,
$R^6$ is hydrogen, hydroxyl, alkyloxy or halogen,
$R^7$ is $NH_2$ or $N(alkyl)_2$, azido, hydroxyl or alkoxy and
$R^8$ has the same meaning as $R^6$, but is independent thereof,
$R^3$ is hydrogen or corresponds to $R^2$ and
$R^4$ is hydrogen, trimethylsilyl or a structure of the formula II or IV, in which
$R^5$ is hydrogen, methyl, hydroxymethyl or alkoxymethyl,
$R^6$ is hydrogen, hydroxyl, alkoxy or halogen,
$R^7$ is $NH_2$, $N(alkyl)_2$, $N(CH_2CN)_2$ or $NH(CH_2CN)$, azido, hydroxyl or alkoxy and $R^8$ has the same meaning as $R^6$, but is independent thereof, or
n) also deblocking the compound formed in stage k) or l) under the conditions of stage g) so that only position 10 is selectively deblocked, to give a compound of the formula I in which
$R^1$ has the meaning given,
$R^2$ is a structure of the formula II or IV, in which
$R^5$ to $R^8$ have the meanings initially given, with the exception of hydroxymethyl, hydroxyl and $NH_2$,
$R^3$ is hydrogen or corresponds to $R^2$ and
$R^4$ is hydrogen,
o) and subsequently, if appropriate, converting the compound of stage m), in which $R^2$ is a structure of the formula II where $R^7$ is $NH_2$ and $R^3$ is hydrogen or $R^2$, into the corresponding compounds in which $R^6$ is $N(alkyl)_2$, again in the manner of reductive amination described for stage h), or, if appropriate,
p) converting a compound of stage m) in which $R^2$ is a structure of the formula II, where $R^7$ is $NH_2$ and $R^3$ is hydrogen or $R^2$, into the corresponding cyanomethyl derivatives in which
$R^7$ is $N(CH_2CN)_2$ or $NH(CH_2CN)$ in accordance with the conditions of stage i), and, if appropriate,
q) glycosidating a compound of stage n) under the conditions already given in stage e), either only position 10 or simultaneously positions 9 and 10 being glycosidated, depending on the amount of glycosyl donor used, to give a compound of the formula I in which
$R^1$ is hydrogen or hydroxyl and
$R^2$ is a structure of the formula II or IV, in which
$R^5$ to $R^8$ have the meanings initially given, with the exception of hydroxymethyl, hydroxyl and $NH_2$,
$R^3$ is hydrogen or $R^2$ or $R^4$ and
$R^4$ is a structure of the formula II or IV, in which
$R^5$ to $R^8$ have the meanings initially given, with the exception of hydroxymethyl, hydroxyl, $NH_2$, N-

$(CH_2CN)_2$ or $NH(CH_2CN)$, and, if appropriate,
r) also carrying out the reaction of stages c), d) and l) on a compound of stage n) so as to give a compound of the formula I in which
$R^1$ has the meaning initially given and
$R^2$ is a structure of the formula II or IV, in which
$R^5$ to $R^8$ have the meanings initially given, with the exception of hydroxymethyl, hydroxyl and $NH_2$,
$R^3$ is hydrogen or $R^2$ or $R^4$ and
$R^4$ is a structure of the formula II or IV in which
$R^5$ to $R^8$ have the meanings initially given, with the exception of hydroxymethyl, hydroxyl, $NH_2$, $N-(CH_2CN)_2$ and $NH(CH_2CN)$, and, if appropriate,
s) deblocking this compound of stage q) or r) again as described in stage g), to give a compound of the formula I in which $R^1$ has the meaning initially given and $R^2$, $R^3$ and $R^4$ independently of one another are hydrogen or a structure of the formula II or IV, in which
$R^5$ to $R^8$ have the meanings initially given, with the exception of acyloxymethyl, acyloxy, benzoyloxy or substituted benzoyloxy or NHacyl, and, if appropriate,
t) converting the compound of stage s) into the corresponding derivative in which the radical $R^7$, which in stage s) was an $NH_2$ group, is converted into $N(alkyl)_2$, by means of reduction amination in accordance with stage h), or
u) moreover converting a compound of stage s) into the corresponding cyanomethyl derivative in which the radical $R^7$, which in stage s) was an $NH_2$ group, into $N(CH_2CN)$ in accordance with the conditions of stage i),
v) and, if appropriate, reacting a compound of the formula I in which $R^1$, $R^2$ and $R^3$ have the meanings given and $R^4$ is trimethylsilyl,
with tetrabutylammonium fluoride in an organic solvent, such as tetrahydrofuran, diethyl ether, dioxane or mixtures thereof, at temperatures between -40 °C and the boiling point of the solvent, to give a compound of the formula I in which $R^1$, $R^2$ and $R^3$ have the meanings given above and $R^4$ is hydroxyl,
w) and, if appropriate, converting compounds of the formula I in which $R^1$, $R^2$, $R^3$ and $R^4$ have the meanings given and $R^7$ is $NH_2$, $N(alkyl)_2$ (C1-C8), $N(CH_2CN)_2$ or $NH(CH_2CN)$ into the salt of an inorganic or organic acid.

**2.** The process for the preparation of a compound as claimed in claim 1, wherein
a compound of the formula I in which $R^1$ has the meaning initially given, $R^2$ together with $R^3$ forms a structure of the formula III and $R^4$ is hydrogen, is converted by acylation with a carboxylic acid anhydride, such as acetic anhydride or trifluoroacetic anhydride, or a phenylcarboxylic acid anhydride or a carboxylic acid halide or by reaction with trimethylsilyl trifluoromethanesulfonate in an organic solvent, such as chloroform, methylene chloride or toluene or mixtures thereof, at a temperature between -40 °C and the boiling point of the solvent and in the presence of a base, such as triethylamine or pyridine, into a compound of the formula I in which
$R^1$ has the meaning initially given,
$R^2$ together with $R^3$ is a structure of the formula III and
$R^4$ is acyl or trimethylsilyl, or
wherein a compound of the formula I in which $R^1$ has the meaning initially given, $R^2$ together with $R^3$ forms a structure of the formula III and $R^4$ is hydrogen, is converted by reaction with 3,4-dihydro-2H-pyran in a suitable organic solvent, such as chloroform, methylene chloride, dimethylformamide or toluene, in the presence of a catalyst, such as para-toluene-sulfonic acid, and a desiccant, such as 4Å molecular sieve, at a temperature between -30 °C and the boiling point of the solvent, into a compound of the formula I in which
$R^1$ has the meaning initially given,
$R^2$ together with $R^3$ is a structure of the formula III and
$R^4$ is a structure of the formula II where $R^5$ to $R^8$ are hydrogen.

**3.** The process for the preparation of a compound as claimed in claim 1,
wherein
a compound of the formula I in which $R^1$ has the meaning initially given, $R^2$ together with $R^3$ forms a structure of the formula III and $R^4$ has the meaning given, excluding hydrogen, can be deblocked at positions 7 and 9 by reacting this compound in a suitable organic solvent, such as chloroform, methylene chloride, dimethylformamide, toluene or methanol, with a catalyst, such as dilute aqueous

solutions of carboxylic acids or para-toluene-sulfonic acid, and if appropriate with a diol, such as 2-methyl-2,4-pentanediol, at a temperature between 0 °C and the boiling point of the solvent, to give a compound of the formula I in which
$R^1$ has the meaning given,
$R^2$ and $R^3$ are hydrogen and
$R^4$ has the meaning given, with the exception of hydrogen.

**4.** The process for the preparation of a compound as claimed in claim 1,
wherein
a compound of the formula I in which $R^1$ to $R^4$ have the meanings given, but $R^2$ or $R^4$ must be hydrogen and $R^5$ to $R^8$ cannot be hydroxyl, hydroxymethyl or $NH_2$, is reacted with a functionalized carbohydrate of the formula V or VI

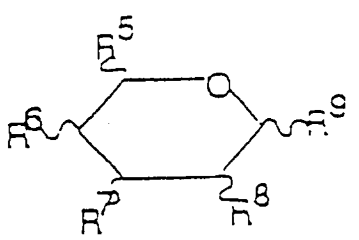

V

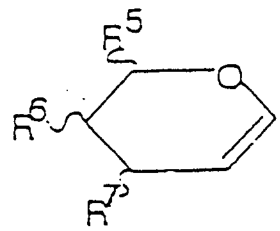

VI

in which
$R^5$ to $R^8$ have the meanings initially given, with the exception of hydroxymethyl, hydroxyl, $NH_2$, $N(CH_2CN)_2$ and $NH(CH_2CN)$ and
$R^9$ is an acyl protective group bonded via oxygen, such as aliphatic acyloxy (C1-C8), such as acetyl, benzoyloxy or substituted benzoyloxy, such as para-nitrobenzoyloxy,
in the presence of an organic solvent, such as chloroform, methylene chloride, toluene, ether, dimethylformamide, acetone, acetonitrile or nitromethane or mixtures thereof, a catalyst, such as para-toluenesulfonic acid or a trialkylsilyl trifluoromethanesulfonate, and if appropriate an acid-trapping agent and a desiccant, such as molecular sieve, at a reaction temperature of -70 °C to +30 °C under a protective gas atmosphere, such as nitrogen or argon, to give a compound of the formula I in which
$R^1$ has the meaning given,
$R^2$ together with $R^3$ is a structure of the formula III or $R^2$, $R^3$ or $R^4$ is a structure of the formula II or IV, in which
$R^5$ to $R^8$ have the meanings initially given, with the exception of hydroxymethyl, hydroxyl and $NH_2$, or
wherein a compound of the formula I in which $R^1$ to $R^4$ have the meanings given, but $R^2$ or $R^4$ must be hydrogen and $R^5$ to $R^8$ cannot be hydroxyl, hydroxymethyl or $NH_2$, is converted in the presence of an organic solvent, such as chloroform, methylene chloride, toluene, ether, acetone or acetonitrile or mixtures thereof, with a glycal of the formula VI, in which $R^5$ to $R^7$ have the meanings initially given, with the exception of hydroxymethyl, hydroxyl, $NH_2$, $N(CH_2CN)_2$ and $NH(CH_2CN)$, with N-iodosuccinimide and if appropriate with a desiccant, such as molecular sieve, at a temperature of -40 °C to +40 °C under a protective gas atmosphere, such as nitrogen or argon, into a compound of the formula I in which
$R^1$ has the meaning given,
$R^2$ together with $R^3$ is a structure of the formula III or $R^2$, $R^3$ or
$R^4$ is a structure of the formula II or IV, in which
$R^5$ to $R^8$ have the meanings initially given, with the exception of hydroxymethyl, hydroxyl, $NH_2$, $N(CH_2CN)_2$ and $NH(CH_2CN)$.

**Revendications**

**Revendications pour les Etats contractants suivants : AT, BE, CH, DE, FR, GB, IT, LI, LU, NL, SE**

1. Composé de formule I, et ses sels avec un acide organique ou minéral,

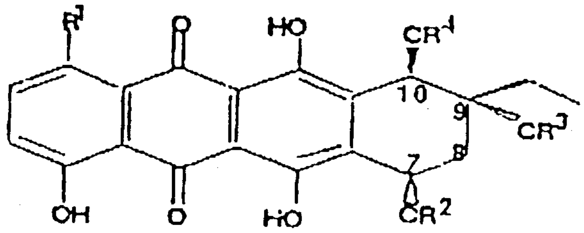

I

formule dans laquelle
$R^1$ est un atome d'hydrogène ou le groupe hydroxy,
$R^2$ est un atome d'hydrogène ou une structure de formule II ou IV ou, conjointement avec $R^3$, de formule III

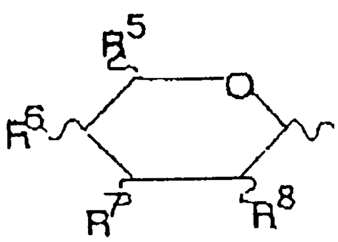

II

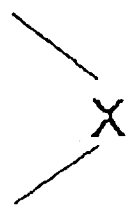

III

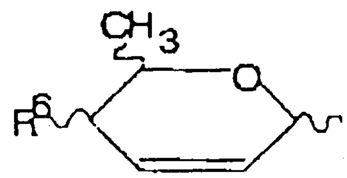

IV

$R^3$ est un atome d'hydrogène ou une structure de formule II ou IV ou, conjointement avec $R^2$, de formule III,
$R^4$ est un atome d'hydrogène ou le groupe triméthylsilyle, ou un groupe protecteur usuel dans la chimie des hydrates de carbone, de préférence le groupe acétyle, trifluoroacétyle, benzoyle ou un groupe benzoyle substitué tel que p-nitrobenzoyle, une structure de formule II ou une structure de formule IV,
$R^5$ est un atome d'hydrogène ou un groupe méthyle, hydroxyméthyle, acyloxy($C_1$-$C_8$)-méthyle ou alkyloxy($C_1$-$C_8$)-méthyle,
$R^6$, $R^7$ et $R^8$ sont, indépendamment les uns des autres, un atome d'hydrogène ou d'halogène ou un groupe hydroxy, acyloxy aliphatique en $C_1$-$C_8$, benzoyloxy ou benzoyloxy substitué, tel que p-nitrobenzoyloxy, un groupe alkyloxy en $C_1$-$C_8$, allyloxy, benzyloxy ou benzyloxy substitué, $R^7$ pouvant en outre être un groupe $NH_2$, NH-acyle en $C_1$-$C_8$, N(alkyle en $C_1$-$C_8$)$_2$, $N(CH_2CN)_2$, $NH(CH_2CN)$ ou azido, et
X est un groupe protecteur bidenté usuel dans la chimie des hydrates de carbone, de préférence un groupe alkylboronyle, phénylboronyle, un ester alkylique d'acide ortho-alkylcarboxylique, de préférence l'orthoformiate de méthyle ou l'orthoacétate d'éthyle, un cétal ou acétal, de préférence le groupe isopropylidène ou benzylidène,
à l'exclusion des composés dans lesquels $R^1 = R^2 = R^3 = R^4 = H$, $R^1 = OH$ et $R^2 = R^3 = R^4 = H$, $R^1 = R^3 = R^4 = H$ et $R^2$ est le groupe $\alpha$-L-daunosaminyle ou $\alpha$-L-rhodosaminyle ou 4'-acyl-$\alpha$-L-rhodosaminyle, $R^1$ = OH ou H, $R^3$ = H et $R^2 = R^4 = \alpha$-L-rhodosaminyle ou 4'-acyl-$\alpha$-L-rhodosaminyle et $R^1 = R^2 = R^3 = H$ et $R^4$ est le groupe $\alpha$-L-rhodosaminyle ou $\alpha$-L-daunosaminyle, ainsi que $R^1$ = OH, $R^3 = R^4 = H$ et $R^2$ représente le groupe rhodosaminyle ou daunosaminyle.

2. Composé selon la revendication 1, caractérisé en ce que $R^2$ et $R^3$ représentent, indépendamment l'un de l'autre, un atome d'hydrogène ou une structure de formule II ou IV, avec les significations indiquées pour $R^5$ à $R^8$ dans la revendication 1.

**3.** Composé selon la revendication 1, caractérisé en ce que $R^2$ et $R^3$ sont, indépendamment l'un de l'autre, un atome d'hydrogène ou une structure de formule II ou IV, $R^5$ étant le groupe méthyle, $R^6$ et $R^7$ ayant les significations données dans la revendication 1, et $R^8$ étant un atome d'hydrogène ou d'halogène.

**4.** Composé selon la revendication 1, caractérisé en ce que $R^2$ et $R^3$ sont, indépendamment l'un de l'autre, un atome d'hydrogène ou une structure de formule II ou IV, $R^5$ étant le groupe méthyle, $R^6$ ayant la signification donnée dans la revendication 1, $R^7$ étant un groupe $NH_2$, NH-acyle en $C_1$-$C_8$, N-(alkyle en $C_1$-$C_8$)$_2$, $N(CH_2CN)_2$, $NH(CH_2CN)$ ou azido, et $R^8$ étant un atome d'hydrogène ou d'halogène.

**5.** Composé selon la revendication 1, caractérisé en ce que $R^2$ et $R^3$ sont, indépendamment l'un de l'autre, un atome d'hydrogène ou une structure de formule II ou IV, $R^5$ étant le groupe méthyle, $R^6$ et $R^7$ ayant les significations données dans la revendication 1, et $R^8$ étant un atome d'hydrogène ou d'halogène, et les structures de formule II ou IV appartenant à la série L des hydrates de carbone.

**6.** Composé selon la revendication 1, caractérisé en ce que $R^2$ et $R^3$ sont, indépendamment l'un de l'autre, un atome d'hydrogène ou une structure de formule II ou IV, $R^5$ étant le groupe méthyle, $R^6$ ayant la signification donnée dans la revendication 1, $R^7$ étant un groupe $NH_2$, NH-acyle en $C_1$-$C_8$, N-(alkyle en $C_1$-$C_8$)$_2$, $N(CH_2CN)_2$, $NH(CH_2CN)$ ou azido, $R^8$ étant un atome d'hydrogène ou d'halogène, et les structures de formule II ou IV appartenant à la série L des hydrates de carbone.

**7.** Procédé pour la préparation d'un composé selon l'une des revendications 1 à 6, caractérisé en ce que

a) on fait réagir un composé de formule I dans lequel $R^1$ a la signification donnée au début et $R^2$ à $R^4$ sont des atomes d'hydrogène, dans un solvant organique approprié tel que le toluène ou le DMF ou des mélanges de ceux-ci, en présence d'un catalyseur tel qu'un acide minéral, carboxylique ou sulfonique, à une température comprise entre 0 °C et le point d'ébullition du solvant, avec un acide borique tel que l'acide phénylborique, ou avec une cétone telle que l'acétone ou un cétal tel que le 2,2-diméthoxypropane ou un acétal tel que le benzaldéhyde-diméthylacétal, éventuellement avec addition d'un déshydratant tel qu'un tamis moléculaire de 4 Å, pour obtenir un composé de formule I dans lequel $R^1$ a la signification donnée au début et $R^2$ et $R^3$ forment ensemble une structure de formule III, qui est isolé par filtration et élimination du solvant, et cristallisé dans un solvant organique tel que l'éther de pétrole, à la suite de quoi on transforme en dérivés, de façon appropriée, le groupe hydroxy en position 10,

b) éventuellement par une acylation avec un anhydride d'acide carboxylique tel que l'anhydride acétique, l'anhydride trifluoroacétique, ou un anhydride d'acide phénylcarboxylique ou un halogénure d'acide carboxylique, ou par une réaction avec du trifluorométhanesulfonate de triméthylsilyle, dans un solvant organique approprié tel que le chloroforme, le dichlorométhane ou le toluène ou des mélanges de ceux-ci, à une température comprise entre -40 °C et le point d'ébullition du solvant, et en présence d'une base telle que la triéthylamine ou la pyridine, on obtient un composé de formule I dans lequel

$R^1$ a la signification donnée au début,

$R^2$ et $R^3$ forment ensemble une structure de formule III et

$R^4$ est un groupe acyle ou triméthylsilyle,

c) ou, par exemple par la réaction du composé obtenu en a) avec du 3,4-dihydro-2H-pyranne, dans un solvant organique approprié, tel que le chloroforme, le dichlorométhane, le DMF ou le toluène, en présence d'un catalyseur tel que l'acide p-toluènesulfonique, et d'un desséchant tel qu'un tamis moléculaire de 4 Å, à une température comprise entre -30 °C et le point d'ébullition du solvant, on obtient un composé de formule I dans lequel

$R^1$ a la signification donnée au début,

$R^2$ et $R^3$ forment ensemble une structure de formule III et

$R^4$ est une structure de formule SS dans laquelle $R^5$ à $R^8$ sont des atomes d'hydrogène,

d) ou, par exemple par la réaction du composé obtenu en a), dans les conditions usuelles dans la chimie des hydrates de carbone, avec un dérivé d'hydrate de carbone de formule V,

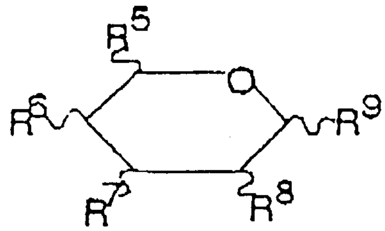

V

dans laquelle
$R^5$ à $R^8$ ont les significations données au début, en tant que groupes protecteurs appropriés, et
$R^9$ est halogène tel que Cl ou Br, un groupe O-acyle ou un autre groupe partant usuel dans des réactions de glycosidation,
on obtient un composé de formule I dans lequel
$R^1$ a la signification donnée au début,
$R^2$ et $R^3$ forment ensemble une structure de formule III et
$R^4$ est une structure de formule II,
e) ou, par exemple, en faisant réagir le composé obtenu en a) avec un hydrate de carbone fonctionnalisé de formule générale V ou VI,

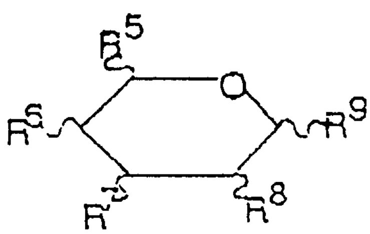

V

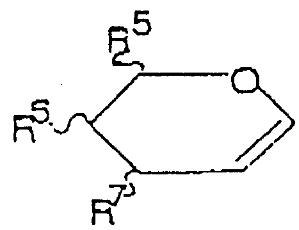

VI

formules dans lesquelles
$R^5$ à $R^8$ ont les significations données au début, à l'exception de celles des groupes hydroxyméthyle, hydroxy, $NH_2$, $N(CH_2CN)_2$ et $NH(CH_2CN)$, et
$R^9$ est un groupe acyle lié à l'atome d'oxygène, tel qu'un groupe acyloxy aliphatique en $C_1$-$C_8$, tel que le groupe acétyle, le groupe benzoyloxy ou un groupe benzoyloxy substitué, tel que p-nitrobenzoyloxy,
en présence d'un solvant organique tel que le chloroforme, le dichlorométhane, le toluène, l'éther, le DMF, l'acétone, l'acétonitrile ou le nitrométhane, ou des mélanges de ceux-ci, d'un catalyseur tel que l'acide p-toluènesulfonique ou un trifluorométhanesulfonate de trialkylsilyle, et éventuellement d'un capteur d'acide et d'un desséchant tel qu'un tamis moléculaire, à une température de réaction de -70 à +30°C, sous une atmosphère constituée d'un gaz protecteur tel que l'azote ou l'argon, on obtient un composé de formule I dans lequel
$R^1$ a la signification indiquée,
$R^2$ et $R^3$ forment ensemble une structure de formule III et
$R^4$ est une structure de formule II ou IV, dans laquelle
$R^5$ à $R^8$ ont les significations données précédemment, à l'exception de celles des groupes hydroxyméthyle, hydroxy, $NH_2$, $N(CH_2CN)_2$ et $NH(CH_2CN)$,
f) et éventuellement on peut débloquer aux positions 7 et 9 le composé des étapes b) à e), en faisant réagir ce composé, dans un solvant organique approprié, tel que le chloroforme, le dichlorométhane, le DMF, le toluène ou le méthanol, avec un catalyseur tel que des solutions aqueuses diluées d'acides carboxyliques ou d'acide p-toluènesulfonique, et éventuellement avec un diol tel que le 2-méthyl-2,4-pentanediol, à une température comprise entre 0°C et le point d'ébullition du solvant, pour obtenir un composé de formule générale I dans lequel
$R^1$ a la signification indiquée,
$R^2$ et $R^3$ sont des atomes d'hydrogène et

$R^4$ a la signification indiquée, à l'exception de celle d'un atome d'hydrogène,

g) à la suite de quoi on peut éventuellement débloquer en partie ou en totalité, sélectivement sur les fonctions hydroxy protégées et/ou sur les fonctions amino protégées, le composé de l'étape f), dans lequel $R^4$ est une structure de formule II ou IV, comportant, en tant que radicaux $R^5$ à $R^8$, l'un des groupes protecteurs usuels dans la chimie des hydrates de carbone, d'une façon connue en soi, dans des conditions utilisées habituellement dans la chimie des hydrates de carbone, au moyen d'une base organique ou minérale, telle que des hydroxydes de métaux alcalins ou alcalino-terreux, le carbonate de sodium et la triéthylamine, dans un solvant tel que l'eau, le méthanol, l'éthanol ou le THF, ou des mélanges de ceux-ci, pour obtenir un composé de formule I dans lequel

$R^1$ est un atome d'hydrogène ou le groupe hydroxy,

$R^2$ et $R^3$ sont des atomes d'hydrogène et

$R^4$ est une structure de formule II ou IV, formules dans lesquelles

$R^5$ est un atome d'hydrogène ou un groupe méthyle, hydroxyméthyle ou alkyloxyméthyle,

$R^6$ est un atome d'hydrogène ou d'halogène ou un groupe hydroxy ou alkyloxy,

$R^7$ est un groupe $NH_2$, N(alkyle)$_2$, azido, hydroxy ou alkyloxy et

$R^8$ a la même signification que $R^6$, mais indépendamment de celui-ci,

h) et on convertit éventuellement le composé de l'étape g), de formule I, dans lequel

$R^1$ à $R^3$ ont les significations données en g) et

$R^4$ est une structure de formule II,

$R^5$, $R^6$ et $R^8$ ayant les significations données en g), et

$R^7$ étant $NH_2$,

dans les conditions connues en soi de l'amination réductrice, en le composé de formule I correspondant, dans lequel

$R^1$ à $R^3$ ont les significations données en g) et

$R^4$ est une structure de formule II, dans laquelle $R^5$, $R^6$ et $R^8$ ont les significations données en g) et

$R^7$ est un groupe N(alkyle)$_2$, ou

i) on convertit en outre un composé de l'étape g), de formule générale I, dans lequel

$R^1$ a $R^3$ ont les significations données en g) et

$R^4$ est une structure de formule II, ayant pour $R^5$, $R^6$ et

$R^7$ les significations indiquées en g), et $R^7$ est $NH_2$, par une réaction avec de l'iodoacétonitrile ou du bromoacétonitrile, dans un solvant convenable, par exemple le diméthylformamide, en présence d'une base appropriée telle que la triéthylamine, en un composé de formule I dans lequel

$R^1$ à $R^3$ ont les significations données en g) et

$R^4$ est une structure de formule II, dans laquelle

$R^5$, $R^6$ et $R^8$ ont les significations données en g) et

$R^7$ est $N(CH_2CN)_2$ ou $NH(CH_2CN)$, et

k) éventuellement on soumet à une glycosidation le composé obtenu dans l'étape f), h) ou i), selon les conditions déjà indiquées dans l'étape e), en fonction de la quantité du donneur de glycosyle utilisée, seule la position 7 ou simultanément la position 7 et la position 9 étant glycosidées, ce qui conduit à des produits qui correspondent à la formule générale I, dans laquelle

$R^1$ a la signification donnée,

$R^2$ est une structure de formule II ou IV, dans laquelle

$R^5$ à $R^8$ ont les significations données au début, à l'exception de celles des groupes hydroxyméthyle, hydroxy, $NH_2$, $N(CH_2CN)_2$ et $N(CH_2CN)$,

$R^3$ est un atome d'hydrogène ou correspond à $R^2$ et

$R^4$ est un groupe acyle, triméthylsilyle ou une structure de formule générale II ou IV, formules dans lesquelles

$R^5$ à $R^8$ ont les significations données au début, à l'exception de celles des groupes hydroxyméthylène, hydroxy et $NH_2$,

et éventuellement

l) on convertit l'un des composés obtenus dans l'étape f), h) ou i), en présence d'un solvant organique tel que le chloroforme, le dichlorométhane, le toluène, l'éther, l'acétone et l'acétonitrile, ou des mélanges de ceux-ci, à l'aide d'un glycal de formule VI, $R^5$ à $R^7$ ayant les significations données dans l'étape e), avec du N-iodosuccinimide et éventuellement avec un desséchant, tel qu'un tamis moléculaire, à une température de -40 à +40 °C, dans une atmosphère constituée d'un gaz protecteur tel que l'azote ou l'argon, en un composé de formule I dans lequel

$R^1$ a la signification donnée et

$R^2$ est une structure de formule II, dans laquelle

$R^5$ est un atome d'hydrogène ou un groupe méthyle, acyloxy($C_1$-$C_8$)-méthyle, alkyloxy($C_1$-$C_8$)-méthyle,
$R^6$ est un groupe acyloxy, alkyloxy, allyloxy ou benzyloxy,
$R^7$ est un groupe acyloxy, alkyloxy, allyloxy, benzyloxy, NH-acyle, N(alkyle)$_2$, azido et
$R^8$ est un atome d'iode, et
$R^3$ et $R^4$ ont les significations données dans l'étape k), et
m) éventuellement on débloque selon les conditions de l'étape g) le composé obtenu dans l'étape k) ou l), pour obtenir un composé de formule I dans lequel
$R^1$ a la signification donnée, et
$R^2$ est une structure de formule II ou IV, formules dans lesquelles
$R^5$ est un atome d'hydrogène ou un groupe méthyle, hydroxyméthyle ou alkyloxyméthyle,
$R^6$ est un atome d'hydrogène ou d'halogène ou un groupe hydroxy ou alkyloxy,
$R^7$ est $NH_2$ ou un groupe N(alkyle)$_2$, azido, hydroxy ou alkyloxy et
$R^8$ a la même signification que $R^6$, mais indépendamment de celui-ci,
$R^3$ est un atome d'hydrogène ou correspond à $R^2$ et
$R^4$ est un atome d'hydrogène, le groupe triméthylsilyle ou une structure de formule générale II ou IV, formules dans lesquelles
$R^5$ représente un atome d'hydrogène ou un groupe
méthyle, hydroxyméthylène ou alkyloxyméthyle,
$R^6$ est un atome d'hydrogène ou d'halogène ou un groupe hydroxy ou alkyloxy,
$R^7$ est un groupe $NH_2$, N(alkyle)$_2$, $N(CH_2CN)_2$ ou $NH(CH_2CN)$, azido, hydroxy ou alkyloxy, et
$R^8$ a la même signification que $R^6$, mais indépendamment de celui-ci, ou
n) on débloque, également dans les conditions de l'étape g), le composé obtenu dans l'étape k) ou l), de manière à ne débloquer sélectivement que la position 10, pour obtenir un composé de formule I dans lequel
$R^1$ a la signification donnée,
$R^2$ est une structure de formule II ou IV, formules dans lesquelles $R^5$ à $R^8$ ont les significations données au début, à l'exception de celles des groupes hydroxyméthyle, hydroxy et $NH_2$,
$R^3$ est un atome d'hydrogène ou correspond à $R^2$ et
$R^4$ est un atome d'hydrogène,
o) à la suite de quoi on convertit éventuellement le composé de l'étape m), dans lequel $R^2$ est une structure de formule II dans laquelle $R^7$ est $NH_2$ et $R^3$ est un atome d'hydrogène ou $R^2$, à nouveau de la façon décrite dans l'étape h) pour l'amination réductrice, en les composés correspondants dans lesquels $R^6$ est un groupe N(alkyle)$_2$,ou éventuellement
p) on convertit un composé de l'étape m), dans lequel $R^2$ est une structure de formule II dans laquelle $R^7$ est $NH_2$ et $R^3$ est un atome d'hydrogène ou $R^2$, selon les conditions de l'étape i), en les dérivés cyanométhyle correspondants dans lesquels $R^7$ est $N(CH_2CN)_2$ ou $NH(CH_2CN)$, et éventuellement
q) on soumet à une glycosidation un composé de l'étape n), selon les conditions déjà indiquées dans l'étape e), en fonction de la quantité du donneur de glycosyle utilisée, seule la position 10, ou en même temps les positions 9 et 10 étant glycosidées, ce qui conduit à un composé de formule I dans lequel
$R^1$ est un atome d'hydrogène ou le groupe hydroxy,
$R^2$ est une structure de formule générale II ou IV, formules dans lesquelles $R^5$ à $R^8$ ont les significations données au début, à l'exception de celles de groupes hydroxyméthyle, hydroxy et $NH_2$,
$R^3$ est un atome d'hydrogène ou $R^2$ ou $R^4$ et
$R^4$ est une structure de formule générale II ou IV, formules dans lesquelles $R^5$ à $R^8$ ont les significations données au début, à l'exception de celles des groupes hydroxyméthyle, hydroxy, $NH_2$, $N(CH_2CN)_2$, $NH(CH_2CN)$, et éventuellement
r) on effectue la réaction des étapes c), d) et l) également sur un composé de l'étape n) de manière à obtenir un composé de formule I dans lequel
$R^1$ a la signification donnée au début et
$R^2$ est une structure de formule II ou IV, formules dans lesquelles
$R^5$ à $R^8$ ont les significations données au début, à l'exception de celles des groupes hydroxyméthyle, hydroxy et $NH_2$,
$R^3$ est un atome d'hydrogène ou $R^2$ ou $R^4$ et
$R^4$ est une structure de formule générale II ou IV, formules dans lesquelles

$R^5$ à $R^8$ ont les significations données au début, à l'exception de groupes hydroxyméthyle, hydroxy, $NH_2$, $N(CH_2CN)_2$ et $NH(CH_2CN)$, et éventuellement

s) on débloque ce composé de l'étape q) ou r), à nouveau comme décrit dans l'étape g), pour obtenir un composé de formule I dans lequel

$R^1$ a la signification donnée au début et

$R^2$, $R^3$ et $R^4$, indépendamment les uns des autres, sont un atome d'hydrogène ou une structure de formule II ou IV, formules dans lesquelles

$R^5$ à $R^8$ ont les significations données au début, à l'exception de celles des groupes acyloxyméthyle, acyloxy, benzoyloxy ou benzoyloxy substitué, NH-acyle, et éventuellement

t) on convertit le composé de l'étape s), selon l'étape h), par une amination réductrice, en le dérivé correspondant dans lequel le radical $R^7$ qui, dans l'étape s), était un groupe $NH_2$, est transformé en groupe $N(alkyle)_2$, ou

u) on convertit en outre un composé de l'étape s), selon les conditions de l'étape i), en le dérivé cyanométhyle correspondant dans lequel le radical $R^7$ qui, dans l'étape s), était un groupe $NH_2$, est converti en le groupe $NH(CH_2CN)$,

v) et on fait éventuellement réagir avec du fluorure de tétrabutylammonium un composé de formule I dans lequel

$R^1$, $R^2$ et $R^3$ ont les significations données et

$R^4$ est le groupe triméthylsilyle,

dans un solvant organique tel que le THF, l'éther diéthylique, le dioxanne ou des mélanges de ceux-ci, à des températures comprises entre -40 °C et le point d'ébullition du solvant, pour aboutir à un composé de formule I dans lequel

$R^1$, $R^2$ et $R^3$ ont les significations données plus haut et

$R^4$ est le groupe hydroxy,

w) et éventuellement on convertit en le sel d'un acide organique ou minéral des composés de formule I dans lesquels $R^1$, $R^2$, $R^3$, $R^4$ ont les significations données et $R^7$ est un groupe $NH_2$, N-(alkyle en $C_1$-$C_8$)$_2$, $N(CH_2CN)_2$ ou $NH(CH_2CN)$.

**8.** Procédé selon la revendication 7, caractérisé en ce que l'on convertit un composé de formule I dans lequel $R^1$ a la signification donnée au début, $R^2$ et $R^3$ forment ensemble une structure de formule III et $R^4$ est un atome d'hydrogène, par une acylation avec un anhydride d'acide carboxylique, tel que l'anhydride acétique, l'anhydride trifluoroacétique ou un anhydride d'acide phénylcarboxylique ou un halogénure d'acide carboxylique, ou par une réaction avec du trifluorométhanesulfonate de triméthylsilyle, dans un solvant organique tel que le chloroforme, le dichlorométhane ou le toluène, ou des mélanges de ceux-ci, à une température comprise entre -40 °C et le point d'ébullition du solvant, et en présence d'une base telle que la triéthylamine ou la pyridine, en un composé de formule I dans lequel

$R^1$ a la signification donnée au début,

$R^2$ et $R^3$ forment ensemble une structure de formule III et

$R^4$ est un groupe acyle ou triméthylsilyle, ou

en ce que l'on convertit un composé de formule I dans lequel $R^1$ a la signification donnée au début, $R^2$ et $R^3$ forment ensemble un composé de formule III et $R^4$ est un atome d'hydrogène, par une réaction avec du 3,4-dihydro-2H-pyranne dans un solvant organique approprié, tel que le chloroforme, le dichlorométhane, le DMF ou le toluène, en présence d'un catalyseur tel que l'acide p-toluènesulfonique et d'un desséchant tel qu'un tamis moléculaire de 4 Å, à une température comprise entre -30 °C et le point d'ébullition du solvant, en un composé de formule I dans lequel

$R^1$ a la signification donnée au début,

$R^2$ et $R^3$ forment ensemble une structure de formule III et

$R^4$ est une structure de formule II dans laquelle $R^5$ à $R^8$ sont des atomes d'hydrogène.

**9.** Procédé selon la revendication 7, caractérisé en ce que l'on peut débloquer aux positions 7 et 9 un composé de formule I dans lequel $R^1$ a la signification donnée au début, $R^2$ et $R^3$ forment ensemble un composé de formule III et $R^4$ a la signification donnée, à l'exception de celle d'un atome d'hydrogène, en faisant réagir ce composé de formule I dans un solvant organique approprié, tel que le chloroforme, le dichlorométhane, le DMF, le toluène ou le méthanol, à l'aide d'un catalyseur, tel que des solutions aqueuses diluées d'acides carboxyliques ou l'acide p-toluènesulfonique, et éventuellement avec un diol tel que le 2-méthyl-2,4-pentanediol, à une température comprise entre 0 °C et le point d'ébullition du solvant, pour obtenir un composé de formule générale I dans lequel

$R^1$ a la signification donnée,

$R^2$ et $R^3$ sont des atomes d'hydrogène et
$R^4$ a la signification donnée, à l'exception de celle d'un atome d'hydrogène.

**10.** Procédé selon la revendication 7, caractérisé en ce que l'on fait réagir un composé de formule I dans lequel $R^1$ à $R^4$ ont les significations données, mais $R^2$ ou $R^4$ doit être un atome d'hydrogène, et $R^5$ à $R^8$ ne peuvent pas être le groupe hydroxy, hydroxyméthyle ni $NH_2$, avec un hydrate de carbone fonctionnalisé de formule générale V ou VI

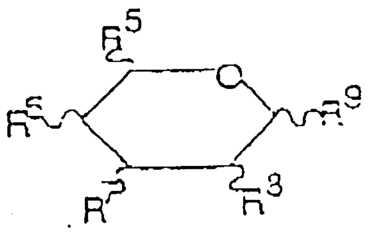

V

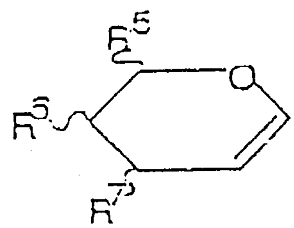

VI

formules dans lesquelles
$R^5$ à $R^8$ ont les significations données au début, à l'exception de celles des groupes hydroxyméthyle, hydroxy, $NH_2$, $N(CH_2CN)_2$ et $NH(CH_2CN)$, et
$R^9$ est un groupe protecteur de fonction acyle, lié par l'atome d'oxygène, tel qu'un groupe acyloxy aliphatique en $C_1$-$C_8$, par exemple le groupe acétyle, benzoyloxy ou un groupe benzoyloxy substitué tel que p-nitrobenzoyloxy, en présence d'un solvant organique tel que le chloroforme, le dichlorométhane, le toluène, l'éther, le DMF, l'acétone, l'acétonitrile ou le nitrométhane, ou des mélanges de ceux-ci, d'un catalyseur tel que l'acide p-toluènesulfonique ou un trifluorométhanesulfonate de trialkylsilyle, et éventuellement d'un capteur d'acide et d'un desséchant tel qu'un tamis moléculaire, à une température de réaction de -70 à +30 °C, sous une atmosphère constituée d'un gaz protecteur tel que l'azote ou l'argon, pour aboutir à un composé de formule I dans lequel
$R^1$ a la signfication donnée,
$R^2$ et $R^3$ forment ensemble une structure de formule III, ou $R^2$, $R^3$ ou $R^4$ est une structure de formule II ou IV, formules dans lesquelles
$R^5$ à $R^8$ ont les significations données au début, à l'exception de celles des groupes hydroxyméthyle, hydroxy, $NH_2$, ou
en ce que l'on convertit un composé de formule I, dans lequel $R^1$ et $R^4$ ont les significations données, mais $R^2$ ou $R^4$ doit être un atome d'hydrogène et $R^5$ à $R^8$ ne peuvent pas être le groupe hydroxy, hydroxyméthyle ou $NH_2$, en présence d'un solvant organique tel que le chloroforme, le dicloromethane, le toluène, l'éther, l'acétone ou l'acétonitrile, ou des mélanges de ceux-ci, à l'aide d'un glycal de formule VI, dans lequel $R^5$ à $R^7$ ont les significations données au début, à l'exception de celles des groupes hydroxyméthyle, hydroxy, $NH_2$, $N(CH_2CN)_2$ et $NH(CH_2CN)$, avec du N-iodosuccinimide et éventuellement avec un desséchant, tel qu'un tamis moléculaire, à une température de -40 à +40 °C, sous une atmosphère constituée d'un gaz protecteur tel que l'azote ou l'argon, en un composé de formule I dans lequel $R^1$ a la signification donnée,
$R^2$ et $R^3$ forment ensemble une structure de formule III, ou
$R^2$, $R^3$ ou $R^4$ est une structure de formule II ou IV, formules dans lesquelles
$R^5$ à $R^8$ ont les significations données au début, à l'exception de celles des groupes hydroxyméthyle, hydroxy, $NH_2$, $N(CH_2CN)_2$ et $NH(CH_2CN)$.

**11.** Utilisation d'un composé selon l'une des revendications 1 à 10, pour la fabrication d'un médicament.

**Revendications pour les Etats contractants suivants : ES, GR**

**1.** Procédé pour la préparation d'un composé de formule I, et éventuellement d'un de ses sels avec un acide organique ou minéral,

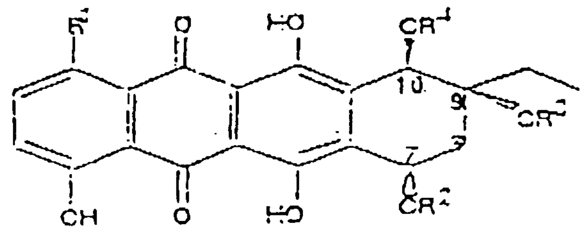

I

formule dans laquelle

$R^1$ est un atome d'hydrogène ou le groupe hydroxy,

$R^2$ est un atome d'hydrogène ou une structure de formule II ou IV ou, conjointement avec $R^3$, de formule III

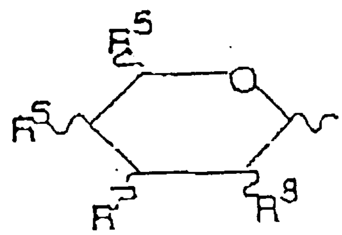

II

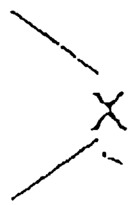

III

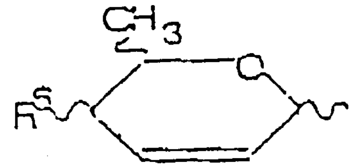

IV

$R^3$ est un atome d'hydrogène ou une structure de formule II ou IV ou, conjointement avec $R^2$, de formule III,

$R^4$ est un atome d'hydrogène ou le groupe triméthylsilyle, ou un groupe protecteur usuel dans la chimie des hydrates de carbone, de préférence le groupe acétyle, trifluoroacétyle, benzoyle ou un groupe benzoyle substitué tel que p-nitrobenzoyle, une structure de formule II ou une structure de formule IV,

$R^5$ est un atome d'hydrogène ou un groupe méthyle, hydroxyméthyle, acyloxy($C_1$-$C_8$)-méthyle ou alkyloxy($C_1$-$C_8$)-méthyle,

$R^6$, $R^7$ et $R^8$ sont, indépendamment les uns des autres, un atome d'hydrogène ou d'halogène ou un groupe hydroxy, acyloxy aliphatique en $C_1$-$C_8$, benzoyloxy ou benzoyloxy substitué, tel que p-nitrobenzoyloxy, un groupe alkyloxy en $C_1$-$C_8$, allyloxy, benzyloxy ou benzyloxy substitué, $R^7$ pouvant en outre être un groupe $NH_2$, NH-acyle en $C_1$-$C_8$, N(alkyle en $C_1$-$C_8$)$_2$, $N(CH_2CN)_2$, $NH(CH_2CN)$ ou azido, et

X est un groupe protecteur bidenté usuel dans la chimie des hydrates de carbone, de préférence un groupe alkylboronyle, phénylboronyle, un ester alkylique d'acide ortho-alkylcarboxylique, de préférence l'orthoformiate de méthyle ou l'orthoacétate d'éthyle, un cétal ou acétal, de préférence le groupe isopropylidène ou benzylidène,

à l'exclusion des composés dans lesquels

$R^1 = R^2 = R^3 = R^4 = H$, $R^1 = OH$ et $R^2 = R^3 = R^4 = H$, $R^1 = R^3 = R^4 = H$ et $R^2$ est le groupe $\alpha$-L-daunosaminyle ou $\alpha$-L-rhodosaminyle ou 4'-acyl-$\alpha$-L-rhodosaminyle, $R^1 = OH$ ou H, $R^3 = H$ et $R^2 = R^4 = \alpha$-L-rhodosaminyle ou 4'-acyl-$\alpha$-L-rhodosaminyle et $R^1 = R^2 = R^3 = H$ et $R^4$ est le groupe $\alpha$-L-rhodosaminyle, ainsi que $R^1 = OH$, $R^3 = R^4 = H$ et $R^2$ représente le groupe rhodosaminyle ou daunosaminyle,

caractérisé en ce que

a) on fait réagir un composé de formule I dans lequel $R^1$ a la signification donnée au début, et $R^2$ à $R^4$ sont des atomes d'hydrogène, dans un solvant organique approprié tel que le toluène ou le DMF ou des mélanges de ceux-ci, en présence d'un catalyseur tel qu'un acide minéral, carboxylique ou sulfonique, à une température comprise entre 0 ° C et le point d'ébullition du solvant, avec un acide borique tel que l'acide phénylborique, ou avec une cétone telle que l'acétone ou un cétal tel que le 2,2-diméthoxypropane ou un acétal tel que le benzaldéhydediméthylacétal, éventuellement avec addition d'un déshydratant tel qu'un tamis moléculaire de 4 Å, pour obtenir un composé de formule I dans lequel $R^1$ a la signification donnée au début et $R^2$ et $R^3$ forment ensemble une structure de formule III, qui est isolé par filtration et élimination du solvant, et cristallisé dans un solvant

organique tel que l'éther de pétrole, à la suite de quoi on transforme en dérivés, de façon appropriée, le groupe hydroxy en position 10,

b) éventuellement par une acylation avec un anhydride d'acide carboxylique tel que l'anhydride acétique, l'anhydride trifluoroacétique, ou un anhydride d'acide phénylcarboxylique ou un halogénure d'acide carboxylique, ou par une réaction avec du trifluorométhanesulfonate de triméthylsilyle, dans un solvant organique approprié tel que le chloroforme, le dichlorométhane ou le toluène ou des mélanges de ceux-ci, à une température comprise entre -40 °C et le point d'ébullition du solvant, et en présence d'une base telle que la triéthylamine ou la pyridine, on obtient un composé de formule I dans lequel

$R^1$ a la signification donnée au début,

$R^2$ et $R^3$ forment ensemble une structure de formule III, et

$R^4$ est un groupe acyle ou triméthylsilyle,

c) ou, par exemple par la réaction du composé obtenu en a) avec du 3,4-dihydro-2H-pyranne, dans un solvant organique approprié tel que le chloroforme, le dichlorométhane, le DMF ou le toluène, en présence d'un catalyseur tel que l'acide p-toluènesulfonique, et d'un desséchant tel qu'un tamis moléculaire de 4 Å, à une température comprise entre -30 °C et le point d'ébullition du solvant, on obtient un composé de formule I dans lequel

$R^1$ a la signification donnée au début,

$R^2$ et $R^3$ forment ensemble une structure de formule III et

$R^4$ est une structure de formule II dans laquelle $R^5$ à $R^8$ sont des atomes d'hydrogène,

d) ou, par exemple par la réaction du composé obtenu en a), dans les conditions usuelles dans la chimie des hydrates de carbone, avec un dérivé d'hydrate de carbone de formule V,

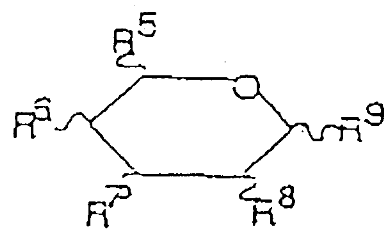

V

dans laquelle

$R^5$ à $R^8$ ont les significations données au début, en tant que groupes protecteurs appropriés, et

$R^9$ est halogène tel que Cl ou Br, un groupe O-acyle ou un autre groupe partant usuel dans des réactions de glycosidation,

on obtient un composé de formule I dans lequel

$R^1$ a la signification donnée au début,

$R^2$ et $R^3$ forment ensemble une structure de formule III et

$R^4$ est une structure de formule II,

e) ou, par exemple, en faisant réagir le composé obtenu en a) avec un hydrate de carbone fonctionnalisé de formule générale V ou VI,

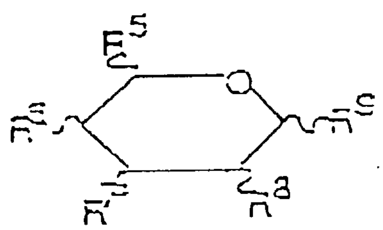

V

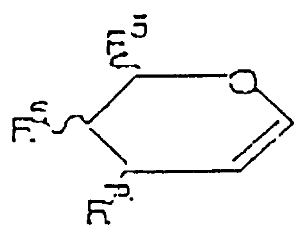

VI

formules dans lesquelles

$R^5$ à $R^8$ ont les significations données au début, à l'exception de celles des groupes hydroxyméthyle, hydroxy, $NH_2$, $N(CH_2CN)_2$ et $NH(CH_2CN)$, et

$R^9$ est un groupe acyle lié à l'atome d'oxygène, tel qu'un groupe acyloxy aliphatique en $C_1$-$C_8$, tel que le groupe acétyle, le groupe benzoyloxy ou un groupe benzoyloxy substitué, tel que p-nitrobenzoyloxy,

en présence d'un solvant organique tel que le chloroforme, le dichlorométhane, le toluène, l'éther, le DMF, l'acétone, l'acétonitrile ou le nitrométhane, ou des mélanges de ceux-ci, d'un catalyseur tel que l'acide p-toluènesulfonique ou un trifluorométhanesulfonate de trialkylsilyle, et éventuellement d'un capteur d'acide et d'un desséchant tel qu'un tamis moléculaire, à une température de réaction de -70 à +30°C, sous une atmosphère constituée d'un gaz protecteur tel que l'azote ou l'argon, on obtient un composé de formule I dans lequel

$R^1$ a la signification indiquée,

$R^2$ et $R^3$ forment ensemble une structure de formule III et

$R^4$ est une structure de formule II ou IV, dans laquelle

$R^5$ à $R^8$ ont les significations données précédemment, à l'exception de celles des groupes hydroxyméthyle, hydroxy, $NH_2$, $N(CH_2CN)_2$ et $NH(CH_2CN)$,

f) et éventuellement on peut débloquer aux positions 7 et 9 le composé des étapes b) à e), en faisant réagir ce composé, dans un solvant organique approprié, tel que le chloroforme, le dichlorométhane, le DMF, le toluène ou le méthanol, avec un catalyseur tel que des solutions aqueuses diluées d'acides carboxyliques ou d'acide p-toluènesulfonique, et éventuellement avec un diol tel que le 2-méthyl-2,4-pentanediol, à une température comprise entre 0°C et le point d'ébullition du solvant, pour obtenir un commposé de formule générale I dans lequel

$R^1$ a la signification indiquée,

$R^2$ et $R^3$ sont des atomes d'hydrogène et

$R^4$ a la signification indiquée, à l'exception de celle d'un atome d'hydrogène,

g) à la suite de quoi on peut éventuellement débloquer en partie ou en totalité, sélectivement sur les fonctions hydroxy protégées et/ou sur les fonctions amino protégées, le composé de l'étape f), dans lequel $R^4$ est une structure de formule II ou IV, comportant, en tant que radicaux $R^5$ à $R^8$, l'un des groupes protecteurs usuels dans la chimie des hydrates de carbone, d'une façon connue en soi, dans des conditions utilisées habituellement dans la chimie des hydrates de carbone, au moyen d'une base organique ou minérale, telle que des hydroxydes de métaux alcalins ou alcalino-terreux, le carbonate de sodium et la triéthylamine, dans un solvant tel que l'eau, le méthanol, l'éthanol ou le THF, ou des mélanges de ceux-ci, pour obtenir un composé de formule I dans lequel

$R^1$ est un atome d'hydrogène ou le groupe hydroxy,

$R^2$ et $R^3$ sont des atomes d'hydrogène et

$R^4$ est une structure de formule II ou IV, formules dans lesquelles

$R^5$ est un atome d'hydrogène ou un groupe méthyle, hydroxyméthyle ou alkyloxyméthyle,

$R^6$ est un atome d'hydrogène ou d'halogène ou un groupe hydroxy ou alkyloxy,

$R^7$ est un groupe $NH_2$, N(alkyle)$_2$, azido, hydroxy ou alkyloxy et

$R^8$ a la même signification que $R^6$, mais indépendamment de celui-ci,

h) et on convertit éventuellement le composé de l'étape g), de formule I, dans lequel

$R^1$ à $R^3$ ont les significations données en g) et

$R^4$ est une structure de formule II,

$R^5$, $R^6$ et $R^8$ ayant les significations données en g), et

$R^7$ étant $NH_2$,

dans les conditions connues en soi de l'amination réductrice, en le composé de formule I correspondant, dans lequel

$R^1$ à $R^3$ ont les significations données en g) et

$R^4$ est une structure de formule II, dans laquelle

$R^5$, $R^6$ et $R^8$ ont les significations données en g) et $R^7$ est un groupe N(alkyle)$_2$, ou

i) on convertit en outre un composé de l'étape g), de formule générale I, dans lequel

$R^1$ à $R^3$ ont les significations données en g) et

$R^4$ est une structure de formule II, ayant pour $R^5$, $R^6$ et

$R^7$ les significations indiquées en g), et $R^7$ est $NH_2$, par une réaction avec de l'iodoacétonitrile ou du bromoacétonitrile, dans un solvant convenable, par exemple le diméthylformamide, en présence d'une base appropriée telle que la triéthylamine, en un composé de formule I dans lequel

$R^1$ à $R^3$ ont les significations données en g) et

$R^4$ est une structure de formule II, dans laquelle

$R^5$, $R^6$ et $R^8$ ont les significations données en g) et

$R^7$ est $N(CH_2CN)_2$ ou $NH(CH_2CN)$, et

k) éventuellement on soumet à une glycosidation le composé obtenu dans l'étape f), h) ou i), selon les conditions déjà indiquées dans l'étape e), en fonction de la quantité du donneur de glycosyle utilisée, seule la position 7 ou simultanément la position 7 et la position 9 étant glycosidées, ce qui conduit à des produits qui correspondent à la formule générale I, dans laquelle

$R^1$ a la signification donnée,

$R^2$ est une structure de formule II ou IV, dans laquelle

$R^5$ à $R^8$ ont les significations données au début, à l'exception de celles des groupes hydroxyméthyle, hydroxy, $NH_2$, $N(CH_2CN)_2$ et $N(CH_2CN)$,

$R^3$ est un atome d'hydrogène ou correspond à $R^2$ et

$R^4$ est un groupe acyle, triméthylsilyle ou une structure de formule générale II ou IV, formules dans lesquelles

$R^5$ à $R^8$ ont les significations données au début, à l'exception de celles des groupes hydroxyméthylène, hydroxy et $NH_2$,

et éventuellement

l) on convertit l'un des composés obtenus dans l'étape f), h) ou i), en présence d'un solvant organique tel que le chloroforme, le dichlorométhane, le toluène, l'éther, l'acétone et l'acétonitrile, ou des mélanges de ceux-ci, à l'aide d'un glycal de formule VI, $R^5$ à $R^7$ ayant les significations données dans l'étape e), avec du N-iodosuccinimide et éventuellement avec un desséchant, tel qu'un tamis moléculaire, à une température de -40 à +40 °C, dans une atmosphère constituée d'un gaz protecteur tel que l'azote ou l'argon, en un composé de formule I dans lequel

$R^1$ a la signification donnée et

$R^2$ est une structure de formule II, dans laquelle

$R^5$ est un atome d'hydrogène ou un groupe méthyle, acyloxy($C_1$-$C_8$)-méthyle, alkyloxy($C_1$-$C_8$)-méthyle,

$R^6$ est un groupe acyloxy, alkyloxy, allyloxy ou benzyloxy,

$R^7$ est un groupe acyloxy, alkyloxy, allyloxy, benzyloxy, NH-acyle, $N(alkyle)_2$, azido et

$R^8$ est un atome d'iode, et

$R^3$ et $R^4$ ont les significations données dans l'étape k), et

m) éventuellement on débloque selon les conditions de l'étape g) le composé obtenu dans l'étape k) ou l), pur obtenir un composé de formule I dans lequel

$R^1$ a la signification donnée et

$R^2$ est une structure de formule II ou IV, formules dans lesquelles

$R^5$ est un atome d'hydrogène ou un groupe méthyle, hydroxyméthyle ou alkyloxyméthyle,

$R^6$ est un atome d'hydrogène ou d'halogène ou un groupe hydroxy ou alkyloxy,

$R^7$ est $NH_2$ ou un groupe $N(alkyle)_2$, azido, hydroxy ou alkyloxy, et

$R^8$ a la même signification que $R^6$, mais indépendamment de celui-ci,

$R^3$ est un atome d'hydrogène ou correspond à $R^2$ et

$R^4$ est un atome d'hydrogène, le groupe triméthylsilyle ou une structure de formule générale II ou IV, formules dans lesquelles

$R^5$ représente un atome d'hydrogène ou un groupe méthyle, hydroxyméthylène ou alkyloxyméthyle,

$R^6$ est un atome d'hydrogène ou d'halogène ou un groupe hydroxy ou alkyloxy,

$R^7$ est un groupe $NH_2$, $N(alkyle)_2$, $N(CH_2CN)_2$ ou $NH(CH_2CN)$, azido, hydroxy ou alkyloxy, et

$R^8$ a la même signification que $R^6$, mais indépendamment de celui-ci, ou

n) on débloque, également dans les conditions de l'étape g), le composé obtenu dans l'étape k) ou l), de manière à ne débloquer sélectivement que la position 10, pour obtenir un composé de formule I dans lequel

$R^1$ a la signification donnée,

$R^2$ est une structure de formule II ou IV, formules dans lesquelles $R^5$ à $R^8$ ont les significations données au début, à l'exception de celles des groupes hydroxyméthyle, hydroxy et $NH_2$,

$R^3$ est un atome d'hydrogène ou correspond à $R^2$ et

$R^4$ est un atome d'hydrogène,

o) à la suite de quoi on convertit éventuellement le compose de l'étape m), dans lequel $R^2$ est une structure de formule II dans laquelle $R^7$ est $NH_2$ et $R^3$ est un atome d'hydrogène ou $R^2$, à nouveau de la façon décrite dans l'étape h) pour l'amination réductrice, en les composés correspondant dans lesquels $R^6$ est un groupe $N(alkyle)_2$,ou éventuellement

p) on convertit un composé de l'étape m), dans lequel $R^2$ est une structure de formule II dans laquelle $R^7$ est $NH_2$ et $R^3$ est un atome d'hydrogène ou $R^2$, selon les conditions de l'étape i), en les dérivés cyanométhyle correspondants, dans lesquels $R^7$ est $N(CH_2CN)_2$ ou $NH(CH_2CN)$, et éventuellement

q) on soumet à une glycosidation un composé de l'étape n), selon les conditions déjà indiquées dans l'étape e), en fonction de la quantité du donneur de glycosyle utilisée, seule la position 10, ou en même temps les positions 9 et 10 étant glycosidées, ce qui conduit à un composé de formule I dans lequel

$R^1$ est un atome d'hydrogène ou le groupe hydroxy,

$R^2$ est une structure de formule générale II ou IV, formules dans lesquelles $R^5$ à $R^8$ ont les significations données au début, à l'exception de celles de groupes hydroxyméthyle, hydroxy et $NH_2$,

$R^3$ est un atome d'hydrogène ou $R^2$ ou $R^4$ et

$R^4$ est une structure de formule générale II ou IV, formules dans lesquelles $R^5$ à $R^8$ ont les significations données au début, à l'exception de celles des groupes hydroxyméthyle, hydroxy, $NH_2$, $N(CH_2CN)_2$, $NH(CH_2CN)$, et éventuellement

r) on effectue la réaction des étapes c), d) et l) également sur un composé de l'étape n) de manière à obtenir un composé de formule I dans lequel

$R^1$ a la signification donnée au début et

$R^2$ est une structure de formule II ou IV, formules dans lesquelles

$R^5$ à $R^8$ ont les significations données au début, à l'exception de celles des groupes hydroxyméthyle, hydroxy et $NH_2$,

$R^3$ est un atome d'hydrogène ou $R^2$ ou $R^4$ et

$R^4$ est une structure de formule générale II ou IV, formules dans lesquelles $R^5$ à $R^8$ ont les significations données au début, à l'exception de groupes hydroxyméthyle, hydroxy, $NH_2$, $N(CH_2CN)_2$ et $NH(CH_2CN)$, et éventuellement

s) on débloque ce composé de l'étape q) ou r), à nouveau comme décrit dans l'étape g), pour obtenir un composé de formule I dans lequel

$R^1$ a la signification donnée au début et

$R^2$, $R^3$ et $R^4$, indépendamment les uns des autres, sont un atome d'hydrogène ou une structure de formule II ou IV, formules dans lesquelles

$R^5$ à $R^8$ ont les significations données au début, à l'exception de celles des groupes acyloxyméthyle, acyloxy, benzoyloxy ou benzoyloxy substitué, NH-acyle, et éventuellement

t) on convertit le composé de l'étape s), selon l'étape h), par une amination réductrice, en le dérivé correspondant dans lequel le radical $R^7$ qui, dans l'étape s), était un groupe $NH_2$, est transformé en gr oupe $N(alkyle)_2$, ou

u) on convertit en outre un composé de l'étape s), selon les conditions de l'étape i), en le dérivé cyanométhyle correspondant dans lequel le radical $R^7$ qui, dans l'étape s), était un groupe $NH_2$, est converti en le groupe $NH(CH_2CN)$,

v) et on fait éventuellement réagir avec du fluorure de tétrabutylammonium un composé de formule I dans lequel

$R^1$, $R^2$ et $R^3$ ont les significations données et

$R^4$ est le groupe triméthylsilyle,

dans un solvant organique tel que le THF, l'éther diéthylique, le dioxanne ou des mélanges de ceux-ci, à des températures comprise entre -40 °C et le point d'ébullition du solvant, pour aboutir à un composé de formule I dans lequel

$R^1$, $R^2$ et $R^3$ ont les significations données plus haut et

$R^4$ est le groupe hydroxy,

w) et éventuellement on convertit en le sel d'un acide organique ou minéral des composés de formule I dans lesquels $R^1$, $R^2$, $R^3$, $R^4$ ont les significations données et $R^7$ est un groupe $NH_2$, N-(alkyle en $C_1$-$C_8$)$_2$, $N(CH_2CN)_2$ ou $NH(CH_2CN)$.

**2.** Procédé pour la préparation d'un composé selon la revendication 1, caractérisé en ce que l'on convertit un composé de formule I dans lequel $R^1$ a la signification donnée au début, $R^2$ et $R^3$ forment ensemble une structure de formule III et $R^4$ est un atome d'hydrogène, par une acylation avec un anhydride d'acide carboxylique, tel que l'anhydride acétique, l'anhydride trifluoroacétique ou un anhydride d'acide phénylcarboxylique ou un halogénure d'acide carboxylique, ou par une réaction avec du trifluorométhanesulfonate de triméthylsilyle, dans un solvant organique tel que le chloroforme, le dichlorométhane ou

le toluène, ou des mélanges de ceux-ci, à une température comprise entre -40 °C et le point d'ébullition du solvant, et en présence d'une base telle que la triéthylamine ou la pyridine, en un composé de formule I dans lequel
$R^1$ a la signification donnée au début,
$R^2$ et $R^3$ forment ensemble une structure de formule III et
$R^4$ est un groupe acyle ou triméthylsilyle, ou
en ce que l'on convertit un composé de formule I, dans lequel $R^1$ a la signification donnée au début, $R^2$ et $R^3$ forment ensemble un composé de formule III et $R^4$ est un atome d'hydrogène, par une réaction avec du 3,4-dihydro-2H-pyranne dans un solvant organique approprié, tel que le chloroforme, le dichlorométhane, le DMF ou le toluène, en présence d'un catalyseur tel que l'acide p-toluènesulfonique et d'un desséchant tel qu'un tamis moléculaire de 4 Å, à une température comprise entre -30 °C et le point d'ébullition du solvant, en un composé de formule I dans lequel
$R^1$ a la signification donnée au début,
$R^2$ et $R^3$ forment ensemble une structure de formule III et
$R^4$ est une structure de formule II dans laquelle $R^5$ à $R^8$ sont des atomes d'hydrogène.

3. Procédé pour la préparation d'un composé selon la revendication 1, caractérisé en ce que l'on peut débloquer aux positions 7 et 9 un composé de formule I dans lequel $R^1$ a la signification donnée au début, $R^2$ et $R^3$ forment ensemble un composé de formule III et $R^4$ a la signification donnée, à l'exception de celle d'un atome d'hydrogène, en faisant réagir ce composé de formule I dans un solvant organique approprié, tel que le chloroforme, le dichlorométhane, le DMF, le toluène ou le méthanol, à l'aide d'un catalyseur, tel que des solutions aqueuses diluées d'acides carboxyliques ou l'acide p-toluènesulfonique, et éventuellement avec un diol tel que le 2-méthyl-2,4-pentanediol, à une température comprise entre 0 °C et le point d'ébullition du solvant, pour obtenir un composé de formule générale I dans lequel
$R^1$ a la signification donnée,
$R^2$ et $R^3$ sont des atomes d'hydrogène et
$R^4$ a la signification donnée, à l'exception de celle d'un atome d'hydrogène.

4. Procédé pour la préparation d'un composé selon la revendication 1, caractérisé en ce que l'on fait réagir un composé de formule I dans lequel $R^1$ à $R^4$ ont les significations données, mais $R^2$ ou $R^4$ doit être un atome d'hydrogène, et $R^5$ à $R^8$ ne peuvent pas être le groupe hydroxy, hydroxyméthyle ni $NH_2$, avec un hydrate de carbone fonctionnalisé de formule générale V ou VI

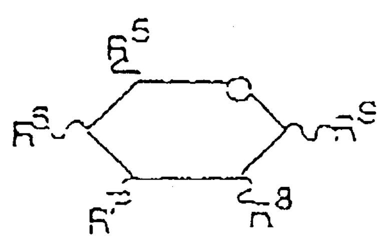

V

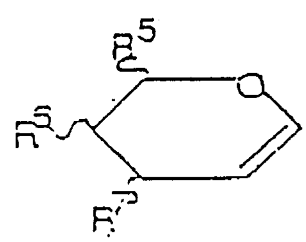

VI

formules dans lesquelles
$R^5$ à $R^8$ ont les significations données au début, à l'exception de celles des groupes hydroxyméthyle, hydroxy, $NH_2$, $N(CH_2CN)_2$ et $NH(CH_2CN)$, et
$R^9$ est un groupe protecteur de fonction acyle, lié par l'atome d'oxygène, tel qu'un groupe acyloxy aliphatique en $C_1$-$C_8$, par exemple le groupe acétyle, benzoyloxy ou un groupe benzoyloxy substitué tel que p-nitrobenzoyloxy, en présence d'un solvant organique tel que le chloroforme, le dichlorométhane, le toluène, l'éther, le DMF, l'acétone, l'acétonitrile ou le nitrométhane, ou des mélanges de ceux-ci, d'un catalyseur tel que l'acide p-toluènesulfonique ou un trifluorométhanesulfonate de trialkylsilyle, et éventuellement d'un capteur d'acide et d'un desséchant tel qu'un tamis moléculaire, à une température de réaction de -70 à +30 °C, sous une atmosphère constituée d'un gaz protecteur tel que l'azote ou l'argon, pour aboutir à un composé de formule I dans lequel
$R^1$ a la signfication donnée,
$R^2$ et $R^3$ forment ensemble une structure de formule III, ou $R^2$, $R^3$ ou $R^4$ est une structure de formule II

ou IV, formules dans lesquelles
$R^5$ à $R^8$ ont les significations données au début, à l'exception de celles des groupes hydroxyméthyle, hydroxy, $NH_2$, ou
en ce que l'on convertit un composé de formule I, dans lequel $R^1$ et $R^4$ ont les significations données, mais $R^2$ ou $R^4$ doit être un atome d'hydrogène et $R^5$ à $R^8$ ne peuvent pas être le groupe hydroxy, hydroxyméthyle ou $NH_2$, en présence d'un solvant organique tel que le chloroforme, le diclorométhane, le toluène, l'éther, l'acétone ou l'acétonitrile, ou des mélanges de ceux-ci, à l'aide d'un glycal de formule VI, dans lequel $R^5$ à $R^7$ ont les significations données au début, à l'exception de celles des groupes hydroxyméthyle, hydroxy, $NH_2$, $N(CH_2CN)_2$ et $NH(CH_2CN)$, avec du N-iodosuccinimide et éventuellement avec un desséchant, tel qu'un tamis moléculaire, à une température de -40 à +40 °C, sous une atmosphère constituée d'un gaz protecteur, tel que l'azote ou l'argon, en un composé de formule I dans lequel
$R^1$ a la signification donnée,
$R^2$ et $R^3$ forment ensemble une structure de formule III ou
$R^2$, $R^3$ ou $R^4$ est une structure de formule II ou IV, formules dans lesquelles
$R^5$ à $R^8$ ont les significations données au début, à l'exception de celles des groupes hydroxyméthyle, hydroxy, $NH_2$, $N(CH_2CN)_2$ et $NH(CH_2CN)$.